# EUROPEAN PATENT SPECIFICATION

(11) **EP 2 588 490 B1**
(45) Date of publication and mention of the grant of the patent: **22.02.2017**
(21) Application number: 11800042.1
(22) Date of filing: 04.07.2011
(51) Int. Cl.: C07K 7/06, C07K 7/08, C07K 14/81

(54) **SHORT AND D-AMINO ACID-CONTAINING POLYPEPTIDES FOR THERAPEUTIC CONJUGATES AND USES THEREOF**
KURZE UND D-AMINOSÄURE-HALTIGE POLYPEPTIDE FÜR THERAPEUTISCHE KONJUGATE UND IHRE VERWENDUNGEN
POLYPEPTIDES COURTS CONTENANT DES ACIDES AMINÉS D POUR CONJUGUÉS THÉRAPEUTIQUES ET LEURS UTILISATIONS

(30) Priority: 07.06.2011 US 201161494368 P; 07.06.2011 US 201161494277 P; 02.07.2010 US 361305 P
(43) Date of publication of application: 08.05.2013
(73) Proprietor: Angiochem Inc., Montréal, QC H2X 3Y7 (CA)
(72) Inventor: CASTAIGNE, Jean-Paul, Mont-Royal Québec H3P 1Y6 (CA); DEMEULE, Michel, Beaconsfield Québec H9W 1Z2 (CA); CHE, Christian, Longueuil Québec J4K 1A7 (CA); THIOT, Carine, Montréal Québec H4E 1X2 (CA); PESLHERBE, Laurence, Côte-St-Luc Québec H4V 2G7 (CA)
(74) Representative: Lahrtz, Fritz
(86) International application number: PCT/CA2011/050408
(87) International publication number: WO 2012/000118

(56) References cited:
- WO-A-2011/153642
- WO-A1-2008/144919
- WO-A1-2009/079790
- WO-A1-2010/063122
- WO-A1-2010/142035
- WO-A2-00/71574
- WO-A2-02/16553
- WO-A2-2004/060403
- M BOULES ET AL.: "Bioactive analogs of neurotensin: focus on CNS effect", PEPTIDES, vol. 27, 2006, pages 2623-2533, XP27957628, ELSEVIER ISSN: 0196-9781
- CHEMICAL ABSTRACTS, vol. 97 1982, Columbus, Ohio, US; abstract no.: 182849,

## Description

### Field of the Invention

This invention relates, in part, to short polypeptides useful as targeting moieties. The invention also relates to conjugates including a targeting moiety linked to a therapeutic agent or a transport vector and uses thereof.

### Background of the Invention

The brain is shielded against potentially toxic substances by the presence of two barrier systems: the blood-brain barrier (BBB) and the blood-cerebrospinal fluid barrier (BCSFB). The BBB is considered to be the major route for the uptake of serum ligands since its surface area is approximately 5000-fold greater than that of BCSFB. The brain endothelium, which constitutes the BBB, represents the major obstacle for the use of potential drugs against many disorders of the central nervous system (CNS). As a general rule, only small lipophilic molecules may pass across the BBB, *i*.*e*., from circulating systemic blood to brain. Many drugs that have a larger size or higher hydrophobicity show promising results in animal studies for treating CNS disorders but often do not cross the BBB. Thus, peptide and protein therapeutics are generally excluded from transport from blood to brain, owing to the negligible permeability of the brain capillary endothelial wall to these drugs.

Treatment of brain diseases is often impaired by the inability of otherwise effective therapeutic agents to cross the BBB. Thus, new strategies for transporting agents into the brain are desired.

Boules M. et al. (Peptides, 2006, 27: 2623-2533) discloses bioactive analogs of neurotensin and effects thereof on CNS.

WO 2010/063122 A1 discloses neurotensin or neurotensin analogs conjugated to targeting peptides which serve to target the neurotensin to the brain and other organs. The conjugates are different from the conjugates of the present invention.

WO 2009/079790 A1 discloses siRNA conjugated to targeting peptides, as well as mutants and fragments thereof and the use of the siRNA conjugates in the treatment of neurodegenerative disorders and cancer. The conjugates are different from the conjugates of the present invention.

WO 2008/144919 A1 discloses polypeptides conjugated to targeting peptides wherein the polypeptides include anticancer agents, antibodies, antibody fragments, leptins or GLP-1 and the use of the polypeptide conjugates in the treatment of cancer and metabolic disorders including obesity. The conjugates are different from the conjugates of the present invention.

WO 00/71574 A2 discloses antigenic peptides from *Neasseria sp.* comprising the sequence KRNNYK.

WO 2010/142035 A1 discloses compounds that include a peptide vector which acts as a carrier across the blood-brain barrier, linked to glial-derived neurotrophic factor (GDNF), or brain-derived neurotrophic factor (BDNF).

### Summary of the Invention

We have now developed short polypeptides (e.g., fewer than 19 amino acids in length) that are capable of crossing the blood-brain barrier (BBB) or entering particular cell types (e.g., liver, eye, lung, kidney, spleen, muscle, or ovary) with enhanced efficiency, We have also developed both short (e.g., 6-18 amino acids in length) and longer (e.g., 19 or more amino acids in length) polypeptides having one or more D-amino acids (*e.g*., 3D-An2 or fragments thereof). These polypeptides can serve as targeting moieties. When these targeting moieties are joined with (*e.g*., conjugated to) one or more agents or transport vectors, efficiency of transport across the BBB or into particular cell types is likewise enhanced. Accordingly, the present invention features targeting moieties optionally conjugated to one or more agents (*e.g*., one or more therapeutic agents) or a transport vector (*e.g*., a nanoparticle or a liposome), and use of such compounds in treatment and diagnosis of disease.

Disclosed is a purified polypeptide, or a pharmaceutically acceptable salt thereof, including the amino acid sequence Lys-Arg-X3-X4-X5-Lys (formula Ia), where X3 is Asn or Gln; X4 is Asn or Gln; and X5 is Phe, Tyr, or Trp; where the polypeptide is fewer than 200 amino acids in length (*e.g*., fewer than 150, 100, 75, 50, 45, 40, 35, 30, 25, 20, 19, 18, 17, 16, 15, 14, 12, 10, 11, 8, or 7 amino acids, or any range between these numbers); where the polypeptide optionally includes one or more D-isomers of an amino acid recited in formula Ia (*e.g*., a D-isomer of Lys, Arg, X3, X4, X5, or Lys); and where the polypeptide is not a peptide in Table 2.

The invention relates to a purified polypeptide, or a pharmaceutically acceptable salt thereof, including the amino acid sequence Lys-Arg-X3-X4-X5-Lys (formula Ia), where X3 is Asn or Gln; X4 is Asn or Gln; and X5 is Phe; where the polypeptide is fewer than 19 amino acids in length (*e.g*., fewer than 18, 17, 16, 15, 14, 12, 10, 11, 8, or 7 amino acids, or any range between these numbers); and where the polypeptide optionally includes one or more D-isomers of an amino acid recited in formula Ia (*e.g*., a D-isomer of Lys, Arg, X3, X4, X5, or Lys), and wherein the following is not claimed:
- a polypeptide which is Ala-Lys-Arg-Asn-Asn-Phe-Lys-Ser,
- a polypeptide which is Cys-Arg-Ala-Lys-Arg-Asn-Asn-Phe-Lys-Ser-Ala.

In any of the polypeptides above, additions of 1, 2, 3, 4, or 5 amino acids (e.g., from 1 to 3 amino acids) maybe made from the amino acid sequence Lys-Arg-X3-X4-X5-Lys.

In some embodiments of the invention, the amino acid sequence is Z1-Lys-Arg-X3-X4-X5-Lys-Z2 (formula Ib), where X3 is Asn or Gln; X4 is Asn or Gln; X5 is Phe; Z1 is absent, Cys, Gly, Cys-Gly, Arg-Gly, Cys-Arg-Gly, Ser-Arg-Gly, Cys-Ser-Arg-Gly, Gly-Ser-Arg-Gly, Cys-Gly-Ser-Arg-Gly, Gly-Gly-Ser-Arg-Gly, Cys-Gly-Gly-Ser-Arg-Gly, Tyr-Gly-Gly-Ser-Arg-Gly, Cys-Tyr-Gly-Gly-Ser-Arg-Gly, Phe-Tyr-Gly-Gly-Ser-Arg-Gly, Cys-Phe-Tyr-Gly-Gly-Ser-Arg-Gly, Phe-Phe-Tyr-Gly-Gly-Ser-Arg-Gly, Cys-Phe-Phe-Tyr-Gly-Gly-Ser-Arg-Gly, Thr-Phe-Phe-Tyr-Gly-Gly-Ser-Arg-Gly, or Cys-Thr-Phe-Phe-Tyr-Gly-Gly-Ser-Arg-Gly; and Z2 is absent, Cys, Tyr, Tyr-Cys, Cys-Tyr, Thr-Glu-Glu-Tyr, or Thr-Glu-Glu-Tyr-Cys; and where the polypeptide optionally comprises one or more D-isomers of an amino acid recited in formula Ib, Z1, or Z2.

In other embodiments of the invention, the polypeptide has one or more additional cysteine residues at the N-terminal of the polypeptide, the C-terminal of the polypeptide, or both. In other embodiments, the polypeptide has one or more additional tyrosine residues at the N-terminal of the polypeptide, the C-terminal of the polypeptide, or both. In yet further embodiments, the polypeptide has the amino acid sequence Tyr-Cys and/or Cys-Tyr at the N-terminal of the polypeptide, the C-terminal of the polypeptide, or both.

In certain embodiments, the amino acid sequence is Lys-Arg-Asn-Asn-Phe-Lys. In other embodiments, the amino acid sequence is Lys-Arg-Asn-Asn-Phe-Lys-Tyr. In yet other embodiments, the amino acid sequence is Lys-Arg-Asn-Asn-Phe-Lys-Tyr-Cys.

In particular embodiments, the amino acid sequence is Xl-X2-Asn-Asn-X5-X6 (formula IIa), where X1 is Lys or D-Lys; X2 is Arg or D-Arg; X5 is Phe or D-Phe; and X6 is Lys or D-Lys; and where at least one (*e.g*., at least two, three, or four) of X1, X2, X5, or X6 is a D-amino acid.

In other embodiments , the amino acid sequence is X1-X2-Asn-Asn-X5-X6-X7 (formula IIb), where X1 is Lys or D-Lys; X2 is Arg or D-Arg; X5 is Phe or D-Phe; X6 is Lys or D-Lys; and X7 is Tyr or D-Tyr; and where at least one (*e.g.*, at least two, three, four, or five) of X1, X2, X5, X6, or X7 is a D-amino acid.

In some embodiments , the amino acid sequence is Z1-Lys-Arg-X3-X4-X5-Lys-Z2 (formula Ib), where X3 is Asn or Gln; X4 is Asn or Gln; X5 is Phe; Z1 is absent, Cys, Gly, Cys-Gly, Arg-Gly, Cys-Arg-Gly, Ser-Arg-Gly, Cys-Ser-Arg-Gly, Gly-Ser-Arg-Gly, Cys-Gly-Ser-Arg-Gly, Gly-Gly-Ser-Arg-Gly, Cys-Gly-Gly-Ser-Arg-Gly, Tyr-Gly-Gly-Ser-Arg-Gly, Cys-Tyr-Gly-Gly-Ser-Arg-Gly, Phe-Tyr-Gly-Gly-Ser-Arg-Gly, Cys-Phe-Tyr-Gly-Gly-Ser-Arg-Gly, Phe-Phe-Tyr-Gly-Gly-Ser-Arg-Gly, Cys-Phe-Phe-Tyr-Gly-Gly-Ser-Arg-Gly, Thr-Phe-Phe-Tyr-Gly-Gly-Ser-Arg-Gly, or Cys-Thr-Phe-Phe-Tyr-Gly-Gly-Ser-Arg-Gly; and Z2 is absent, Cys, Tyr, Tyr-Cys, Cys-Tyr, Thr-Glu-Glu-Tyr, or Thr-Glu-Glu-Tyr-Cys; where at least one of X1, X2, X5, X6, or X7 is a D-amino acid; and where the polypeptide optionally comprises one or more D-isomers of an amino acid recited in Z1 or Z2.

In further embodiments of any of the above aspects, the polypeptide is fewer than 15 amino acids in length (e.g., fewer than 10 amino acids in length).

In other embodiments of any of the above aspects, the polypeptide is Phe-Tyr-Gly-Gly-Ser-Arg-Gly-Lys-Arg-Asn-Asn-Phe-Lys-Thr-Glu-Glu-Tyr-Cys (P1); Phe-Tyr-Gly-Gly-Ser-Arg-Gly-D-Lys-D-Arg-Asn-Asn-D-Phe-Lys-Thr-Glu-Glu-Tyr-Cys (P1a); Phe-Tyr-Gly-Gly-Ser-Arg-Gly-D-Lys-D-Arg-Asn-Asn-D-Phe-D-Lys-Thr-Glu-Glu-Tyr-Cys (P1b); Phe-Tyr-Gly-Gly-Ser-Arg-Gly-D-Lys-D-Arg-Asn-Asn-D-Phe-D-Lys-Thr-Glu-Glu-D-Tyr-Cys (P1c); D-Phe-D-Tyr-Gly-Gly-Ser-D-Arg-Gly-D-Lys-D-Arg-Asn-Asn-D-Phe-D-Lys-Thr-Glu-D-Glu-D-Tyr-Cys (P1d); Gly-Gly-Ser-Arg-Gly-Lys-Arg-Asn-Asn-Phe-Lys-Thr-Glu-Glu-Tyr-Cys (P2); Ser-Arg-Gly-Lys-Arg-Asn-Asn-Phe-Lys-Thr-Glu-Glu-Tyr-Cys (P3); Gly-Lys-Arg-Asn-Asn-Phe-Lys-Thr-Glu-Glu-Tyr-Cys (P4); Lys-Arg-Asn-Asn-Phe-Lys-Thr-Glu-Glu-Tyr-Cys (P5); D-Lys-D-Arg-Asn-Asn-D-Phe-Lys-Thr-Glu-Glu-Tyr-Cys (P5a); D-Lys-D-Arg-Asn-Asn-D-Phe-D-Lys-Thr-Glu-Glu-Tyr-Cys (P5b); D-Lys-D-Arg-Asn-Asn-D-Phe-D-Lys-Thr-Glu-Glu-D-Tyr-Cys (P5c); Lys-Arg-Asn-Asn-Phe-Lys-Tyr-Cys (P6); D-Lys-D-Arg-Asn-Asn-D-Phe-Lys-Tyr-Cys (P6a); D-Lys-D-Arg-Asn-Asn-D-Phe-D-Lys-Tyr-Cys (P6b); and D-Lys-D-Arg-Asn-Asn-D-Phe-D-Lys-D-Tyr-Cys (P6c)

Disclosed is that the polypeptide includes a sequence having from 0 to 5 (*e.g*., from 0 to 4, 0 to 3, 0 to 2, 0 to 1, 1 to 5, 1 to 4, 1 to 3, 1 to 2,2 to 5, 2 to 4, 2 to 3, 3 to 5, 3 to 4, or 4 to 5) substitutions, deletions, or additions of amino acids relative to one or more sequence selected from Thr-Phe-Phe-Tyr-Gly-Gly-Ser-D-Arg-Gly-D-Lys-D-Arg-Asn-Asn-Phe-Lys-Thr-Glu-Glu-Tyr (3D-An2); Phe-Tyr-Gly-Gly-Ser-Arg-Gly-Lys-Arg-Asn-Asn-Phe-Lys-Thr-Glu-Glu-Tyr-Cys (P1); Phe-Tyr-Gly-Gly-Ser-Arg-Gly-D-Lys-D-Arg-Asn-Asn-D-Phe-Lys-Thr-Glu-Glu-Tyr-Cys (P1a); Phe-Tyr-Gly-Gly-Ser-Arg-Gly-D-Lys-D-Arg-Asn-Asn-D-Phe-D-Lys-Thr-Glu-Glu-Tyr-Cys (P1b); Phe-Tyr-Gly-Gly-Ser-Arg-Gly-D-Lys-D-Arg-Asn-Asn-D-Phe-D-Lys-Thr-Glu-Glu-D-Tyr-Cys (P1c); D-Phe-D-Tyr-Gly-Gly-Ser-D-Arg-Gly-D-Lys-D-Arg-Asn-Asn-D-Phe-D-Lys-Thr-Glu-D-Glu-D-Tyr-Cys (P1d); Gly-Gly-Ser-Arg-Gly-Lys-Arg-Asn-Asn-Phe-Lys-Thr-Glu-Glu-Tyr-Cys (P2); Ser-Arg-Gly-Lys-Arg-Asn-Asn-Phe-Lys-Thr-Glu-Glu-Tyr-Cys (P3); Gly-Lys-Arg-Asn-Asn-Phe-Lys-Thr-Glu-Glu-Tyr-Cys (P4); Lys-Arg-Asn-Asn-Phe-Lys-Thr-Glu-Glu-Tyr-Cys (P5); D-Lys-D-Arg-Asn-Asn-D-Phe-Lys-Thr-Glu-Glu-Tyr-Cys (P5a); D-Lys-D-Arg-Asn-Asn-D-Phe-D-Lys-Thr-Glu-Glu-Tyr-Cys (P5b); D-Lys-D-Arg-Asn-Asn-D-Phe-D-Lys-Thr-Glu-Glu-D-Tyr-Cys (P5c); Lys-Arg-Asn-Asn-Phe-Lys-Tyr-Cys (P6); D-Lys-D-Arg-Asn-Asn-D-Phe-Lys-Tyr-Cys (P6a); D-Lys-D-Arg-Asn-Asn-D-Phe-D-Lys-Tyr-Cys (P6b); and D-Lys-D-Arg-Asn-Asn-D-Phe-D-Lys-D-Tyr-Cys (P6c); or a fragment thereof.

In some embodiments of any of the invention, the polypeptide is Phe-Tyr-Gly-Gly-Ser-Arg-Gly-Lys-Arg-Asn-Asn-Phe-Lys-Thr-Glu-Glu; Gly-Gly-Ser-Arg-Gly-Lys-Arg-Asn-Asn-Phe-Lys-Thr-Glu-Glu; Ser-Arg-Gly-Lys-Arg-Asn-Asn-Phe-Lys-Thr-Glu-Glu; Gly-Lys-Arg-Asn-Asn-Phe-Lys-Thr-Glu-Glu; Lys-Arg-Asn-Asn-Phe-Lys-Thr-Glu-Glu; or Lys-Arg-Asn-Asn-Phe-Lys

In yet other embodiments, the polypeptide is Phe-Tyr-Gly-Gly-Ser-Arg-Gly-Lys-Arg-Asn-Asn-Phe-Lys-Thr-Glu-Glu-Tyr-Cys (P1); Phe-Tyr-Gly-Gly-Ser-Arg-Gly-D-Lys-D-Arg-Asn-Asn-D-Phe-Lys-Thr-Glu-Glu-Tyr-Cys (P1a); Phe-Tyr-Gly-Gly-Ser-Arg-Gly-D-Lys-D-Arg-Asn-Asn-D-Phe-D-Lys-Thr-Glu-Glu-Tyr-Cys (P1b); Phe-Tyr-Gly-Gly-Ser-Arg-Gly-D-Lys-D-Arg-Asn-Asn-D-Phe-D-Lys-Thr-Glu-Glu-D-Tyr-Cys (P1c); D-Phe-D-Tyr-Gly-Gly-Ser-D-Arg-Gly-D-Lys-D-Arg-Asn-Asn-D-Phe-D-Lys-Thr-Glu-D-Glu-D-Tyr-Cys (P1d) or a fragment thereof having a deletion of 1 to 7 amino acids from the N-terminus of P1, P1a, P1b, P1c, or P1d; a deletion of 1 to 5 amino acids from the C-terminus of P1, P1a, P1b, P1c, or Pld; or deletions of 1 to 7 amino acids from the N-terminus of P1, P1a, P1b, P1c, or P1d and 1 to 5 amino acids from the C-terminus of P1, P1a, P1b, P1c, or P1d.

In any of the above aspects of the invention, the polypeptide may have a C-terminus that is amidated. In other embodiments, the polypeptide is efficiently transported across the blood-brain barrier (e.g., the polypeptide is transported across the blood-brain barrier more efficiently than Angiopep-2).

In a third aspect, the invention features a therapeutic polypeptide including a targeting moiety consisting of a polypeptide of the invention and a therapeutic peptidic agent, where the targeting moiety is linked to the therapeutic peptidic agent. In certain embodiments, the targeting moiety is linked to the therapeutic agent by a covalent bond (*e.g.*, a peptide bond). In other embodiments, the therapeutic polypeptide is a fusion protein. In particular embodiments, the therapeutic polypeptide includes one or more therapeutic peptidic agents.

The targeting moiety may be heterologous with respect to the therapeutic peptidic agent.

In particular embodiments of the invention, the therapeutic peptidic agent is neurotensin or a neurotensin analog (*e.g*., neurotensin(6-13), neurotensin(8-13), Lys(7)-D-Tyr(11)-neurotensin(7-13), p-Glu(1)-neurotensin, p-Glu(1)-neurotensin-OH, D-Lys(6)-neurotensin(6-13), D-Tyr(11)-neurotensin(6-13), D-Lys(6)-D-Tyr(11)-neurotensin(6-13), D-Arg(8)-neurotensin(6-13), D-Arg(9)-neurotensin(6-13), D-Arg(8)-D-Arg(9)-neurotensin(6-13), D-Pro(10)neurotensin(6-13), D-Tyr(1)-neurotensin(6-13), D-Trp(11)-neurotensin(6-13), D-Phe(11)-neurotensin(6-13), D-Arg(8)-D-Tyr(11)-neurotensin(6-13), D-Arg(8)-D-Trp(11)-neurotensin(6-13), D-Arg(8)-neurotensin(8-13), D-Arg(9)-neurotensin(8-13), D-Arg(8)-D-Arg(9)-neurotensin(8-13), D-Pro(10)neurotensin(8-13), D-Tyr(11)-neurotensin(8-13), D-Trp(11)-neurotensin(8-13), D-Phe(11)-neurotensin(8-13), D-Arg(8)-D-Tyr(11)-neurotensin(8-13), and D-Arg(8)-D-Trp(11)-neurotensin(8-13), or an acetylated form thereof, or any described herein, such as acetyl-Lys(7)-D-Tyr(11)-neurotensin(7-13)), a neurotensin receptor agonist, a neurotrophic factor or a neurotrophic factor analog (*e*.*g.*, a neuroglial-derived neurotrophic factor (GDNF) or a GDNF analog, or brain-derived neurotrophic factor (BDNF) or a BDNF analog), a GLP-1 agonist, or leptin or a leptin analog.

In other embodiments of the invention, the therapeutic peptidic agent is a polypeptide that specifically binds a biological molecule, such as an immunoglobulin or a fragment thereof that retains the ability to specifically bind the biological molecule. For example, the immunoglobulin can be a tetrameric antibody or a single-chain antibody.

The invention features a conjugate having the formula A-X-B, where A is a targeting moiety of any of the polypeptides of the invention; X is a linker; and B is a therapeutic agent or a transport vector. In particular embodiments, the conjugate has the formula A-(X-B)ₙ or A-X-(B)ₙ, wherein n is an integer of one or more (*e.g*., 1,2, 3,4,5, 6, 7, 8,9, or 10). In certain embodiments, n is an integer of two or more (e.g., 3, 4, 5, 6, 7, 8, 9, or 10). In some embodiments, n is an integer from 1 to 10 (e.g., from 1 to 9, from 1 to 8, from 1 to 7, from 1 to 6, from 1 to 5, from 1 to 4, from 1 to 3, from 2 to 9, from 2 to 8, from 2 to 7, from 2 to 6, from 2 to 5, from 2 to 4, from 3 to 9, from 3 to 8, from 3 to 7, from 3 to 6, from 3 to 5, or from 3 to 4).

In some embodiments of the fourth aspect, X is a peptide bond. In other embodiments, X is at least one amino acid; and A and B are each covalently bonded to X by a peptide bond. In certain embodiments, X is an ester linker. In some embodiments, X has the formula -NH-(CH₂)ₙ-C(O)O-, where n is an integer between 2 and 10 (e.g., where n is an integer between 2 and 5, 2 and 6, 2 and 7, 2 and 8, 2 and 9, 2 and 10, 3 and 5, 3 and 6, 3 and 7, 3 and 8, 3 and 9, 3 and 10, and 5, and 6, and 7, 4 and 8, 4 and 9, 4 and 10, 5 and 6, 5 and 7, 5 and 8, 5 and 9, and 5 and 10, such as when n is 5 for aminohexanoic acid (Ahx)). In certain embodiments, X has the formula: 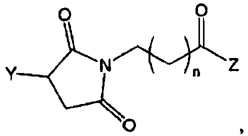 where n is an integer between 2 and 15 (*e.g*., n is 3, 6, or 11); and either Y is a thiol on A and Z is a primary amine on B or Y is a thiol on B and Z is a primary amine on A.

In certain embodiments of the invention, B is the therapeutic agent, such as an anticancer agent (*e.g*., paclitaxel (Taxol®) or a paclitaxel derivative, such as docetaxel (Taxotere®); etoposide; doxorubicin; or an analog thereof), a therapeutic nucleic acid agent (*e.g*., an RNAi agent, such as siRNA, dsRNA, miRNA, shRNA or ptgsRNA), a small molecule drug (*e.g*., an antibiotic, an antiproliferative agent, or a growth factor inhibitor), a label (*e.g*., an isotope, a radioimaging label, a fluorescent label, or a reporter molecule), or a therapeutic peptidic agent. In particular embodiments, the conjugate has one or more B or therapeutic agents. In certain embodiments, the therapeutic agent is the therapeutic peptidic agent, and the conjugate is a fusion protein. In yet further embodiments, the therapeutic peptidic agent is selected from neurotensin or a neurotensin analog (*e.g*., neurotensin(6-13), neurotensin(8-13), Lys(7)-D-Tyr(11)-neurotensin(7-13), p-Glu(1)-neurotensin, p-Glu(1)-neurotensin-OH, D-Lys(6)-neurotensin(6-13), D-Tyr(11)-neurotensin(6-13), D-Lys(6)-D-Tyr(11)-neurotensin(6-13), D-Arg(8)-neurotensin(6-13), D-Arg(9)-neurotensin(6-13), D-Arg(8)-D-Arg(9)-neurotensin(6-13), D-Pro(10)neurotensin(6-13), D-Tyr(11)-neurotensin(6-13), D-Trp(11)-neurotensin(6-13), D-Phe(11)-neurotensin(6-13), D-Arg(8)-D-Tyr(11)-neurotensin(6-13), D-Arg(8)-D-Trp(11)-neurotensin(6-13), D-Arg(8)-neurotensin(8-13), D-Arg(9)-neumtensin(8-13), D-Arg(8)-D-Arg(9)-neurotensin(8-13), D-Pro(10)neurotensin(8-13), D-Tyr(11)-neurotensin(8-13), D-Trp(11)-neurotensin(8-13), D-Phe(11)-neurotensin(8-13), D-Arg(8)-D-Tyr(11)-neurotensin(8-13), and D-Arg(8)-D-Trp(11)-neurotensin(8-13), or an acetylated form thereof, or any described herein, such as acetyl-Lys(7)-D-Tyr(11)-neurotensin(7-13)), a neurotrophic factor or a neurotrophic factor analog (*e*.*g*., glial cell line-derived neurotrophic factor (GDNF) or a GDNF analog, and brain-derived neurotrophic factor (BDNF) or a BDNF analog)), a GLP-1 agonist, and leptin or a leptin analog.

In certain embodiments of the invention, B is the transport vector, such as a lipid vector (*e.g*., a liposome, a micelle, or a lipoplex), a polyplex, a dendrimer, or a nanoparticle (*e.g*., a polymeric nanoparticle, a solid lipid nanoparticle, or a nanometer-sized micelle). In other embodiments, the transport vector is bound to or contains a therapeutic agent (*e.g*., an anticancer agent, a therapeutic nucleic acid agent, a small molecule drug, a label, and a therapeutic peptidic agent).

In other embodiments , the therapeutic peptidic agent is a polypeptide that specifically binds a biological molecule, such as an immunoglobulin or a fragment thereof that retains the ability to specifically bind the biological molecule. For example, the immunoglobulin can be a tetrameric antibody or a single-chain antibody.

The invention features a composition including any therapeutic polypeptide or any conjugate described above. In some embodiments, the composition further includes a pharmaceutically acceptable carrier.

Disclosed is a method of treating (*e.g*., prophylactically) a subject (*e.g*., a human) in need of treatment, including a subject having cancer. The methods can include the step of administering to the subject an effective amount of a therapeutic polypeptide or conjugate of the invention (*e.g*., a polypeptide or conjugate including, as a therapeutic peptidic agent, an immunoglobulin or a fragment thereof that specifically binds a biological molecule). Cancers include brain cancer (*e.g*., glioma, mixed glioma, glioblastoma multiforme, astrocytoma, pilocytic astrocytoma, dysembryoplastic neuroepithelial tumor, oligodendroglioma, ependymoma, oligoastrocytoma, medullo-blastoma, retinoblastoma, neuroblastoma, germinoma, and teratoma). The subject may have been diagnosed as having a cancer or determined to be at high risk of developing a cancer.

The invention features any therapeutic polypeptide or conjugate described herein for use in the treatment or the prophylactic treatment of cancer in a subject. Disclosed is the use of any therapeutic polypeptide or conjugate described herein in the manufacture of a medicament for treating (*e*.*g*., prophylactically) cancer in a subject. Cancers include brain cancer (*e.g*., glioma, mixed glioma, glioblastoma multiforme, astrocytoma, pilocytic astrocytoma, dysembryoplastic neuroepithelial tumor, oligodendroglioma, ependymoma, oligoastrocytoma, medulloblastoma, retinoblastoma, neuroblastoma, germinoma, and teratoma).

Disclosed is a method of reducing body temperature of a subject by administering a therapeutic polypeptide or a conjugate of the invention in a sufficient amount to reduce body temperature. The subject can be suffering from or has suffered from stroke, heart attack, cerebral ischemia, cardiac ischemia, a nerve injury or is in need of neuroprotection, or malignant hypothermia.

Disclosed is any therapeutic polypeptide or conjugate described herein for use in reducing body temperature of a subject.

Disclosed is the use of any therapeutic polypeptide or conjugate described herein in the manufacture of a medicament for reducing body temperature of a subject. The subject can be suffering from or has suffered from stroke, heart attack, cerebral ischemia, cardiac ischemia, a nerve injury or is in need of neuroprotection, or malignant hypothermia.

Disclosed is a method of treating (*e.g*., prophylactically) hypertension in a subject by administering to the subject an effective amount of a therapeutic polypeptide or conjugate of the invention.

Disclosed is any therapeutic polypeptide or conjugate described herein for use in the treatment or the prophylactic treatment of hypertension in a subject. Disclosed is the use of any therapeutic polypeptide or conjugate described herein in the manufacture of a medicament for treating (*e.g*., prophylactically) hypertension in a subject.

Disclosed is a method of treating (*e*.*g*., prophylactically) pain or decreasing sensitivity to pain in a subject by administering a therapeutic polypeptide or a conjugate of the invention in a sufficient amount to treat the pain. The pain can be acute pain, peripheral or central neuropathic pain, inflammatory pain, migraine-related pain, headache-related pain, irritable bowel syndrome-related pain, fibromyalgia-related pain, arthritic pain, skeletal pain, joint pain, gastrointestinal pain, muscle pain, angina pain, facial pain, pelvic pain, claudication, postoperative pain, post traumatic pain, tension-type headache, obstetric pain, gynecological pain, or chemotherapy-induced pain.

In particular embodiments, the invention features a therapeutic polypeptide or conjugate of the invention for use in the treatment or the prophylactic treatment of pain or for use in decreasing the sensitivity to pain in a subject. Disclosed is the use of any therapeutic polypeptide or conjugate described herein in the manufacture of a medicament for treating (*e.g*., prophylactically) pain or decreasing sensitivity to pain in a subject. The pain can be acute pain, peripheral or central neuropathic pain, inflammatory pain, migraine-related pain, headache-related pain, irritable bowel syndrome-related pain, fibromyalgia-related pain, arthritic pain, skeletal pain, joint pain, gastrointestinal pain, muscle pain, angina pain, facial pain, pelvic pain, claudication, postoperative pain, post traumatic pain, tension-type headache, obstetric pain, gynecological pain, or chemotherapy-induced pain.

Disclosed is treating (e.g., prophylactically) a subject having a disorder by administering a therapeutic polypeptide or a conjugate of the invention in a sufficient amount to treat the disorder. These disorders include a psychotic disorder (*e*.*g*., schizophrenia or any other psychotic disorder described herein), drug addiction or drug abuse (*e.g*., addiction or abuse of amphetamine, methamphetamine, 3,4-methylenedioxy-methamphetamine, nicotine, cocaine, methylphenidate, or arecoline), a metabolic disorder (*e.g*., diabetes (*e*.*g*., Type I or Type II), obesity, diabetes as a consequence of obesity, hyperglycemia, dyslipidemia, hypertriglyceridemia, syndrome X, insulin resistance, impaired glucose tolerance (IGT), diabetic dyslipidemia, hyperlipidemia, a cardiovascular disease, or hypertension), or a neurological disorder (*e.g*., a neurodegenerative disease; a condition of the central nervous system (CNS), the peripheral nervous system, or the autonomous nervous system; or any neurological disorder described herein) in a subject.

In particular embodiments, the invention features a therapeutic polypeptide or conjugate of the invention for use in the treatment or the prophylactic treatment of a disorder in a subject. Disclosed is the use of any therapeutic polypeptide or conjugate described herein in the manufacture of a medicament for treating (*e.g*., prophylactically) a disorder in a subject. These disorders include a psychotic disorder (*e.g*., schizophrenia or any other psychotic disorder described herein), drug addiction or drug abuse (*e.g*., addiction or abuse of amphetamine, methamphetamine, 3,4-methylenedioxy-methamphetamine, nicotine, cocaine, methylphenidate, or arecoline), a metabolic disorder (*e.g*., diabetes (*e.g*., Type I or Type II), obesity, diabetes as a consequence of obesity, hyperglycemia, dyslipidemia, hypertriglyceridemia, syndrome X, insulin resistance, impaired glucose tolerance (IGT), diabetic dyslipidemia, hyperlipidemia, a cardiovascular disease, or hypertension), or a neurological disorder (e.g., a neurodegenerative disease; a condition of the central nervous system (CNS), the peripheral nervous system, or the autonomous nervous system; or any neurological disorder described herein) in a subject.

In any of the above methods or use, the subject may be a human.

In the treatment methods or use of the invention, the conjugate or therapeutic polypeptide may be administered at a lower (*e.g*., less than 95%, 75%, 60%, 50%, 40%, 30%, 25%, 10%, 5%, or 1%) equivalent dosage as compared to the recommended dosage of the unconjugated therapeutic agent or transport vector (*e.g*., bound to or containing a therapeutic agent). In other embodiments, the conjugate or therapeutic polypeptide is administered at a higher (1.5x, 2x, 2.5x, 3.0x, 5x, 8x, 10x 15x, 20x, 25x) equivalent dosage than a dosage recommended for the unconjugated agent.

The targeting moiety, conjugate, or therapeutic polypeptide of the invention may be efficiently transported into a particular cell type, organ, or tissue (*e*.*g*., any one, two, three, four, or five of liver, eye, lung, kidney, spleen, muscle, or ovary cell type, organ, or tissue) or may cross the mammalian BBB. In accordance with the present invention, the targeting moiety may promote accumulation of a therapeutic agent in a tissue such as, for example, a liver (liver tissue), an eye (eye tissue), the lungs (lung tissue), a kidney (kidney tissue), a spleen (spleen tissue), muscle (muscle tissue), and ovary (ovary tissue) of a subject. Accordingly, the targeting moiety, conjugate, or therapeutic polypeptide may be used to treat a disease associated with these tissues (*e.g*., a cancer, such as any described herein; an infection, such as a bacterial infection or a viral infection; or an inflammatory condition). The targeting moiety may be any length fewer than 19, 18, 17, 16, 15, 14, 12, 10, 11, 8, or 7 amino acids, or any range between these numbers. The targeting moiety may be any length fewer than 19 amino acids, for example, fewer than 18, 17, 16, 15, 14, 12, 10, 11, 8, or 7 amino acids, or any range between these numbers.

The targeting moiety may be produced by recombinant genetic technology or chemical synthesis.

The therapeutic polypeptide, conjugate, or targeting moiety includes a polypeptide shorter than Angiopep-2 (An2) having one or more D-amino acid substitutions for one or more of positions 1, 2, 3, 4, 8, 10, 11, 13, 14, 15, 16, 17, 18, and 19 in Angiopep-2 (SEQ ID NO:97). In further embodiments, the therapeutic polypeptide, conjugate, or targeting moiety has any length fewer than 19, 18, 17, 16, 15, 14, 12, 10, 11, 8, or 7 amino acids, i.e. any length fewer than 19 amino acids, such as fewer than 18, 17, 16, 15, 14, 12, 10, 11, 8, or 7 amino acids, or any range between these numbers.

In any of the above aspects, the therapeutic polypeptide, conjugate, or targeting moiety is efficiently transported across the blood-brain barrier (*e.g*., the therapeutic polypeptide, conjugate, or targeting moiety is transported across the blood-brain barrier more efficiently than Angiopep-2 or Angiopep-2 conjugated to a therapeutic peptidic agent, any other therapeutic agent, or a transport vector).

In certain embodiments of any of the above aspects, the targeting moiety, conjugate, or therapeutic polypeptide described herein is modified (*e.g*., as described herein). The targeting moiety, conjugate, or therapeutic polypeptide may be amidated, acetylated, or both. Such modifications may be at the amino or carboxy terminus of the targeting moiety. The targeting moiety, conjugate, or therapeutic polypeptide may also include peptidomimetics (*e*.*g*., those described herein) of any of the peptides described herein. The targeting moiety, conjugate, or therapeutic polypeptide may also include one or more (*e.g*., 2, 3, 4, 5, 6, 7, 8, 9, 10, 11, 12, 13, 14, or 15) substitutions, deletions, or additions of amino acids relative to one of the sequences described herein. In particular, these substitutions, deletions, or additions of 1, 2, 3, 4, or 5 amino acids (*e.g*., from 1 to 3 amino acids) may be made from the amino acid sequence Lys-Arg-X3-X4-X5-Lys (formula Ia), Z1-Lys-Arg-X3-X4-X5-Lys-Z2 (formula Ib), X1-X2-Asn-Asn-X5-X6 (formula IIa), X1-X2-Asn-Asn-X5-X6-X7 (formula IIb), or Z1-X1-X2-Asn-Asn-X5-X6-X7-Z2 (formula IIc), where X1-X7, Z1, and Z2 for each formula are described herein. Other modifications include posttranslational processing or by chemical modification, including ubiquitination, pegylation, acetylation, acylation, cyclization, amidation, oxidation, sulfation, formation of cysteine, or covalent attachment of one or more therapeutic agents. In particular, cyclization may be a preferred modification.

It is disclosed that the targeting moiety, conjugate, or therapeutic polypeptide described herein is multimeric (*e.g*., dimeric, trimeric, or higher order multimeric, or as described herein). The targeting moiety may be a multimeric targeting moiety (*e.g*., as described herein). The conjugate may include multimeric targeting moieties and include one or more therapeutic agents or one or more transport vectors (*e.g*., as described herein). It is disclosed that multimeric targeting moieties and conjugates include any of modifications or further conjugations described herein for polypeptides (*e.g*., posttranslational processing or by chemical modification, including ubiquitination, pegylation, acetylation, acylation, cyclization, amidation, oxidation, sulfation, formation of cysteine, or covalent attachment of one or more therapeutic agents).

In certain embodiments of any of the above aspects, the linkers can be monovalent or polyvalent (e.g., homomultifunctional, heteromultifunctional, bifunctional, or trifunctional agents). In other embodiments, the linkers can include a flexible arm (*e.g*., 2, 3, 4, 5, 6, 7, 8, 9, 10, 11, 12, 13, 14, or 15 carbon atoms; or a polyethylene glycol spacer, such as (PEG)ₙ, where n is 1-20).

### Definitions

By "conjugate" is meant a compound having a targeting moiety and a therapeutic agent or a transport vector (e.g., any described herein) linked to the targeting moiety.

By a targeting moiety which is "transported across the blood-brain barrier" is meant a targeting moiety that is able to cross the BBB (*e.g*., 25%, 50%, 100%, 200%, 500%, 1,000%, 5,000%, or 10,000%) greater extent than either a control substance, or, in the case of a conjugate, as compared to the unconjugated agent. Ability to cross the BBB may be determined using any method known in the art (*e.g*., an *in vitro* model of the BBB or *in situ* brain perfusion as described in U.S. Patent No. 7,557,182).

By a targeting moiety, conjugate, or therapeutic polypeptide which is "efficiently transported to a particular cell type" is meant that the targeting moiety, conjugate, or therapeutic polypeptide is able to accumulate (*e*.*g*., either due to increased transport into the cell, decreased efflux from the cell, or a combination thereof) in that cell type to at least a 10% (*e.g*., 25%, 50%, 100%, 200%, 500%, 1,000%, 5,000%, or 10,000%) greater extent than either a control substance, or, in the case of a conjugate, as compared to the unconjugated agent. Such activities are described in detail in International Application Publication No. WO 2007/009229.

By "equivalent dosage" is meant the amount of a conjugate of the invention required to achieve the same molar amount of agent in the conjugate of the invention, as compared to the unconjugated molecule.

By "fragment" is meant a portion of a full-length amino acid or nucleic acid sequence (e.g., any sequence described herein). Fragments may include at least 4, 5, 6, 8, 10, 11, 12, 14, 15, 16, 17, 18, 20, 25, 30, 35, 40, 45, or 50 amino acids or nucleic acids of the full length sequence. A fragment may retain at least one of the biological activities of the full length protein.

By a targeting moiety "linked to" a therapeutic agent or a transport vector is meant a covalent or non-covalent interaction between the targeting moiety and the therapeutic agent or the transport vector. Non-covalent interactions include, but are not limited to, hydrogen bonding, ionic interactions among charged groups, electrostatic binding, van der Waals interactions, hydrophobic interactions among non-polar groups, lipophobic interactions, and LogP-based attractions.

By a "multimeric targeting moiety" is meant a polypeptide including more than one targeting moieties, where the targeting moiety can be the same or different.

By "RNAi agent" is meant any agent or compound that exerts a gene silencing effect by way of an RNA interference pathway. RNAi agents include any nucleic acid molecules that are capable of mediating sequence-specific RNAi, for example, a short interfering RNA (siRNA), double-stranded RNA (dsRNA), microRNA (miRNA), short hairpin RNA (shRNA), short interfering oligonucleotide, short interfering nucleic acid, short interfering modified oligonucleotide, chemically-modified siRNA, and post-transcriptional gene silencing RNA (ptgsRNA).

By "substantially identical" is meant a polypeptide or nucleic acid having at least or about 35%, 40%, 50%, 55%, 60%, 65%, 70%, 75%, 85%, 90%, 95%, or even 99% identity as compared to a reference amino acid or nucleic acid sequence. For polypeptides, the length of comparison sequences will generally be at least 4 (*e.g*., at least 5, 6, 7, 8, 9, 10, 11, 12, 13, 14, 15, 16, 17, 18, 19, 20, 25, 50, or 100) amino acids. It is to be understood herein that gaps may be found between the amino acids of sequences that are identical or similar to amino acids of the original polypeptide. The gaps may include no amino acids, one or more amino acids that are not identical or similar to the original polypeptide. Percent identity may be determined, for example, with an algorithm GAP, BESTFIT, or FASTA in the Wisconsin Genetics Software Package Release 7.0, using default gap weights.

By "subject" is meant a human or non-human animal (e.g., a mammal).

By "targeting moiety" is meant a polypeptide, polypeptide derivative, or peptidomimetic that is capable of transport across the BBB or into a particular cell type.

By "therapeutic agent" is meant an agent that is capable of being used in the treatment or prophylactic treatment of a disease or condition or in the diagnosis of a disease or a condition.

By "therapeutic nucleic acid agent" is meant a RNA-based or DNA-based therapeutic agent.

By "therapeutic peptidic agent" is meant a protein-based or peptide-based therapeutic agent.

By "therapeutic polypeptide" is meant a conjugate having a targeting moiety and a therapeutic peptidic agent linked to the targeting moiety.

By "transport vector" is meant any compound or composition (e.g., lipid, carbohydrate, polymer, or surfactant) capable of binding or containing a therapeutic agent. The transport vector may be capable of transporting the agent, such as a small molecule drug or therapeutic peptidic agent. Exemplary transport vectors include lipid micelles, liposomes, lipoplexes, dendrimers, and nanoparticles.

By "treating" a disease, disorder, or condition in a subject is meant reducing at least one sign or symptom of the disease, disorder, or condition by administrating a conjugate or therapeutic polypeptide to the subject.

By "prophylactically treating" a disease, disorder, or condition in a subject is meant reducing the frequency of occurrence or severity of (*e.g*., preventing) a disease, disorder or condition by administering to the subject a conjugate or therapeutic polypeptide to the subject prior to the appearance of a disease symptom or symptoms.

Recitation of an amino acid residue refers to a naturally occurring L-amino acid, unless otherwise specified.

### Brief Description of the Drawings

**Figure 1** is a graph showing *in situ* brain perfusion in mice for Angiopep-2 (An2), Phe-Tyr-Gly-Gly-Ser-Arg-Gly-Lys-Arg-Asn-Asn-Phe-Lys-Thr-Glu-Glu-Tyr-Cys (P1), and Lys-Arg-Asn-Asn-Phe-Lys-Thr-Glu-Glu-Tyr-Cys (P5).
**Figure 2** is a graph showing latency in the hot plate test in mice of the paw licking response in control mice (vehicle), mice receiving ANG2002 (NT-An2, 20 mg/kg, intravenously), mice receiving P5-NT (16 mg/kg, intravenously), and mice receiving P6-NT (14 mg/kg, intravenously).
**Figure 3** is a graph showing the effect of body temperature in mice upon administration of control (vehicle), ANG2002 (NT-An2, 20 mg/kg, intravenously), P5-NT (16 mg/kg, intravenously), and P6-NT (14 mg/kg, intravenously).
**Figure 4** shows pepsin and trypsin cleavage sites for Angiopep-2 and ANGP6a (P6a).
**Figure 5** is a graph showing a competitive binding assay of [³H]-neurotensin using human colon adenocarcinoma (HT29) cells.
**Figure 6** is a graph showing *in situ* brain perfusion in mice for ANG2002, P6-NT, and P6a-NT.
**Figure 7** is a graph showing the effect of body temperature in mice upon administration of control (PBS), AN2-NT (neurotensin conjugated to An2, 20 mg/kg, 4.683 µmol/kg, intravenously), P5-NT (4.683 µmol/kg, intravenously), and P5a-NT (4.683 µmol/kg, intravenously).
**Figure 8** is a graph showing the effect of body temperature in mice upon administration of control (PBS), AN2-NT (neurotensin conjugated to An2, 20 mg/kg, 4.683 µmol/kg, intravenously), P6-NT (4.683 µmol/kg, intravenously), and P6a-NT (4.683 µmol/kg, intravenously).
**Figure 9** shows pepsin and trypsin cleavage sites for An2-NT, P6a-NT(6-13) (ANGP6a-NT(6-13)), P6b-NT(6-13, D-Arg8) (ANGP6b-NT(6-13, D-Arg8)), and P6b-NT(6-13, D-Arg8, D-Tyr11) (ANGP6b-NT(6-13, D-Arg8, D-Tyr11)).
**Figure 10** is a graph showing *in situ* brain perfusion in mice for NT, P5a-NT1, P5b-NT1, P5c-NT1, and P6a-NT1.

### Detailed Description

We have now developed short polypeptides (*e*.*g*. 6-18 amino acids in length) that are able to cross the blood-brain barrier (BBB) or are able to enter particular cell types (*e.g*., liver, eye, lung, spleen, kidney, muscle, or ovary) with enhanced efficiency. We have also developed both short (*e*.*g*., 6-18 amino acids in length) and longer (*e.g*., 19 or more amino acids in length) polypeptides having one or more D-amino acids (*e.g*., 3D-An2). These polypeptides can transport an agent across the BBB or into particular cells and act as targeting moieties. In some cases, the targeting moiety is capable of crossing the BBB or entering particular cell types more efficiently, and in certain cases as described herein, far more efficiently, than a longer form of the same polypeptide that is 19 amino acids in length or longer. This increased efficiency in transport may allow for lower dosages of the conjugate as compared either to the unconjugated agent or to the agent conjugated to a longer form of the polypeptide. In other cases, by directing the agent more efficiently to its target tissue(s), the compounds of the invention may administered in higher dosages than either the unconjugated agent or the agent conjugated to a longer form of the polypeptide, as the greater targeting efficiency can reduce side effects. Compounds including such targeting moieties and their use in treatment of disease are described in detail below.

### Targeting moiety

The invention encompasses short polypeptides that are used as targeting moieties. The polypeptides of the invention include a consensus sequence (e.g., Lys-Arg-Asn-Asn-Phe-Lys) and conservative substitutions thereof and are fewer than 19 amino acids in length (*i.e*., 18 amino acids and shorter).

The targeting moiety may have one or more (*e*.*g*., 2, 3, 4, 5, 6, 7, 8, 9, 10, 11, 12, 13, 14, or 15) additions and deletions of amino acids relative to one of the sequences of the invention. Other modifications are described in greater detail below.

The targeting moieties of the invention include a consensus sequence of

Lys-Arg-X3-X4-X5-Lys (formula Ia),

where
X3 is Asn or Gln;
X4 is Asn or Gln; and
X5 is Phe,
wherein the targeting moiety is not:
- a polypeptide which is Ala-Lys-Arg-Asn-Asn-Phe-Lys-Ser,
- a polypeptide which is Cys-Arg-Ala-Lys-Arg-Asn-Asn-Phe-Lys-Ser-Ala.

The targeting moieties of the invention also include a consensus sequence of

Z1-Lys-Arg-X3-X4-X5-Lys-Z2 (formula Ib),

where
X3 is Asn or Gln;
X4 is Asn or Gln;
X5 is Phe;
Z1 is absent, Cys, Gly, Cys-Gly, Arg-Gly, Cys-Arg-Gly, Ser-Arg-Gly, Cys-Ser-Arg-Gly, Gly-Ser-Arg-Gly, Cys-Gly-Ser-Arg-Gly, Gly-Gly-Ser-Arg-Gly, Cys-Gly-Gly-Ser-Arg-Gly, Tyr-Gly-Gly-Ser-Arg-Gly, Cys-Tyr-Gly-Gly-Ser-Arg-Gly, Phe-Tyr-Gly-Gly-Ser-Arg-Gly, Cys-Phe-Tyr-Gly-Gly-Ser-Arg-Gly, Phe-Phe-Tyr-Gly-Gly-Ser-Arg-Gly, Cys-Phe-Phe-Tyr-Gly-Gly-Ser-Arg-Gly, Thr-Phe-Phe-Tyr-Gly-Gly-Ser-Arg-Gly, or Cys-Thr-Phe-Phe-Tyr-Gly-Gly-Ser-Arg-Gly; and
Z2 is absent, Cys, Tyr, Tyr-Cys, Cys-Tyr, Thr-Glu-Glu-Tyr, or Thr-Glu-Glu-Tyr-Cys.

The consensus sequence of formulas (Ia) and (Ib) include the amino acid sequence Lys-Arg-Asn-Asn-Phe-Lys and defined conservative substitutions. Conservative substitutions and derivatives of amino acids and peptides are well known in the art and can be determined by any useful methods (*e.g*., by using a substitution matrix or any other method described herein). A derivative of a targeting moiety disclosed herein includes a targeting moiety containing one or more conservative substitutions selected from the following groups or a subset of these groups: Ser, Thr, and Cys; Leu, Ile, and Val; Glu and Asp; Lys and Arg; Phe, Tyr, and Trp (*e*.*g*., Phe and Tyr); and Gln, Asn, Glu, Asp, and His (*e.g*., Gln and Asn). Conservative substitutions may also be determined by other methods, such as by the BLAST (Basic Local Alignment Search Tool) algorithm, the BLOSUM substitution matrix (*e.g*., BLOSUM 62 matrix), and PAM substitution matrix (*e.g*., PAM 250 matrix).

The consensus sequences also include those having one or more D-amino acid substitutions, where one or more amino acid residues of formula (Ia) or (Ib) are substituted with a corresponding D-isomer. D-amino acid substitutions may provide peptides having increased resistance to cleavage by digestive enzymes (*e.g*., pepsin and/or trypsin). For example, one or more of amino acids in formula (Ia) or (Ib) having possible cleavage sites by pepsin or trypsin can be substituted with the D-isomer of that amino acid. Exemplary cleavage sites in formula (Ia) or (Ib) by pepsin and trypsin include the bond that is N-terminal or C-terminal to position 1 for Lys; position 2 for Arg; position 5 for X5 being Phe;
and position 6 for Lys. Accordingly, the polypeptides of the invention also include those having one or more D-isomers for the amino acids recited at positions 1, 2, 5, and/or 6 of formula (Ia) or (Ib).

Other targeting moieties of the invention include a consensus sequence of

X1-X2-Asn-Asn-X5-X6 (formula IIa),

where
X1 is Lys or D-Lys;
X2 is Arg or D-Arg;
X5 is Phe or D-Phe; and
X6 is Lys or D-Lys; and
where at least one of X1, X2, X5, or X6 is a D-amino acid.

Yet other targeting moieties of the invention include a consensus sequence of

X1-X2-Asn-Asn-X5-X6-X7 (formula IIb),

where
X1 is Lys or D-Lys;
X2 is Arg or D-Arg;
X5 is Phe or D-Phe;
X6 is Lys or D-Lys;
X7 is Tyr or D-Tyr; and
where at least one of X1, X2, X5, X6, or X7 is a D-amino acid.

The targeting moieties of the invention also include a consensus sequence of

Z1-X1-X2-Asn-Asn-X5-X6-X7-Z2 (formula IIc),

where
X1 is Lys or D-Lys;
X2 is Arg or D-Arg;
X5 is Phe or D-Phe;
X6 is Lys or D-Lys;
X7 is Tyr or D-Tyr;
Z1 is absent, Cys, Gly, Cys-Gly, Arg-Gly, Cys-Arg-Gly, Ser-Arg-Gly, Cys-Ser-Arg-Gly, Gly-Ser-Arg-Gly, Cys-Gly-Ser-Arg-Gly, Gly-Gly-Ser-Arg-Gly, Cys-Gly-Gly-Ser-Arg-Gly, Tyr-Gly-Gly-Ser-Arg-Gly, Cys-Tyr-Gly-Gly-Ser-Arg-Gly, Phe-Tyr-Gly-Gly-Ser-Arg-Gly, Cys-Phe-Tyr-Gly-Gly-Ser-Arg-Gly, Phe-Phe-Tyr-Gly-Gly-Ser-Arg-Gly, Cys-Phe-Phe-Tyr-Gly-Gly-Ser-Arg-Gly, Thr-Phe-Phe-Tyr-Gly-Gly-Ser-Arg-Gly, or Cys-Thr-Phe-Phe-Tyr-Gly-Gly-Ser-Arg-Gly; and
Z2 is absent, Cys, Tyr, Tyr-Cys, Cys-Tyr, Thr-Glu-Glu-Tyr, or Thr-Glu-Glu-Tyr-Cys;
where at least one of X1, X2, X5, X6, or X7 is a D-amino acid; and
where the polypeptide optionally includes one or more D-isomers of an amino acid recited in Z1 or Z2.

Disclosed is that these consensus sequences also include conservative substitutions. Conservative substitutions and derivatives of amino acids and peptides are well known in the art and can be determined by any useful methods (*e.g*., by using a substitution matrix or any other method described herein). A derivative of a targeting moiety includes a targeting moiety containing one or more conservative substitutions selected from the following groups or a subset of these groups: Ser, Thr, and Cys; Leu, Ile, and Val; Glu and Asp; Lys and Arg; Phe, Tyr, and Trp (*e.g*., Phe and Tyr); and Gln, Asn, Glu, Asp, and His (*e*.*g*., Gln and Asn), Conservative substitutions may also be determined by other methods, such as by the BLAST (Basic Local Alignment Search Tool) algorithm, the BLOSUM substitution matrix (*e.g*., BLOSUM 62 matrix), and PAM substitution matrix (*e.g*., PAM 250 matrix).

The targeting moieties of the invention include additions of amino acids to the consensus sequence of Lys-Arg-X3-X4-X5-Lys (formula Ia), where X3-X5 are as defined above; the consensus sequences of X1-X2-Asn-Asn-X5-X6 and X1-X2-Asn-Asn-X5-X6-X7 (formulas IIa and IIb, respectively), where X1, X2, X5, X6, and X7 are as defined above. The additions can include any part of the consensus sequence of Lys-Arg-X3-X4-X5-Lys, X1-X2-Asn-Asn-X5-X6, X1-X2-Asn-Asn-X5-X6-X7, Lys-Arg-Asn-Asn-Phe-Lys, D-Lys-D-Arg-Asn-Asn-D-Phe-D-Lys, or D-Lys-D-Arg-Asn-Asn-D-Phe-D-Lys-D-Tyr.

In some embodiments, additions of 1, 2, 3, 4, or 5 amino acids may be made from the consensus sequence of the targeting moiety. In particular embodiments, the additions may be from 1 to 3 amino acids.

Useful substitutions, additions, and deletions do not destroy significantly a desired biological activity (e.g., ability to cross the BBB or agonist activity). The modification may reduce (*e.g*., by at least 5%, 10%, 20%, 25%, 35%, 50%, 60%, 70%, 75%, 80%, 90%, or 95%), may have no effect, or may increase (*e.g*., by at least 5%, 10%, 25%, 50%, 100%, 200%, 500%, or 1000%) the biological activity of the original polypeptide.

In particular, substitutions of D-amino acids can be made within the targeting moiety. Such substitutions may provide peptides having increased resistance to cleavage by enzymes, where one of more amino acids for cleavage sites can be substituted with its D-isomer. Exemplary enzymes include pepsin, trypsin, Arg-C proteinase, Asp-N endopeptidase, chymotrypsin, glutamyl endopeptidase, LysC lysyl endopeptidase, LysN peptidyl-Lys metalloendopeptidase, proteinase K, and thermolysin; and exemplary cleavage sites for these enzymes are described herein.

Furthermore, substitutions, additions and deletions may have or may optimize a characteristic of the consensus sequence or polypeptide, such as charge (*e*.*g*., positive or negative charge), hydrophilicity, hydrophobicity, *in vivo* stability, bioavailability, toxicity, immunological activity, immunological identity, and conjugation properties. For example, positive charge can be promoted by deleting one or more amino acids (*e.g*., from 1 to 3 amino acids) that are not basic/positively charged (as described below based on common side chain properties) or less positively charged (*e.g*., as determined by pKa). In another example, positive charge can be promoted by inserting one or more amino acids (*e.g*., from 1 to 3 amino acids) that are basic/positively charged or more positively charged (*e*.*g*., as determined by pKa).

*In vivo* stability may be optimized in any useful way. For example, stability in the presence of one or more digestive enzymes can be improved by substituting a naturally occurring L-amino acid for its D-isomer. Exemplary digestive enzymes include pepsin and trypsin. Using the subsite nomenclature for cleavage sites, S1-S1' indicates the cleavage site for a peptide Sn---S4-S3-S2-S1-S1'-S2'-S3'-S4'---Sm. Cleavage by pepsin generally occurs when Phe, Tyr, Trp, or Leu is in position S1 or S1'; or when Pro is in position S3 or S4. Cleavage by trypsin generally occurs when Arg or Lys is in position S1; when Pro is in position S1', Lys is in position S1, and Trp is in position S2; when Pro is in position S1', Arg is in position S1, and Met is in position S2; or when Pro is in position S1' and Glu is in position S2. Other exemplary cleavage sites include those for cleavage by Arg-C proteinase (*e.g*., Arg in position S1), Asp-N endopeptidase (*e.g*., Asp or Glu in position S1'), chymotrypsin (*e.g*., Trp, Tyr, or Phe in position S1 for cleavage with high specificity; Leu, Met, or His in position S1 for cleavage with low specificity), glutamyl endopeptidase (*e.g*., Glu at position S1), LysC lysyl endopeptidase (*e.g.*, Lys at position S1), LysN peptidyl-Lys metalloendopeptidase (*e.g*., Lys at position S1'), proteinase K (*e.g*., an aliphatic or amino acid residue, such as Ala, Glu, Phe, Ile, Leu, Thr, Val, Trp, or Tyr, at position S1), and thermolysin (*e.g*., a bulky or an amino acid residue, such as Ile, Leu, Val, Ala, Met, or Phe, at position S1').

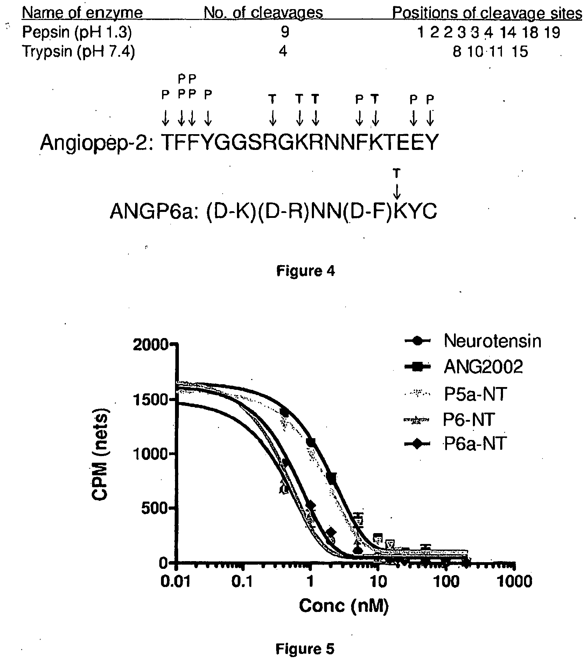

Predictive models are also available for determining cleavage sites, such as PeptideCutter available on the ExPASy proteomics server. Exemplary cleavage sites for targeting moieties are shown in Figure 4, such as C-terminal to positions 1, 2, 3, 4, 14, 18, and 19 in Angiopep-2 (SEQ ID NO:97) for cleavage by pepsin and C-terminal to positions 8, 10, 11, and 15 in Angiopep-2 (SEQ ID NO:97) for cleavage by trypsin. Other exemplary cleavage sites in Angiopep-2 (SEQ ID NO:97) include C-terminal to positions 8 and 11 for cleavage by Arg-C proteinase; positions 16 and 17 for cleavage by Asp-N endopeptidase; positions 2, 3, 4, 14, and 19 for cleavage by chymotrypsin; positions 17 and 18 for cleavage by glutamyl endopeptidase; positions 10 and 15 for cleavage by LysC lysyl endopeptidase; positions 9 and 14 for cleavage by LysN peptidyl-Lys metalloendopeptidase; positions 1, 2, 3, 4, 14, 16, and 19 for cleavage by proteinase K; and positions 1, 2, and 13 for cleavage by thermolysin. Accordingly, the targeting moieties of the invention also include polypeptides shorter than Angiopep-2 (An2) having one or more D-amino acid substitutions for one or more of positions 1, 2, 3, 4, 8, 10, 11, 13, 14, 15, 16, 17, 18, and 19 in Angiopep-2 (SEQ ID NO:97).

Substantial modifications in function or immunological identity are accomplished by selecting substitutions, additions, and deletions that differ significantly in their effect on maintaining (a) the structure of the polypeptide backbone in the area of the substitution, for example, as a sheet or helical conformation; (b) the charge or hydrophobicity of the molecule at the target site; or (c) the bulk of the side chain. Naturally occurring residues are divided into groups based on common side chain properties:
(1) hydrophobic: norleucine, methionine (Met), Alanine (Ala), Valine (Val), Leucine (Leu), Isoleucine (Ile), Histidine (His), Tryptophan (Trp), Tyrosine (Tyr), and Phenylalanine (Phe);
(2) neutral hydrophilic: Cysteine (Cys), Serine (Ser), and Threonine (Thr);
(3) acidic/negatively charged: Aspartic acid (Asp) and Glutamic acid (Glu);
(4) basic: Asparagine (Asn), Glutamine (Gln), Histidine (His), Lysine (Lys), and Arginine (Arg);
(5) residues that influence chain orientation: Glycine (Gly) and Proline (Pro);
(6) aromatic: Tryptophan (Trp), Tyrosine (Tyr), Phenylalanine (Phe), and Histidine (His);
(7) polar: Ser, Thor, Asn, Gln;
(8) basic positively charged: Arg, Lys, His; and
(9) charged: Asp, Glu, Arg, Lys, His.

Other amino acid substitutions are listed in Table 1.

**Table 1: Amino acid substitutions**

| **Original residue** | **Exemplary substitution** | **Conservative substitution** |
|---|---|---|
| Ala (A) | Val, Leu, Ile | Val |
| Arg (R) | Lys, Gln, Asn | Lys |
| Asn (N) | Gln, His, Lys, Arg | Gln |
| Asp (D) | Glu | Glu |
| Cys (C) | Ser | Ser |
| Gln (Q) | Asn | Asn |
| Glu (E) | Asp | Asp |
| Gly (G) | Pro | Pro |
| His (H) | Asn, Gln, Lys, Arg | Arg |
| Ile (I) | Leu, Val, Met, Ala, Phe, Norleucine | Leu |
| Leu (L) | Norleucine, Ile, Val, Met, Ala, Phe | Ile |
| Lys (K) | Arg, Gln, Asn | Arg |
| Met (M) | Leu, Phe, Ile | Leu |
| Phe (F) | Leu, Val, Ile, Ala | Leu |
| Pro (P) | Gly | Gly |
| Ser (S) | Thr | Thr |
| Thr (T) | Ser | Ser |
| Trp (W) | Tyr | Tyr |
| Tyr (Y) | Trp, Phe, Thr, Ser | Phe |
| Val (V) | Ile, Leu, Mel, Phe, Ala, Norleucine | Leu |

The targeting moiety includes the amino acid sequence Lys-Arg-X3-X4-X5-Lys (formula Ia) and Z1-Lys-Arg-X3-X4-X5-Lys-Z2 (formula Ib), as described above and optionally having one or more substitutions for a corresponding D-isomer, and is fewer than 19 amino acids in length. Furthermore, the targeting moiety is not a peptide in Table 2.

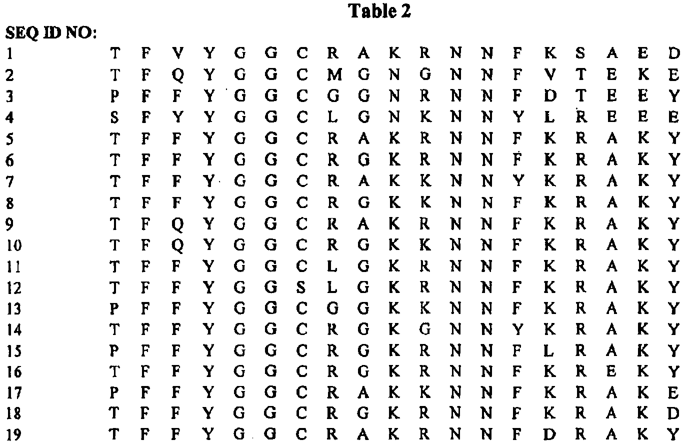

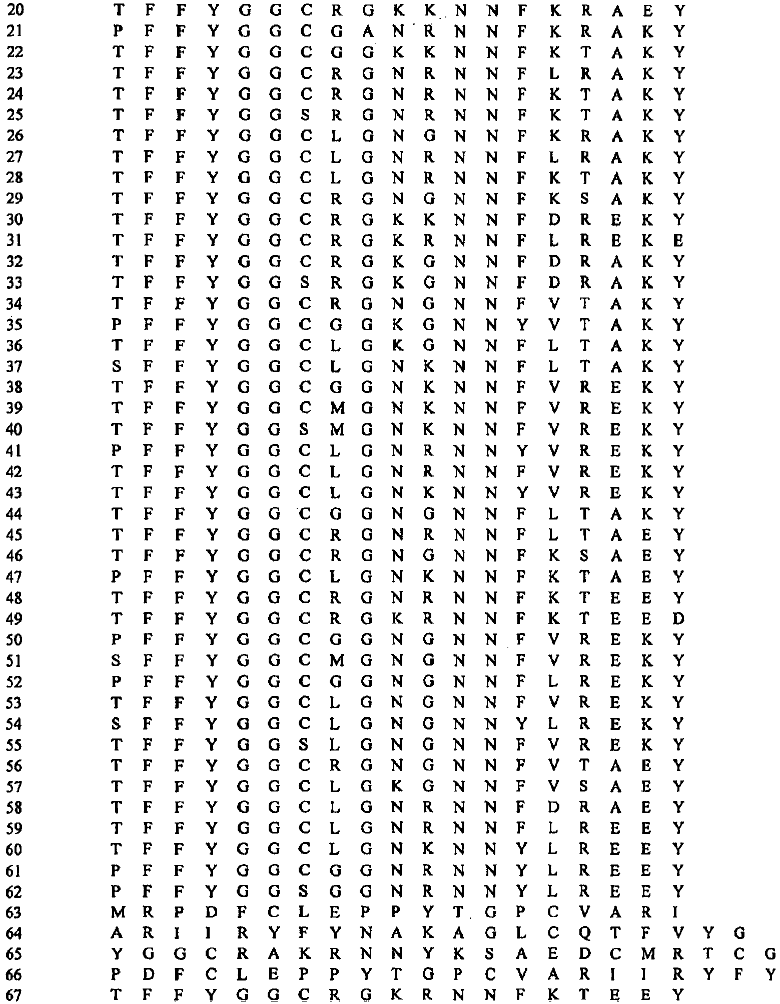

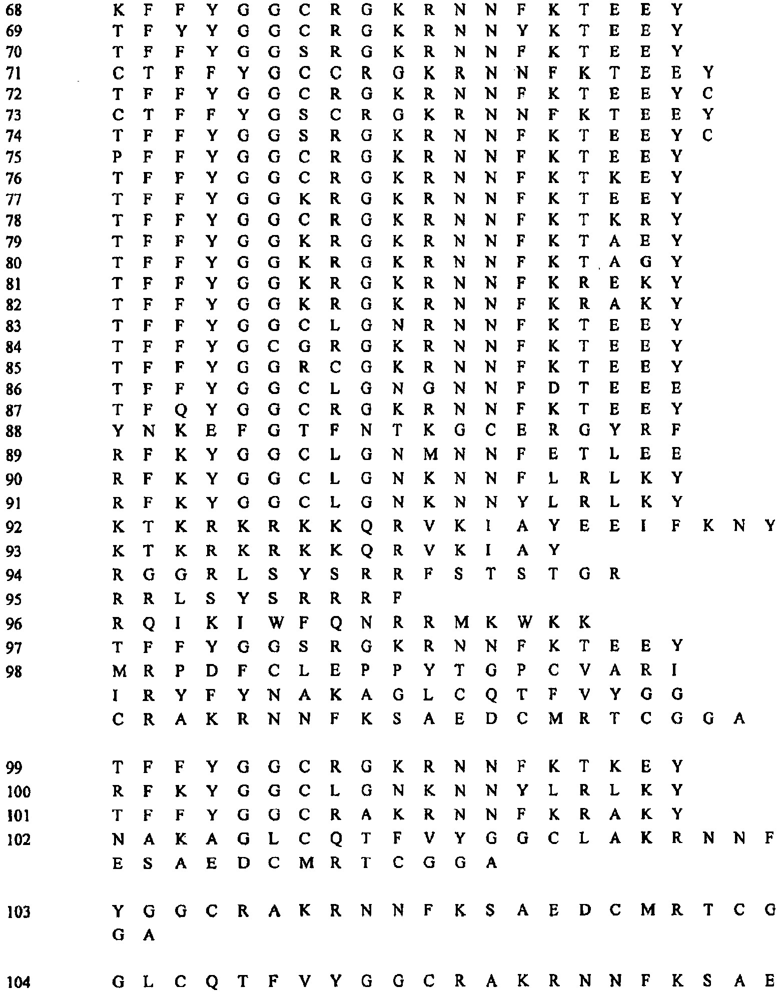

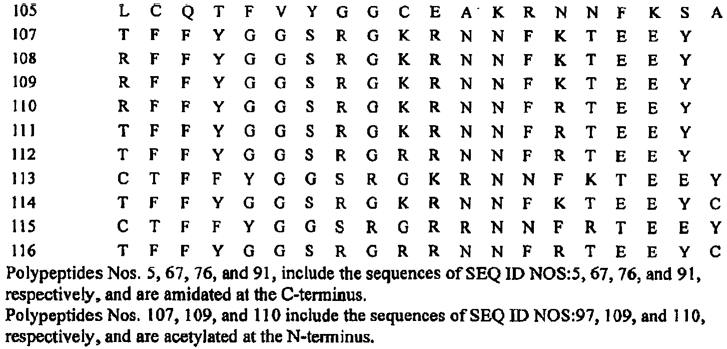

The targeting moieties of the invention also include polypeptides shorter than Angiopep-2 (An2) or 3D-An2 having the amino acid sequence:

| | | | | | | | | | | | | | | | | | | | | |
|---|---|---|---|---|---|---|---|---|---|---|---|---|---|---|---|---|---|---|---|---|
| 97 | T | F | F | Y | G | G | S | R | G | K | R | N | N | F | K | T | E | E | Y | (An2) |
| | T | F | F | Y | G | G | S | D-R | G | D-K | D-R | N | N | F | K | T | E | E | Y | (3D-An2) |

In other embodiments, the targeting moieties are shorter than An2-Cys having the amino acid sequence:

| | | | | | | | | | | | | | | | | | | | | |
|---|---|---|---|---|---|---|---|---|---|---|---|---|---|---|---|---|---|---|---|---|
| 114 | T | F | F | Y | G | G | S | R | G | K | R | N | N | F | K | T | E | E | Y | C |

Exemplary targeting moieties include:

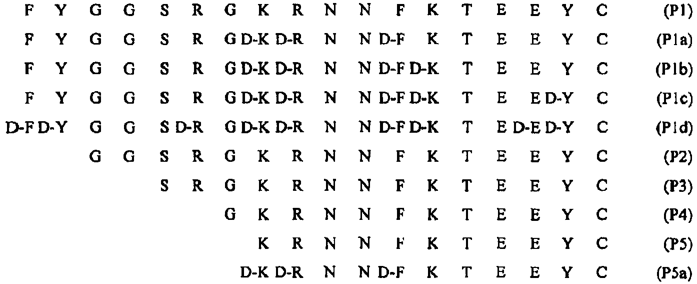

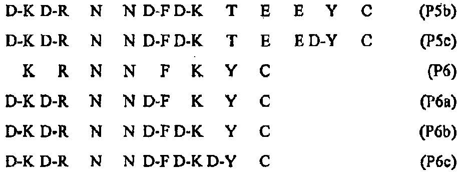

The targeting moieties of the invention include additions and deletions of amino acids of the sequences of P1, P1a, P1b, P1c, P1d, P2, P3, P4, P5, P5a, P5b, P5c, P6, P6a, P6b, and P6c. In some embodiments, deletions or additions of 1, 2, 3, 4, or 5 amino acids may be made from these sequences of the targeting moiety. In particular embodiments, the deletions or additions may be from 1 to 3 amino acids.

The invention also features fragments of these targeting moieties (e.g., a functional fragment). In certain embodiments, the fragments are capable of efficiently being transported to or accumulating in a particular cell type (e.g., liver, eye, lung, kidney, spleen, muscle, or ovary) or are efficiently transported across the BBB. Truncations of the polypeptide may be 1, 2, 3, 4, 5, 6, 7, 8, 9, 10, 11, 12, or more amino acids from either the N-terminus of the polypeptide, the C-terminus of the polypeptide, or a combination thereof.

### Identification of targeting moieties

Additional targeting moieties may be identified by using one of the assays or methods described herein. For example, a candidate polypeptide may be produced by conventional peptide synthesis, conjugated with paclitaxel and administered to a laboratory animal. A biologically-active conjugate may be identified, for example, based on its ability to increase survival of an animal injected with tumor cells and treated with the conjugate as compared to a control which has not been treated with a conjugate (*e*.*g*., treated with the unconjugated agent). For example, a biologically active polypeptide may be identified based on its location in the parenchyma in an *in situ* cerebral perfusion assay.

Assays to determine accumulation in other tissues may be performed as well. Labeled conjugates of a polypeptide can be administered to an animal, and accumulation in different organs can be measured. For example, a candidate polypeptide conjugated to a detectable label (e.g., a near-IR fluorescence spectroscopy label such as Cy5.5) allows live *in vivo* visualization. Such a polypeptide can be administered to an animal, and the presence of the polypeptide in an organ can be detected, thus allowing determination of the rate and amount of accumulation of the polypeptide in the desired organ. In other embodiments, the polypeptide can be labeled with a radioactive isotope (*e.g*., ¹²⁵I). The polypeptide is then administered to an animal. After a period of time, the animal is sacrificed and the organs are extracted. The amount of radioisotope in each organ can then be measured using any means known in the art. By comparing the amount of a labeled candidate polypeptide in a particular organ relative to the amount of a labeled control polypeptide, the ability of the candidate polypeptide to access and accumulate in a particular tissue can be ascertained. Appropriate negative controls include any peptide or polypeptide known not to be efficiently transported into a particular cell type (*e.g*., a peptide related to Angiopep that does not cross the BBB, or any other peptide).

### Conjugates

The targeting moiety can be linked to a therapeutic agent or a transport vector to form a conjugate. In a conjugate, the targeting moiety is joined by a chemical bond either directly (*e.g*., a covalent bond such as a disulfide or a peptide bond) or indirectly (*e.g*., through a linker such as those described herein). In a conjugate having a transport vector, a therapeutic agent may be releasable after transport across the BBB, for example, by enzymatic cleavage or breakage of a chemical bond between the transport vector and the agent. The released agent may then function in its intended capacity in the absence of the vector. Exemplary linkers, therapeutic agents, and transport vectors are described below.

### Therapeutic polypeptides

When the conjugate includes a therapeutic peptidic agent linked to the targeting moiety through a peptide bond or an amino acid or peptide linker, the resultant conjugate is a therapeutic polypeptide (*e.g*., a fusion protein). In embodiments where the agent is a therapeutic peptidic agent, the agent may be linked to the polypeptide by a covalent bond. The covalent bond may be a peptide bond (*e.g*., produced synthetically or recombinantly as a fusion protein). Exemplary therapeutic peptidic agents are described below.

### Joining of the targeting moiety to a transport vector

To form a conjugate including a transport vector, at least two general approaches can be used. In a first approach, a transport vector containing the agent is formed. Then, a targeting moiety described herein is conjugated to the transport vector. In a second approach, the conjugation of targeting moiety to a molecule forming the transport vector is performed first, and then the transport vector is formed subsequently using the conjugated molecule. In either approach, the targeting moiety may be conjugated through a tether molecule.

A conjugate including a transport vector can be formed in a step-wise process. For example, the transport vector molecule is first attached to the linker and transport vectors are formed containing the transport vector molecule. Then, the transport vector is incubated with the targeting moiety to form a covalent bond with the linker. In a particular example, a lipid molecule is attached to the linker and the resultant compound is used to form liposomes. Then, the liposomes are incubated with a solution containing the targeting moiety to attach the targeting moiety to the distal end of the linker.

In another example, the transport vector is covalently linked to a linker with an activated group, the targeting moiety is covalently linked to a second linker, and then the modified transport vector and modified targeting moiety are reacted together to form a covalent bond between the first linker and a second linker. For example, the amino group of a transport vector forms a covalent bond by displacing the N-hydroxysuccinimidyl group of the linker succinimidyl 4-formylbenzoate. This modified transport vector has a terminal carbonyl group on the linker. Then, the amino group of the targeting moiety forms a covalent bond by displacing the N-hydroxysuccinimidyl group of the linker succinimidyl 4-hydrazinonicotinate acetone hydrazone. This modified targeting moiety has a terminal hydrazine group on the linker. Finally, the modified transport vector and the modified targeting moiety are combined to form a covalent bond between the hydrazine group of the modified targeting moiety and the terminal carbonyl group of the transport vector.

In another example, polyoxyethylene-(p-nitrophenyl carbonate)-phosphoethanolamine is used in the formation of lipid micelles containing siRNA molecules. Briefly, in this example, polyoxyethylene-bis (p-nitrophenyl carbonate) ((pNP)₂-PEG) is conjugated to a lipid capable of forming liposomes or micelles such as 1,2-dipalmitoyl-*sn*-glycero-3-phosphoethanolamine (DPPE), resulting in production of pNP-PEG-PE. This molecule can then, in turn, be conjugated to a targeting moiety (e.g., any described herein) to form a peptide-PEG-PE conjugate. This conjugate can then be used in the formation of liposomes that contain PEG moieties which serve as anchors for binding polypeptide molecules on the external face of the liposome. See, *e.g*., Zhang et al., J. Control. Release 112:229-239 (2006).

Production of lipid vectors can also be achieved by conjugating a targeting moiety to a liposome following its formation. In one example of this procedure, a mixture of lipids suitable for encapsulating a molecule and having sufficient *in vivo* stability are provided, where some of the lipids are attached to a tether (such as PEG) containing a linker (*e*.*g*., any linker described herein). The mixture is dried, reconstituted in aqueous solution with the desired polynucleotide, and subject to conditions capable of forming liposomes (*e*.*g*., sonication or extrusion). A targeting moiety described herein is then conjugated to the linker on the tether. In one particular example of this method, the mixture of 93% 1-palmitoyl-2-oleoyl-sn-glycerol-3-phosphocholine (POPC), 3% didodecyldimethylammonium bromide (DDAB), 3% distearoylphosphatidylethanolamine (DSPE)-PEG2000 and 1% DSPE-PEG2000-maleimide is provided. This mixture is then prepared in chloroform, evaporated under nitrogen, and then dissolved in Tris buffer to which the desired polynucleotide is added. The mixture is then passed through a series of polycarbonate filters of reduced pore size 400 nm to 50 nm to generate 80-100 nm liposomes. The liposomes are mixed with a nuclease or protease to remove unencapsulated therapeutic agents. If the therapeutic agent is a DNA molecule, then DNA endonuclease I and exonuclease III can be used. The transport vector described herein can then be conjugated to the DSPE-PEG200 that contains the linker (*e.g*., maleimide or any linker herein. These lipid vectors, which contain a therapeutic agent and are conjugated to a targeting moiety described herein can then be administered to a subject to deliver the therapeutic agent across the BBB or to specific tissues. Further examples of this approach are described in Boado, Pharm. Res. 24:1772-1787 (2007); Pardridge, Pharm. Res. 24:1733-1744 (2007); and Zhang et al., Clin. Canc. Res. 10:3667-3677, 2004.

Alternatively, the conjugate is formed without the use of a linker. Rather, a zero-length coupling agent is used to activate the functional groups within the transport vector or the targeting moiety without introducing additional atoms. Examples of zero-length coupling agents include dicyclohexylcarbodiimide and ethylchloroformate.

### Linkers

The targeting moiety may be bound to a therapeutic agent or a transport vector either directly (*e.g*., through a covalent bond such as a peptide bond) or may be bound through a linker. Linkers include chemical linking agents (*e*.*g*., cleavable linkers) and peptides. Any of the linkers described below may be used in the compounds of the invention.

### Chemical linking agents

In some embodiments, the linker is a chemical linking agent. The targeting moiety may be conjugated through sulfhydryl groups, amino groups (amines), or any appropriate reactive group. Homomultifunctional and heteromultifunctional cross-linkers (conjugation agents, including bifunctional and trifunctional agents) are available from many commercial sources. Sites available for cross-linking may be found on the targeting moieties and therapeutic agents or transport vectors described herein. The cross-linker may comprise a flexible arm, *e.g*., 2, 3, 4, 5, 6, 7, 8, 4, 10, 11, 12, 13, 14, or 15 carbon atoms. The flexible arm can be polyethylene glycol spacer, such as (PEG)ₙ, where n is an integer between 1 and 20, or an amino acid, such as -NH-(CH₂)ₙ-C(O)O-, where n is an integer between 2 and 10 (*e*.*g*., when n is 5).

Exemplary cross-linkers include BS³ ([Bis(sulfosuceinimidyl)suberate]; BS³ is a homobifunctional N-hydroxysuccinimide ester that targets accessible primary amines), NHS/EDC (N-hydroxysuccinimide and 1-ethyl-3-(3-dimethylaminopropyl)carbodiimide; NHS/EDC allows for the conjugation of primary amine groups with carboxyl groups), sulfo-EMCS ([N-ε-maleimidocaproic acid]hydrazide; sulfo-EMCS are heterobifunctional reactive groups (maleimide and NHS-ester) that are reactive toward sulfhydryl and amino groups), hydrazide (most proteins contain exposed carbohydrates and hydrazide is a useful reagent for linking carboxyl groups to primary amines), SATA (N-succinimidyl-S-acetylthioacetate; SATA is reactive towards amines and adds protected sulfhydryls groups), and BMOE (bis-maleimidoethane).

To form covalent bonds, one can use as a chemically reactive group a wide variety of active carboxyl groups (e.g., esters) where the hydroxyl moiety is physiologically acceptable at the levels required to modify the peptide. Particular agents include N-hydroxysuccinimide (NHS), N-hydroxy-sulfosuccinimide (sulfo-NHS), maleimide-benzoyl-succinimide (MBS), gamma-maleimido-butyryloxy succinimide ester (GMBS), maleimido propionic acid (MPA), maleimido hexanoic acid (MHA), and maleimido undecanoic acid (MUA).

Primary amines are the principal targets for NHS esters. Accessible α-amine groups present on the N-termini of proteins and the ε-amine of lysine react with NHS esters. Thus, compounds of the invention can include a linker having a NHS ester conjugated to an N-terminal amino of a peptide or to an ε-amine of lysine. An amide bond is formed when the NHS ester conjugation reaction reacts with primary amines releasing N-hydroxysuccinimide. These succinimide containing reactive groups are herein referred to as succinimidyl groups. In certain embodiments of the invention, the functional group on the protein will be a thiol group and the chemically reactive group will be a maleimido-containing group such as gamma-maleimide-butrylamide (GMBA or MPA). Such maleimide containing groups are referred to herein as maleido groups.

The maleimido group is most selective for sulfhydryl groups on peptides when the pH of the reaction mixture is 6.5-7.4. At pH 7.0, the rate of reaction of maleimido groups with sulfhydryls (*e.g*., thiol groups on proteins such as serum albumin) is 1000-fold faster than with amines. Thus, a stable thioether linkage between the maleimido group and the sulfhydryl can be formed. Accordingly, a compound of the invention can include a linker having a maleimido group conjugated to a sulfhydryl group of a targeting moiety or of an agent.

Amine-to-amine linkers include NHS esters and imidoesters. Exemplary NHS esters are DSG (disuccinimidyl glutarate), DSS (disuccinimidyl suberate), BS³ (bis[sulfosuccinimidyl] suberate), TSAT (*tris*-succinimidyl aminotriacetate), variants of bis-succinimide ester-activated compounds that include a polyethylene glycol spacer, such as BS(PEG)ₙ, where n is 1-20 (e.g., BS(PEG)₅ and BS(PEG)₉), DSP (Dithiobis[succinimidyl propionate]), DTSSP (3,3'-dithiobis[sulfosuccinimidylpropionate]), DST (disuccinimidyl tartarate), BSOCOES (bis[2-(succinimidooxycarbonyloxy)ethyl]sulfone), EGS (ethylene glycol bis[succinimidylsuccinate]), and sulfo-EGS (ethylene glycol bis[sulfosuccinimidylsuccinate]). Imidoesters include DMA (dimethyl adipimidate•2 HCl), DMP (dimethyl pimelimidate•2 HCl), DMS (dimethyl suberimidate•2 HCl), and DTBP (dimethyl 3,3'-dithiobispropionimidate•2 HCl). Other amine-to-amine linkers include DFDNB (1,5-difluoro-2,4-dinitrobenzene) and THPP (β-[tris(hydroxymethyl) phosphino] propionic acid (betaine)).

The linker may be a sulfhydryl-to-sulfhydryl linker. Such linkers include maleimides and pyridyldithiols. Exemplary maleimides include BMOE (bis-maleimidoethane), BMB (1,4-bismaleimidobutane), BMH (bismaleimidohexane), TMEA (*tris*[2-maleimidoethyl]amine), BM(PEG)2 1,8-bis-maleimidodiethyleneglycol) or BM(PEG)ₙ, where n is 1 to 20 (*e.g*., 2 or 3), BMDB (1,4 bismaleimidyl-2,3-dihydroxybutane), and DTME (dithio-bismaleimidoethane). Exemplary pyridyldithiols include DPDPB (1,4-di-[3'-(2'-pyridyldithio)-propionamido]butane). Other sulfhydryl linkers include HBVS (1,6-hexane-bis-vinylsulfone).

The linker may be an amine-to-sulfhydryl linker, which includes NHS ester/maleimide compounds. Such amine-to-sulfhydryl linkers can include ester linkers (e.g., any linker described herein containing an ester group). Examples of these compounds are AMAS (N-(α-maleimidoacetoxy)succinimide ester), BMPS (*N*-[β-maleimidopropyloxy]succinimide ester), GMBS (N-[-maleimidobutyryloxy]succinimide ester), sulfo-GMBS (N-[γ-maleimidobutyryloxy]sulfosuccinimide ester), MBS (*m*-maleimidobenzoyl-*N*-hydroxysuccinimide ester), sulfo-MBS (*m*-maleimidobenzoyl-*N-*hydroxysulfosuccinimide ester), SMCC (succinimidyl 4-[*N*-maleimidomethyl]cyclohexane-1-carboxylate), sulfo-SMCC (Sulfosuccinimidyl 4-[*N*-maleimidomethyl]cyclohexane-1-carboxylate), EMCS ([N-ε-maleimidocaproyloxy]succinimide ester), Sulfo-EMCS ([N-e-maleimidocaproyloxy]sulfosuccinimide ester), SMPB (succinimidyl 4-[*p-*maleimidophenyl]butyrate), sulfo-SMPB (sulfosuccinimidyl 4-[*p*-maleimidophenyl]butyrate), SMPH (succinimidyl-6-[β-maleimidopropionamido]hexanoate), LC-SMCC (succinimidyl-4-[N-maleimidomethyl]cyclohexane-1-carboxy-[6-amidocaproate]), sulfo-KMUS (*N*-[κ-maleimidoundecanoyloxy]sulfosuccinimide ester), SM(PEG)ₙ (succinimidyl-([*N-*maleimidopropionamido-polyethyleneglycol) ester), where n is 1 to 30 (*e.g*., 2, 4, 6, 8, 12, or 24), SPDP (*N*-succinimidyl 3-(2-pyridyldithio)-propionate), LC-SPDP (succinimidyl 6-(3-[2-pyridyldithio]-propionamido)hexanoate), sulfo-LC-SPDP (sulfosuccinimidyl 6-(3'-[2-pyridyldithio]-propionamido)hexanoate), SMPT (4-succinimidyloxycarbonyl-α-methyl-α-[2-pyridyidithio]toluene), Sulfo-LC-SMPT (4-sulfosuccinimidyl-6-[α-methyl-α-(2-pyridyldithio)toluamido]hexanoate), SIA (*N*-succinimidyl iodoacetate), SBAP (succinimidyl 3-[bromoacetamido]propionate), SIAB (*N*-succinimidyl[4-iodoacetyl]aminobenzoate), and sulfo-SIAB (*N*-sulfosuccinimidyl[4-iodoacetyl]aminobenzoate).

In particular embodiments, the linker has the formula: 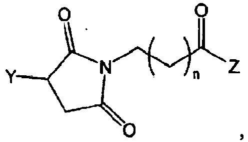 where n is an integer between 2 and 15 (*e*.*g*., n is 3, 6, or 11); and either Y is a thiol on A and Z is a primary amine on B or Y is a thiol on B and Z is a primary amine on A.

In other embodiments, the linker is an amino-to-nonselective linker. Examples of such linkers include NHS ester/aryl azide and NHS ester/diazirine linkers. NHS ester/aryl azide linkers include NHS-ASA (*N*-hydroxysuccinimidyl-4-azidosalicylic acid), ANB-NOS (*N*-5-azido-2-nitrobenzoyloxysuccinimide), sulfo-HSAB (*N*-hydroxysulfosuccinimidyl-4-azidobenzoate), sulfo-NHS-LC-ASA (sulfosuccinimidyl[4-azidosalicylamido]hexanoate), SANPAH (*N*-succinimidyl-6-(4'-azido-2'-nitrophenylamino)hexanoate), sulfo-SANPAH (*N*-sulfosuccinimidyl-6-(4'-azido-2'-nitrophenylamino)hexanoate), sulfo-SFAD (sulfosuccinimidyl-(perfluoroazidobenzamido)-ethyl-1,3'-dithioproprionate), sulfo-SAND (sulfosuccinimidyl-2-(*m*-azido-*o*-nitrobenzamido)-ethyl-1,3'-proprionate), and sulfo-SAED (sulfosuccinimidyl 2-[7-amino-4-methylcoumarin-3-acetamido]ethyl-1,3'dithiopropionate). NHS ester/diazirine linkers include SDA (succinimidyl 4,4'-azipentanoate), LC-SDA (succinimidyl 6-(4,4'-azipentanamido)hexanoate), SDAD (succinimidyl 2-([4,4'-azipentanamido]ethyl)-1,3'-dithioproprionate), sulfo-SDA (sulfosuccinimidyl 4,4'-azipentanoate), sulfo-LC-SDA (sulfosuccinimidyl 6-(4,4'-azipentanamido)hexanoate), and sulfo-SDAD (sulfosuccinimidyl 2-([4,4'-azipentanamido]ethyl)-1,3'-dithioproprionate).

Exemplary amine-to-carboxyl linkers include carbodiimide compounds (*e*.*g*., DCC (N,N-dicyclohexylcarbodimide) and EDC (1-ethyl-3-[3-dimethylaminopropyl]carbodiimide)). Exemplary sulthydryl-to-nonselective linkers include pyridyldithiol/aryl azide compounds (*e*.*g*., APDP ((*N*-[4-(*p*-azidosalicylamido)butyl]-3'-(2'-pyridyldithio)propion-amide)). Exemplary sulfhydryl-to-carbohydrate linkers include maleimide/hydrazide compounds *(e.g.,* BMPH (*N*-[β-maleimidopropionic acid]hydrazide), EMCH ([*N-*ε-maleimidocaproic acid]hydrazide), MPBH 4-(4-*N*-maleimidophenyl)butyric acid hydrazide), and KMUH (*N*-[κ-maleimidoundecanoic acid]hydrazide)) and pyridyldithiol/hydrazide compounds (e.g., PDPH (3-(2-pyridyldithio)propionyl hydrazide)). Exemplary carbohydrate-to-nonselective linkers include hydrazide/aryl azide compounds (*e.g*., ABH (*p*-azidobenzoyl hydrazide)). Exemplary hydroxyl-to-sulfhydryl linkers include isocyanate/maleimide compounds (*e.g*., (*N*-[*p*-maleimidophenyl]isocyanate)). Exemplary amine-to-DNA linkers include NHS ester/psoralen compounds (*e.g*., SPB (succinimidyl-[4-(psoralen-8-yloxy)]-butyrate)).

Linkers are also described in U.S. Patent No. 4,680,338 having the formula Y=C=N-Q-A-C(O)-Z, where Q is a homoaromatic or heteroaromatic ring system; A is a single bond or an unsubstituted or substituted divalent C₁₋₃₀ bridging group, Y is O or S; and Z is Cl, Br, I, N₃, N-succinimidyloxy, imidazolyl, 1-benzotriazolyloxy, OAr where Ar is an electron-deficient activating aryl group, or OC(O)R where R is -A-Q-N=C=Y or C₄ -20 tertiary-alkyl.

Linkers are also described in U.S. Patent No. 5,306,809, which describes linkers having the formula 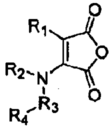 where R₁ is H, C₁₋₆ alkyl, C₂₋₆ alkenyl, C₆₋₁₂ aryl or aralkyl or these coupled with a divalent organic -Q-, -S-, or 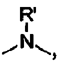 where R' is C₁₋₆ alkyl, linking moiety; R₂ is H, C₁₋₁₂ alkyl, C₆₋₁₂ aryl, or C₆₋₁₂ aralkyl, R₃ is 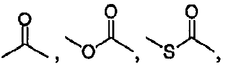 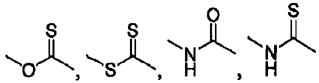 or another chemical structure which is able to delocalize the lone pair electrons of the adjacent nitrogen and R₄ is a pendant reactive group capable of linking R₃ to a targeting moiety or to an agent.

The linker can be polyvalent or monovalent. A monovalent linker has only one activated group available for forming a covalent bond. However, the monovalent linker can include one or more functional groups that can be chemically modified by using a coupling agent, as described herein, to form a second activated group. For example, a terminal hydroxyl group of the linker can be activated by any number of coupling agents. Examples of coupling agents include N-hydroxysuccinimide, ethylchloroformate, dicyclohexylcarbodiimide, and trifluoromethanesulfonyl chloride. See, *e.g.* U.S. Patent Nos. 5,395,619 and 6,316,024.

A polyvalent linker (*e*.*g*., a multifunctional linker) has two or more activated groups. The activated groups in the linker can be the same, as in a homopolyvalent linker, or different, as in a heteropolyvalent linker. Heteropolyvalent linkers allow for conjugating a polypeptide and a transport vector with different functional groups. Examples of heteropolyvalent linkers include polyoxyethylene-bis(p-nitrophenyl carbonate), mal-PEG-DSPE, diisocyanate, succinimidyl 4-hydrazinonicotinate acetone hydrazone.

Examples of homopolyvalent linkers with two activated groups include disuccinimidyl glutarate, disuccinimidyl suberate, bis(sulfosuccinimidyl) suberate, bis(NHS)PEG₅, bis(NHS)PEG₉, dithiobis(succinimidyl propionate), 3,3'-dithiobis(sulfosuccinimidylpropionate), disuccinimidyl tartrate, bis[2-(succinimido oxycarbonyloxy)ethyl]sulfone, ethylene glycol bis[succinimidylsuccinate]), ethylene glycol bis[sulfosuccinimidylsuccinate]), dimethyl adipimidate, dimethyl pimelimidate, dimethyl suberimidate, dimethyl 3,3'-dithiobispropionimidate, 1,5-difluoro-2,4-dinitrobenzene, bis-maleimidoethane, 1,4-bismaleimidobutane, bismaleimidohexane, 1,8-bis-maleimidodiethyleneglycol, 1,11-bis-maleimido-triethyleneglycol, 1,4-di-[3'-(2'-pyridyldithio)-propionamido]butane, 1,6-hexane-bis-vinylsulfone, and bis-[b-(4-azidosalicylamido)ethyl]disulfide.

Examples of homopolyvalent linkers with three activated groups include tris-succinimidyl aminotriacetate, β-[tris(hydroxymethyl) phosphino] propionic acid, and tris[2-maleimidoethyl]amine.

Examples of heteropolyvalent linkers include those with an maleimide activated group and a succinimide activated group, such as N-[α-maleimidoacetoxy]succinimide ester, N-[β-mateimidopropyloxy]-succinimide ester, N-[γ-maleimidobutyryloxy]succinimide ester, m-maleimidobenzoyl-N-hydroxysuccinimide ester, succinimidyl 4-[*N-*maleimidomethyl]cyclohexane-1-carboxylate, N-[ε-maleimidocaproyloxy]succinimide ester, and succinimidyl 4-[*p*-maleimidophenyl]butyrate, including N-sulfosuccinimidyl derivatives; those with a PEG spacer molecule, such as succinimidyl-([*N*-maleimidopropionamido)-(ethyleneglycol)ₓ)ester, wherein x is from 2 to 24; those with a pyridyldithio activated group and a succinimide activated group, such as N-succinimidyl-3-(2-pyridyldithio)propionate, succinimidyl 6-(3-[2-pyridyldithio]-propionamido)hexanoate, 4-succinimidyloxycarbonyl-methyl-a-[2-pyridyldithio]toluene, and 4-sulfosuccinimidyl-6-methyl-a-(2-pyridyldithio)toluamido]hexanoate); those with a haloacetyl activated group and a succinimide activated group, such as N-succinimidyl iodoacetate and N-succinimidyl[4-iodoacetyl]aminobenzoate; those with an aryl azide activated group and a succinimide activated group, such as *N*-hydroxysuccinimidyl-4-azidosalicylic acid, sulfosuccinimidyl[4-azidosalicylamido]-hexanoate, and *N*-succinimidyl-6-(4'-azido-2'-nitrophenylamino) hexanoate; those with an diazirine activated group and a succinimide activated group, such as succinimidyl 4,4'-azipentanoate and succinimidyl 6-(4,4'-azipentanamido)hexanoate; N-[4-(p-azidosalicylamido)butyl]-3'-(2'-pyridyldithio)propionamide; N-[β-maleimidopropionic acid] hydrazide; N-(ε-maleimidocaproic acid) hydrazide; 4-(4-N-maleimidophenyl)butyric acid hydrazide hydrochloride; (N-[κ-maleimidoundecanoic acid]-hydrazide); 3-(2-pyridyldithio)propionyl hydrazide; p-azidobenzoyl hydrazide; and N-[p-maleimidophenyl]isocyanate.

In other embodiments, the linker is a trifunctional, tetrafunctional, or greater linking agent. Exemplary trifunctional linkers include TMEA, THPP, TSAT, LC-TSAT (*tris-*succinimidyl (6-aminocaproyl)aminotriacetate), *tris*-succinimidyl-1,3,5-benzenetricarboxylate, MDSI (maleimido-3,5-disuccinimidyl isophthalate), SDMB (succinimidyl-3,5-dimaleimidophenyl benzoate, Mal-4 (*tetrakis*-(3-maleimidopropyl)pentaerythritol, NHS-4 (*tetrakis*-(N-suceinimidylcarboxypropyl)pentaerythritol)).

TMEA has the structure: 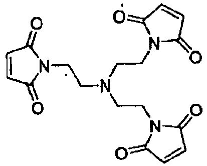 TMEA, through its maleimide groups, can react with sulfhydryl groups (*e*.*g*., through cysteine amino acid side chains).

THPP has the structure: 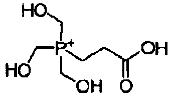 The hydroxyl groups and carboxy group of THPP can react with primary or secondary amines.

### Amino acid and peptide linkers

In other embodiments, the linker includes at least one amino acid (*e*.*g*., a peptide of at least 2, 3, 4, 5, 6, 7, 10, 15, 20, 25, 40, or 50 amino acids). In certain embodiments, the linker is a single amino acid (*e*.*g*., any naturally occurring amino acid such as Cys). In other embodiments, a glycine-rich peptide such as a peptide having the sequence [Gly-Gly-Gly-Gly-Ser]ₙ where n is 1, 2, 3, 4, 5 or 6 is used, as described in U.S. Patent No. 7,271,149. In other embodiments, a serine-rich peptide linker is used, as described in U.S. Patent No. 5,525,491. Serine rich peptide linkers include those of the formula [X-X-X-X-Gly]y, where up to two of the X are Thr, and the remaining X are Ser, and y is 1 to 5 (*e.g*., Ser-Ser-Ser-Ser-Gly, where y is greater than 1). In some cases, the linker is a single amino acid (*e.g*., any amino acid, such as Gly or Cys).

Amino acid linkers may be selected for flexibility (*e.g*., flexible or rigid) or may be selected on the basis of charge (e.g., positive, negative, or neutral). Flexible linkers typically include those with Gly resides (*e*.*g*., [Gly-Gly-Gly-Gly-Ser]ₙ where n is 1, 2, 3, 4, 5 or 6). Other linkers include rigid linkers (*e*.*g*., PAPAP and (PT)ₙP, where n is 2,3,4,5,6, or 7) and α-helical linkers (*e*.*g*., A(EAAAK)ₙA, where n is 1, 2, 3, 4, or 5).

Examples of suitable linkers are succinic acid, Lys, Glu, and Asp, or a dipeptide such as Gly-Lys. When the linker is succinic acid, one carboxyl group thereof may form an amide bond with an amino group of the amino acid residue, and the other carboxyl group thereof may, for example, form an amide bond with an amino group of the peptide or substituent. When the linker is Lys, Glu, or Asp, the carboxyl group thereof may form an amide bond with an amino group of the amino acid residue, and the amino group thereof may, for example, form an amide bond with a carboxyl group of the substituent. When Lys is used as the linker, a further linker may be inserted between the ε-amino group of Lys and the substituent. In one particular embodiment, the further linker is succinic acid, which can form an amide bond with the ε- amino group of Lys and with an amino group present in the substituent. In one embodiment, the further linker is Glu or Asp (*e*.*g*., which forms an amide bond with the ε-amino group of Lys and another amide bond with a carboxyl group present in the substituent), that is, the substituent is an N^{ε}-acylated lysine residue.

In other embodiments, the peptide linker is a branched polypeptide. Exemplary branched peptide linkers are described in U.S. Patent No. 6,759,509. Such linkers include those of the formula: 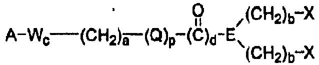 where A is a thiol acceptor; W is a bridging moiety; c is an integer of 0 to 1; a is an integer of 2 to 12; Q is O, NH, or N-lower alkyl; p is an integer of 0 or 1; d is an integer of 0 or 1; E is a polyvalent atom; each b is an integer of 1 to 10; each X is of the formula:

-CO-Y-Zₘ-Gₙ

where Y is two amino acid residues in the L form; Z is one or two amino acid residues; m is an integer of 0 or 1; G is a self-immolative spacer; and n is a integer of 0 or 1; provided that when n is 0 then -Y-Zₘ is Ala-Leu-Ala-Leu or Gly-Phe-Leu-Gly; or each X is of the formula: 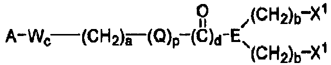 where each X¹ is of the formula -CO-Y-Zₘ-Gₙ; and where Y, Z, Q, E, G, m, d, p, a, b, and n are as defined above; or each X¹ is of the formula: 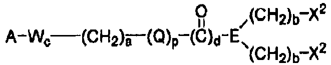 where each X² is of the formula -CO-Y-Zₘ-Gₙ; and where Y, Z, G, Q, E, m, d, p, a, b, and n are as defined above; or each X² is of the formula: 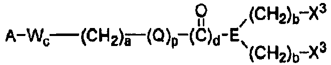 where each X³ is of the formula -CO-Y-Zₘ-Gₙ; and wherein Y, Z, G, Q, E, m, d, p, a, b, and n are as defined above; or each X³ is of the formula: 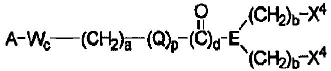 where each X⁴ is of the formula -CO-Y-Zₘ-Gₙ; and where Y, Z, G, Q, E, m, d, p, a, b, and n are as defined above.

The branched linker may employ an intermediate self-immolative spacer moiety (G), which covalently links together the agent or peptide vector and the branched peptide linker. A self-immolative spacer can be a bifunctional chemical moiety capable of covalently linking together two chemical moieties and releasing one of said spaced chemical moieties from the tripartate molecule by means of enzymatic cleavage (*e*.*g*., any appropriate linker described herein. In certain embodiments, G is a self-immolative spacer moiety which spaces and covalently links together the agent or peptide vector and the peptide linker, where the spacer is linked to the peptide vector or agent via the T moiety (as used in the following formulas "T" represents a nucleophilic atom which is already contained in the agent or peptide vector), and which may be represented by 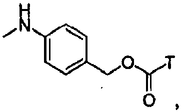 where T is O, N or S; -HN-R¹-COT, where T is O, N or S, and R¹ is C₁₋₅ alkyl; 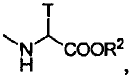 where T is O, N, or S, and R² is H or C₁₋₅ alkyl; 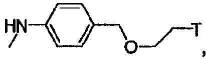 where T is O, N or S; or 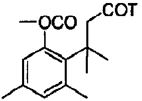 where T is O, N, or S. Preferred Gs include PABC (p-aminobenzyl-carbamoyl), GABA (γ-aminobutyric acid), α,α-dimethyl GABA, and β,β-dimethyl GABA.

In the branched linker, the thiol acceptor "A" is linked to a peptide vector or agent by a sulfur atom derived from the peptide vector or agent. The thiol acceptor can be, for example, an α-substituted acetyl group. Such a group has the formula: 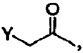 where Y is a leaving group such as Cl, Br, I, mesylate, tosylate, and the like. If the thiol acceptor is an alpha-substituted acetyl group, the thiol adduct after linkage to the ligand forms the bond - S-CH₂-. Preferably, the thiol acceptor is a Michael Addition acceptor. A representative Michael Addition acceptor of this invention has the formula 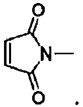 After linkage the thiol group of the ligand, the Michael Addition acceptor becomes a Michael Addition adduct, *e*.*g*., 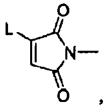 where L is an agent or peptide vector.

The bridging group "W" is a bifunctional chemical moiety capable of covalently linking together two spaced chemical moieties into a stable tripartate molecule. Examples of bridging groups are described in S. S. Wong, Chemistry of Protein Conjugation and Crosslinking. CRC Press, Florida, (1991); and G. E. Means and R. E. Feeney, Bioconjugate Chemistry, vol. 1, pp.2-12, (1990), the disclosures of which are incorporated herein by reference. W can covalently link the thiol acceptor to a keto moiety. An exemplary a bridging group has the formula -(CH₂)_{f}-(Z)_{g}-(CH₂)ₕ-, where f is 0 to 10; h is 0 to 10; g is 0 or 1, provided that when g is 0, then f+h is 1 to 10; Z is S, O, NH, SO₂, phenyl, naphthyl, a polyethylene glycol, a cycloaliphatic hydrocarbon ring containing 3 to 10 carbon atoms, or a heteroaromatic hydrocarbon ring containing 3 to 6 carbon atoms and 1 or 2 heteroatoms selected from O, N, or S. Preferred cycloaliphatic moieties include cyclopropyl, cyclobutyl, cyclopentyl, cyclohexyl, and the like. Preferred heteroaromatic moieties include pyridyl, polyethylene glycol (1-20 repeating units), furanyl, pyranyl, pyrimidinyl, pyrazinyl, pyridazinyl, oxazinyl, pyrrolyl, thiazolyl, morpholinyl, and the like. In the bridging group, it is preferred that when g is 0, f+h is an integer of 2 to 6 (*e*.*g*., 2 to 4 such as 2). When g is 1, it is preferred that f is 0, 1 or 2; and that h is 0, 1 or 2. Preferred bridging groups coupled to thiol acceptors are shown in the Pierce Catalog, pp. E-12, E-13, E-14, E-15, E-16, and E-17 7 (1992).

### Modifications to linkers

Any of the linkers described herein (*e*.*g*., chemical linking agents or amino acid linkers) may be modified. For example, the linkers can include a spacer molecule. The spacer molecule within linker can be of any suitable molecule. Examples of spacer molecules include aliphatic carbon groups (*e*.*g*., C₂-C₂₀ alkyl groups), cleavable heteroatomic carbon groups (*e*.*g*., C₂-C₂₀ alkyl groups with dithio groups), and hydrophilic polymer groups. Examples of hydrophilic polymer groups include poly(ethylene glycol) (PEG), polyvinylpyrrolidone, polyvinylmethylether, polymethyloxazoline, polyethyloxazoline, polyhydroxypropyloxazoline, polyhydroxypropylmethacrylamide, polymethacrylamide, polydimethylacrylamide, polyhydroxypropylmethacrylate, polyhydroxyethylacrylate, hydroxymethylcellulose, hydroxyethylcellulose, polyethyleneglycol, polyaspartamide, and a hydrophilic peptide sequence.

In one example, the hydrophilic polymer is PEG, such as a PEG chain having a molecular weight between 500-10,000 Da (*e*.*g*., between 1,000-5,000 Da such as 2,000 Da). Methoxy or ethoxy-capped analogues of PEG can also be used. These are commercially available in sizes ranging between 120-20,000 Da. Preparation of lipid-tether conjugates for use in liposomes is described, for example, in U.S. Patent No. 5,395,619.

Other spacer molecules include polynucleotides (*e*.*g*., DNA or RNA), polysaccharides such as dextran or xanthan, cellulose derivatives (*e*.*g*., carboxymethyl cellulose), polystyrene, polyvinyl alcohol, poly methylacrylic acid, and poly(NIPAM). Synthetic reaction schemes for activating PEG with coupling agents are set forth in U.S. Pat. Nos. 5,631,018, 5,527,528, and 5,395,619. Synthetic reaction schemes for linkers with PEG spacer molecules are set forth in U.S. Pat. Nos. 6,828,401, and 7,217,845.

PEG, for example, can be conjugated to a polypeptide of the invention by any means known in the art. In certain embodiments, the PEG molecule is derivatized with a linker, which is then reacted with the protein to form a conjugate. Suitable linkers include aldehydes, tresyl or tosyl linkers, dichlorotriazine or chlorotriazine, epoxide, carboxylates such as succinimidyl succinate, carbonates such as a p-nitrophenyl carbonate, benzotriazolyl carbonate, 2,3,5-trichlorophenyl carbonate, and PEG-succinimidyl carbonate, or reactive thiols such as pyridyldisufide, maleimide, vinylsulfone, and iodo acetamide. Conjugation can take place at amino groups (*e*.*g*., the N-terminal amino group or amino groups within the lysine side chain), or at thiol hydroxyl, or amide groups, depending on the linker used. See, *e*.*g*., Veronese et al., Drug Discov. Today 10:1451-1458,2005.

### Therapeutic agents

The conjugate can include any useful therapeutic agent. Any of the therapeutic agents described below may be used in the compounds of the invention. Agents of particular interest include anticancer agents (*e*.*g*., paclitaxel or a paclitaxel derivative, such as docetaxel; etoposide; doxorubicin; and analogs thereof), therapeutic nucleic acid agents, and therapeutic peptidic agents (*e*.*g*., neurotensin and neurotensin receptor agonists, GDNF and analogs thereof, BDNF and analogs thereof, GLP-1 agonists, leptin, and OB receptor agonists).

### Anticancer agents

In accordance with the present invention, the agent may be an anticancer agent. An anticancer agent encompassed by the present invention may include, for example, a drug having a group allowing its conjugation to the targeting moiety of the invention. Particular anticancer agents include those selected from the group consisting of paclitaxel (Taxol®), a paclitaxel derivative (*e*.*g*., docetaxel (Taxotere®)), vinblastine, vincristine, etoposide, doxorubicin, cyclophosphamide, melphalan, and chlorambucil; derivatives (or analogs) thereof; pharmaceutically acceptable salts thereof; or a combination thereof. In particular embodiments, the anticancer agent is paclitaxel, docetaxel, etoposide, or doxorubicin; a pharmaceutically acceptable salt thereof; or a derivative thereof.

As used herein, a "paclitaxel derivative" refers to a mitotic inhibitor compound having a biological activity that is at least 70% (*e*.*g*., 75%, 80%, 85%, 90%, 95%, 99%, or more) as effective as paclitaxel. Exemplary biological activity includes one or more IC₅₀ values as determined by competitive ELISA assays, such as with rabbit antiserum; tubulin disassembly assays; and/or cytotoxicity assays; and one or more pharmacokinetic parameters, such as Cₘₐₓ, AUC, and/or Cₘᵢₙ.

Exemplary paclitaxel derivatives include docetaxel and analogs thereof, as described herein. In particular embodiments, the anticancer agent is paclitaxel or a paclitaxel analog or a paclitaxel derivative. Paclitaxel has the formula:

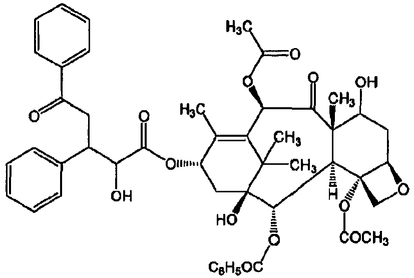

Structural analogs or derivatives of paclitaxel are described in U.S. Patent No. 6,911,549, and can be described by the formula: 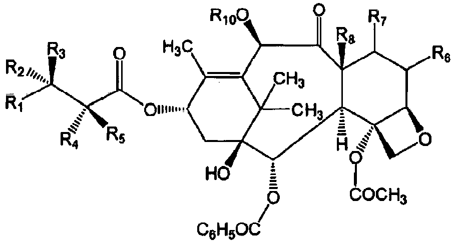 where R₁ is selected from the group consisting of -CH₃; -C₆H₅, or phenyl substituted with 1, 2 or 3 C₁-C₄ alkyl, C₁-C₃ alkoxy, halo, C₁-C₃ alkylthio, trifluoromethyl, C₂-C₆ dialkylamino, hydroxyl, or nitro; and 2-furyl, 2-thienyl, 1-naphthyl, 2-naphthyl or 3,4-methylenedioxyphenyl; R₂ is selected from the group consisting of -H, -NHC(O)H,-NHC(O)C₁-C₁₀ alkyl (preferably -NHC(O)C₄-C₆, alkyl), -NHC(O)phenyl, NHC(O)phenyl substituted with one, 2, or 3 C₁-C₄ alkyl, C₁-C₃ alkoxy, halo, C₁-C₃ alkylthio, trifluoromethyl, C₂-C₆ dialkylamino, hydroxy or nitro, -NHC(O)C(CH₃)=CHCH₃, -NHC(O)OC(CH₃)₃, - NHC(O)OCH₂ phenyl, -NH₂, -NHSO₂-4-methylphenyl, -NHC(O)(CH₂)₃COOH, -NHC(O)-4-(SO₃H)phenyl, -OH, NHC(O)-1-adamantyl, NHC(O)O-3-tetrahydrofuranyl, - NHC(O)O-4-tetrahydropyranyl, -NHC(O)CH₂C(CH₃)₃, -NHC(O)C(CH₃)₃, -NHC(O)OC₁-C₁₀ alkyl, -NHC(O)NHC₁-C₁₀ alkyl, -NHC(O)NHPh, NHC(O)NHPh substituted with one, 2, or 3 C₁-C₄ alkyl, C₁-C₃ alkoxy, halo, C₁-C₃ alkylthio, trifluoromethyl, C₂-C₆ dialkylamino, or nitro, -NHC(O)C₃-C₈ cycloalkyl, -NHC(O)C(CH₂CH₃)₂CH₃, -NHC(O)C(CH₃)₂CH₂Cl, - NHC(O)C(CH₃)₂CH₂CH₃, phthalimido, -NHC(O)-1-phenyl-1-cyclopentyl, NHC(O)-1-methyl-1-cyclohexyl, -NHC(S)NHC(CH₃)₃, -NHC(O)NHCC(CH₃)₃, or -NHC(O)NHPh; R₃ is selected from the group consisting of -H, -NHC(O)phenyl, or -NHC(O)OC(CH₃)₃, with the overall proviso that one of R₂ and R₃ is -H but R₂ and R₃ are not both -H; R₄ is -H or selected from the group consisting of -OH, -OAc (-OC(O)CH₃), -OC(O)OCH₂C(Cl)₃, - OCOCH₂CH₂NH₃⁺ HCOO⁻, -NHC(O)phenyl, -NHC(O)OC(CH₃)₃, -OCOCH₂CH₂COOH and pharmaceutically acceptable salts thereof, -OCO(CH₂)₃COOH and pharmaceutically acceptable salts thereof, and -OC(O)-Z-C(O)-R' [where Z is ethylene (-CH₂CH₂-), propylene (-CH₂CH₂CH₂-), -CH=CH-, 1,2-cyclohexane, or 1,2-phenylene, R' is -OH, -OH base, -NR'₂R'₃, -OR'₃, -SR'₃, -OCH₂C(O)NR'₄R'₅ where R'₂ is -H or -CH₃, R'₃ is - (CH₂)ₙNR'₆R'₇ or (CH₂)ₙN⁺R'₆R'₇R'₈X⁻ where n is 1-3, R'₄ is -H or -C₁-C₄ alkyl, R'₅ is -H, -C₁-C₄ alkyl, benzyl, hydroxyethyl, -CH₂CO₂H, or dimethylaminoethyl, R'₆ and R'₇ are-CH₃, -CH₂CH₃, benzyl or R'₆ and R'₇ together with the nitrogen of NR'₆R'₇ form a pyrrolidino, piperidino, morpholino, or N-methylpiperizino group; R'₈ is -CH₃, -CH₂CH₃ or benzyl, X- is halide, and base is NH₃, (HOC₂H₄)₃N, N(CH₃)₃, CH₃N(C₂H₄)₂NH, NH₂(CH₂)₆NH₂, N-methylglucamine, NaOH, or KOH], -OC(O)(CH₂)ₙNR²R³ [where n is 1-3, R² is -H or -C₁-C₃ alkyl and R³ is -H or -C₁-C₃ alkyl], -OC(O)CH(R")NH₂ [where R" is selected from the group consisting of -H, -CH₃, -CH₂CH(CH₃)₂, -CH(CH₃)CH₂CH₃,-CH(CH₃)₂, -CH₂ phenyl, -(CH₂)₄NH₂, -CH₂CH₂ COOH, -(CH₂)₃NHC(=NH)NH₂], the residue of the amino acid proline, -OC(O)CH=CH₂, -C(O)CH₂CH₂C(O)NHCH₂CH₂SO₃⁻Y⁺, -OC(O)CH₂CH₂C(O)NHCH₂CH₂CH₂SO₃⁻Y⁺ wherein Y⁺ is Na⁺ or N⁺(Bu)₄,-OC(O)CH₂CH₂C(O)OCH₂CH₂OH; R₅ is -H or -OH, with the overall proviso that when R₅ is -OH, R₄ is -H and with the further proviso that when R₅ is -H, R₄ is not -H; R₆ is -H:-H when R₇ is α-R₇₁:β-R₇₂ where one of R₇₁ and R₇₂ is -H and the other of R₇₁ and R₇₂ is -X where X is halo and R₈ is -CH₃; R₆ is -H:-H when R₇ is α-H: β-R₇₄ where R₇₄ and R₈ are taken together to form a cyclopropyl ring; R₁₀ is -H or -C(O)CH₃; and pharmaceutically acceptable salts thereof when the compound contains either an acidic or basic functional group.

Exemplary embodiments of paclitaxel (Taxol®) derivatives (or analogs) include derivatives disclosed and referred to in U.S. Patent No. 6,911,549 issued on June 28, 2005.

Particular paclitaxel derivatives include docetaxel (Taxotere^{®}), ((azidophenyl)ureido)taxoid, (2α,5α,7β,9α,10β,13α)-5,10,13,20-tetraacetoxytax-11-ene-2,7,9-triol, (2α,5α,9α,10β-2,9,10-triacetoxy-5-((β-D-glucopyranosyl)oxy)-3,11-cyclotax-11-en-13-one, 1 β-hydroxybaccatin I, 1,7-dihydroxytaxinine, 1-acety-5,7,10-deacetyl-baccatin I, 1-dehydroxybaccatin VI, 1-hydroxy-2-deacetoxy-5-decinnamoyl-taxinine j, 1-hydroxy-7,9-dideacetylbaccatin I, 1-hydroxybaccatin I, 10-acetyl-4-deacetyltaxotere, 10-deacetoxypaclitaxel, 10-Deacetyl baccatin III dimethyl sulfoxide disolvate, 10-deacetyl-10-(3-aminobenzoyl)paclitaxel, 10-deacetyl-10-(7-(diethylamino)coumarin-3-carbonyl)paclitaxel, 10-deacetyl-9-dihydrotaxol, 10-deacetylbaccatine III, 10-deacetylpaclitaxel, 10-deacetyltaxinine, 10-deacetyltaxol, 10-deoxy-10-C-morpholinoethyl docetaxel, 10-O-acetyl-2-O-(cyclohexylcarbonyl)-2-debenzoyltaxotere, 10-O-sec-aminoethyl docetaxel, 11-desmethyllaulimalide, 13-deoxo-13-acetyloxy-7,9-diacetyl-1,2-dideoxytaxine, 13-deoxybaccatin III, 14-hydroxy-10-deacetyl-2-O-debenzoylbacatin III, 14-hydroxy-10-deacetylbaccatin III, 14β-benzoyloxy-13-deacetylbaccatin IV, 14β-benzoyloxy-2-deacetylbaccatin VI, 14β-benzoyloxybaccatin IV, 19-hydroxybaccatin III, 2',2"-methylenedocetaxel, 2',2"-methylenepaclitaxel, 2'-(valyl-leucyl-lysyl-PABC)paclitaxel, 2'-acetyltaxol, 2'-O-acetyl-7-O-(N-(4'-fluoresceincarbonyl)alanyl)taxol, 2,10,13-triacetoxy-taxa-4(20),11-diene-5,7,9-triol, 2,20-0-diacetyltaxumairol N, 2-(4-azidobenzoyl)taxol, 2-deacetoxytaxinine J, 2-debenzoyl-2-m-methoxybenozyl-7-triethylsilyl-13-oxo-14-hydroxybaccatin III 1,14-carbonate, 2-O-(cyclohexylcarbonyl)-2-debenzoylbaccatin III 13-O-(N-(cyclohexylcarbonyl)-3-cyclohexylisoserinate), 2α,7β,9α,10β,13α-pentaacetoxyltaxa-4 (20), 11-dien-5-ol, 2α,5α,7β,9α,13α-pentahydroxy-10β-acetoxytaxa-4(20),11-diene, 2α,7β,9α,10β,13-pentaacetoxy-11β-hydroxy-5α-(3'-N,N-dimethylamino-3'-phenyl)-propionyloxytaxa-4(20),12-diene, 2α,7β-diacetoxy-5α,10β,13β-trihydroxy-2(3-20)abeotaxa-4(20),11-dien-9-one, 2α,9α-dihydroxy-10β,13α-diacetoxy-5α-(3'-methylamino-3'-phenyl)-propionyloxytaxa-4(20),11-diene, 2α-hydroxy-7β,9α,10β,13α-tetraacetoxy-5α-(2'-hydroxy-3'-N,N-dimethylamino-3'-phenyl)-propionyloxytaxa-4(20),11-diene, 3'-(4-azidobenzamido)taxol, 3'-N-(4-benzoyldihydrocinnamoyl)-3'-N-debenzoylpaclitaxel, 3'-N-m-aminobenzamido-3'-debenzamidopaclitaxel, 3'-p-hydroxypaclitaxel, 3,11-cyclotaxinine NN-2, 4-deacetyltaxol, 5,13-diacetoxy-taxa-4(20),11-diene-9,10-diol, 5-O-benzoylated taxinine K, 5-O-phenylpropionyloxytaxinine A, 5α,13α-diacetoxy-10β-cinnamoyloxy-4(20),11-taxadien-9α-ol, 6,3'-p-dihydroxypaclitaxel, 6-α-hydroxy-7-deoxy-10-deacetylbaccatin-III, 6-fluoro-10-acetyldocetaxel, 6-hydroxytaxol, 7,13-diacetoxy-5-cinnamyloxy-2(3-20)-abeo-taxa-4(20),11-diene-2,10-diol, 7,9-dideacetylbaccatin VI, 7-(5'-Biotinylamidopropanoyl)paclitaxel, 7-acetyltaxol, 7-deoxy-10-deacetylbaccatin-III, 7-deoxy-9-dihydropaclitaxel, 7-epipaclitaxel, 7-methylthiomethylpaclitaxel, 7-O-(4-benzoyldihydrocinnamoyl)paclitaxel, 7-O-(N-(4'-fluoresceincarbonyl)alanyl)taxol, 7-xylosyl-10-deacetyltaxol, 8,9-single-epoxy brevifolin, 9-dihydrobaccatin III, 9-dihydrotaxol, 9α-hydroxy-2α,10β,13α-triacetoxy-5α-(3'-N,N-dimethylamino-3'-phenyl)-propionyloxytaxa-4(20),11-diene, baccatin III, baccatin III 13-0-(N-benzoyl-3-cyclohexylisoserinate), BAY59, benzoyltaxol, BMS 181339, BMS 185660, BMS 188797, brevifoliol, butitaxel, cephalomannine, dantaxusin A, dantaxusin B, dantaxusin C, dantaxusin D, dibromo-10-deacetylcephalomannine, DJ927, Flutax 2, glutarylpaclitaxel 6-aminohexanol glucuronide, IDN 5109, IDN 5111, IDN 5127, IDN 5390, isolaulimalide, laulimalide, MST 997, N-(paclitaxel-2'-O-(2-amino)phenylpropionate)-O-(β-glucuronyl)carbamate, N-(paclitaxel-2'-O-3,3-dimethyl butanoate)-O-(β-glucuronyl)carbamate, N-debenzoyl-N-(3-(dimethylamino)benzoyl)paclitaxel, nonataxel, octreotide-conjugated paclitaxel, Paclitaxel, paclitaxel-transferrin, PNU 166945, poly(ethylene glycol)-conjugated paclitaxel-2'-glycinate, polyglutamic acid-paclitaxel, protax, protaxel, RPR 109881A, SB T-101187, SB T-1102, SB T-1213, SB T-1214, SB T-1250, SB T-12843, tasumatrol E, tasumatrol F, tasumatrol G, taxa-4(20),11(12)-dien-5-yl acetate, taxa-4(20),11(12)-diene-5-ol, taxane, taxchinin N, taxcultine, taxezopidine M, taxezopidine N, taxine, taxinine, taxinine A, taxinine M, taxinine NN-1, taxinine NN-7, taxol C-7-xylose, taxol-sialyl conjugate, taxumairol A, taxumairol B, taxumairol G, taxumairol H, taxumairol I, taxumairol K, taxumairol M, taxumairol N, taxumairol O, taxumairol U, taxumairol V, taxumairol W, taxumairol-X, taxumairol-Y, taxumairol-Z, taxusin, taxuspinanane A, taxuspinanane B, taxuspine C, taxuspine D, taxuspine F, taxuyunnanine C, taxuyunnanine S, taxuyunnanine T, taxuyunnanine U, taxuyunnanine V, tRA-96023, and wallifoliol. Other paclitaxel analogs include 1-deoxypaclitaxel, 10-deacetoxy-7-deoxypaclitaxel, 10-O-deacetylpaclitaxel 10-monosuccinyl ester, 10-succinyl paclitaxel, 12b-acetyloxy-2a,3,4,4a,5,6,9,10,11,12,12a,12b-dodecahydro-4,11-dihydroxy-12-(2,5-dimethoxybenzyloxy)-4a,8,13,13-tetramethyl-5-oxo-7,11-methano-1H-cyclodeca(3,4)benz(1,2-b)oxet-9-yl 3-(tert-butyloxycarbonyl)amino-2-hydroxy-5-methyl-4-hexaenoate, 130-nm albumin-bound paclitaxel, 2'-paclitaxel methyl 2-glucopyranosyl succinate, 3'-(4-azidophenyl)-3'-dephenylpaclitaxel, 4-fluoropaclitaxel, 6,6,8-trimethyl-4,4a,5,6,7,7a,8,9-octahydrocyclopenta(4,5)cyclohepta(1,2-c)-furan-4,8-diol 4-(N-acetyl-3-phenylisoserinate), 6,6,8-trimethyl-4,4a,5,6,7,7a,8,9-octahydrocyclopenta(4,5)cyclohepta(1,2-c)-furan-4,8-diol 4-(N-tert-butoxycarbonyl-3-phenylisoserinate), 7-(3-methyl-3-nitrosothiobutyryl)paclitaxel, 7-deoxypaclitaxel, 7-succinylpaclitaxel, A-Z-CINN 310, AI-850, albumin-bound paclitaxel, AZ 10992,isotaxel, MAC321, MBT-0206, NK105, Pacliex, paclitaxel poliglumex, paclitaxel-EC-1 conjugate, polilactofate, and TXD 258. Other paclitaxel analogs are described in U.S. Patents Nos. 4,814,470, 4,857,653, 4,942,184, 4,924,011, 4,924,012,4,960,790; 5,015,744; 5,157,049; 5,059,699; 5,136,060; 4,876,399; and 5,227,400.

Exemplary etoposide derivatives (or analogs) include podophyllotoxin derivatives. Other derivatives of etoposide include etoposide phosphate (ETOPOPHOS^{®}), etoposide 4'-dimethylglycine, etoposide_{DMG}, teniposide, and NK611, or any pharmaceutically acceptable salts thereof (e.g., -OP(O)(ONa)₂). Still other podophyllotoxin derivatives suitable for use in the invention are described in U.S. Patent Nos. 4,567,253; 4,609,644; 4,900,814; 4,958,010; 5,489,698; 5,536,847; 5,571,914; 6,051,721; 6,107,284; 6,475,486; 6,610,299; 6,878,746; 6,894,075; 7,087,641; 7,176,236; 7,241,595; 7,342,114; and 7,378,419; and in U.S. Patent Publication Nos. 20030064482, 20030162722, 20040044058, 20060148728, and 20070249651.

Exemplary doxorubicin (hydroxydaunorubicin or Adriamycin^{®}) derivatives (or analogs) include epirubicin (Ellence^{®} or Pharmorubicin^{®}). Other doxorubicin derivatives can be found in U.S. Patent Nos. 4,098,884, 4,301,277, 4,314,054, 4,464,529,4,585,859, 4,672,057, 4,684,629, 4,826,964, 5,200,513, 5,304,687, 5,594,158, 5,625,043, and 5,874,412.

### Therapeutic nucleic acid agents

The targeting moiety may be conjugated to any therapeutic nucleic acid agent, including expression vectors (*e*.*g*., a plasmid) and RNAi agents. The expression vector may encode a polypeptide (*e*.*g*., a therapeutic polypeptide such as an interferon, a therapeutic cytokine (*e.g*., IL-12), or FGF-2) or may encode a therapeutic nucleic acid (*e*.*g*., an RNAi agent such as those described herein). Nucleic acids include any type known in the art, such as double and single-stranded DNA and RNA molecules of any length, conformation, charge, or shape (*i*.*e*., linear, concatemer, circular (*e*.*g*., a plasmid), nicked circular, coiled, supercoiled, or charged). Additionally, the nucleic acid can contain 5' and 3' terminal modifications and include blunt and overhanging nucleotides at these termini, or combinations thereof. In certain embodiments of the invention, the nucleic acid is or encodes an RNA interference sequence (*e*.*g*., an siRNA, shRNA, miRNA, or dsRNA nucleotide sequence) that can silence a targeted gene product. The nucleic acid can be, for example, a DNA molecule, an RNA molecule, or a modified form thereof.

Exemplary RNAi targets include growth factors (*e*.*g*., epidermal growth factor (EGF), vascular endothelial growth factor (VEGF), transforming growth factor-β (TGF-β)), growth factor receptors, including receptor tyrosine kinases (*e*.*g*., EGF receptor (EGFR), including Her2/neu (ErbB), VEGF receptor (VEGFR), platelet-derived growth factor receptor (PDGFR), cytokines, chemokines, kinases, including cytoplasmic tyrosine and serine/threonine kinases (*e*.*g*., focal adhesion kinase, cyclin-dependent kinase, SRC kinases, syk-ZAP70 kinases, BTK kinases, RAF kinase, MAP kinases (including ERK), and Wnt kinases), phosphatases, regulatory GTPases (*e*.*g*., Ras protein), transcription factors (*e*.*g*., MYC), hormones and hormone receptors (*e*.*g*., estrogen and estrogen receptor), anti-apoptotic molecules (*e*.*g*., survivin, Bcl-2, Bcl-xL), oncogenes (*e*.*g*., tumor suppressor regulators such as mdm2), enzymes (*e*.*g*., superoxide dismutase 1 (SOD-1), α, β (BACE), and γ secretases, alpha-L-iduronidase, iduronate sulfatase, heparan N-sulfatase, alpha-N-acetylglucosaminidase, acetyl-CoAlpha-glucosaminide acetyltransferase, N-acetylglucosamine 6-sulfatase, N-acetylgalactosamine 4-sulfatase, beta-galactosidase, sphingomyelinase, glucocerebrosidase, alpha-galactosidase-A, ceramidase, galactosylceramidase, arylsulfatase A, aspartoacylase, phytanoyl-CoA hydroxylase, peroxin-7, beta-hexosaminidase A, aspartyl-glucosaminidase, fucosidase, and alpha-mannosidase, sialidase), and other proteins (*e*.*g*., Huntingtin (Htt protein), amyloid precursor protein (APP), sorting nexins (including SNX6), α-synuclein, LINGO-1, Nogo-A, and Nogo receptor 1 (NgR-1)), and glial fibrillary acidic protein. Table 3 illustrates the relationship between exemplary RNAi targets and diseases.

Exemplary RNAi sequences to silence EGFR are SEQ ID NO: 117 (GGAGCUGCCCAUGAGAAAU) and SEQ ID NO:118 (AUUUCUCAUGGGCAGCUCC). Likewise, VEGF can be silenced with an RNAi molecule having the sequence, for example, set forth in SEQ ID NO: 119 (GGAGTACCCTGATGAGATC). Additional RNAi sequences for use in the agents of the invention may be either commercially available (*e*.*g*., Dharmacon, Ambion) or the practitioner may use one of several publicly available software tools for the construction of viable RNAi sequences (*e*.*g*., The siRNA Selection Server, maintained by MIT/Whitehead; available at: http://jura.wi.mit.edu/bioc/siRNAext/. Examples of diseases or conditions, and RNAi target that may be useful in treatment of such diseases, are shown in Table 3.

**Table 3: Exemplary Diseases and Target Molecules**

| **Disease/Condition** | **RNAi Target Molecules** |
|---|---|
| ***Cancer*** | |
| Glioblastoma | Epidermal growth factor receptor (EGFR), Vascular endothelial growth factor (VEGF) |
| Glioma | EGFR, VEGF |
| Astrocytoma | EGFR, VEGF |
| Neuroblastoma | EGFR, VEGF |
| Lung cancer | EGFR, VEGF |
| Breast cancer | EGFR, VEGF |
| Hepatocellular carcinoma | EGFR, VEGF |
| ***Neurodegenerative Disease*** | |
| Huntington's disease | Huntingtin (Htt) |
| Parkinson's disease | Alpha-synuclein |
| Alzheimer's disease | Amyloid precursor protein (APP), Presenilin-1 or -2, Apolipoprotein E (ApoE) |
| Amyotropic lateral sclerosis | Superoxide dismutase 1 (SOD-1) |
| Multiple sclerosis | Sorting nexin-6 (SNX6), LINGO-1, Nogo-A, NgR-1, APP |
| ***Lysosomal Storage Disease*** | |
| MPS-I (Hurler, Scheie diseases) | Alpha-L-iduronidase |
| MPS-II (Hunter syndrome) | Iduronate sulfatase |
| MPS-IIIA (Sanfilippo syndrome A) | Heparan N-sulfatase |
| MPS-IIIB (Sanfilippo syndrome B) | Alpha-N-acetylglucosaminidase |
| MPS-IIIC (Sanfilippo syndrome C) | Acetyl-CoAlpha-glucosaminide acetyltransferase |
| MPS-IIID (Sanfilippo syndrome D) | N-acetylglucosamine 6-sulfatase |
| MPS-VI (Maroteaux-Lamy syndrome) | N-acetylgalactosamine 4-sulfatase |
| MPS-VII (Sly syndrome) | Beta-glucuronidase |
| Niemann-Pick disease | Sphingomyelinase |
| Gaucher's disease | Glucocerebrosidase |
| Fabry disease | Alpha-galoctosidase-A |
| Farber's disease | Ceramidase |
| Krabbé disease | Galactosylceramidase |
| Metachromatic leukodystrophy | Arylsulfatase A |
| Alexander disease | Glial fibrillary acidic protein |
| Canavan disease | Aspartoacylase |
| Refsum's disease | Phytanoyl-CoA hydroxylase or peroxin-7 |
| GM1 gangliosidoses | Beta-galactosidase |
| GM2 gangliosidoses (*e*.*g*., Tay-Sachs, Sandhoff diseases) | Beta-hexosaminidase A |
| Aspartylglucosaminuria | Aspartylglucossminidase (AGA). |
| Fucosidosis | Fucosidase |
| Mannosidosis | Alpha-mannosidase |
| Mucolipodosis (sialidosis | Sialidase |

### Small molecule drugs

Any small molecule drug can be linked with the targeting moiety. Small molecule drugs include an anticancer agent, an antibiotic, a cytotoxic agent, an alkylating agent, an antineoplastic agent, an antimetabolic agent, an antiproliferative agent, a tubulin inhibitor, a topoisomerase I or II inhibitor, a hormonal agonist or antagonist, an apoptotic agent, an immunomodulator, and a radioactive agent (*e*.*g*., an isotope), or any agent described herein. Exemplary small molecule drugs include paclitaxel (Taxol®), a paclitaxel derivative (*e*.*g*., docetaxel (Taxotere®)), vinblastine, vincristine, etoposide, doxorubicin, cyclophosphamide, melphalan, chlorambucil, methotrexate, camptothecin, homocamptothecin, thiocolchicine, colchicine, combretastatin, combretastin A-4, podophyllotoxin, rhizoxin, rhizoxin-d, dolistatin, CC1065, ansamitocin p3, maytansinoid, and any pharmaceutically acceptable salts, etc. and combinations thereof, as well as any drug which may be a P-gp substrate.

Exemplary small molecule drugs include analgesics and antiinflammatory agents (*e*.*g*., aloxiprin, auranofin, azapropazone, benorylate, diflunisal, etodolac, fenbufen, fenoprofen calcim, flurbiprofen, ibuprofen, indomethacin, ketoprofen, meclofenamic acid, mefenamic acid, nabumetone, naproxen, oxyphenbutazone, phenylbutazone, piroxicam, sulindac), antibiotics (*e*.*g*., penicillin, cephalosporins, aminoglycosides, macrolides, quinolones, and tetracyclines), antihelmintics (*e*.*g*., albendazole, bephenium hydroxynaphthoate, cambendazole, dichlorophen, ivermectin, mebendazole, oxamniquine, oxfendazole, oxantel embonate, praziquantel, pyrantel embonate, thiabendazole), anti-arrhythmic agents (*e.g*., amiodarone (*e*.*g*., HCl), disopyramide, flecainide (*e*.*g*., acetate), quinidine (*e*.*g*., sulfate), anti-bacterial agents (*e*.*g*., benethamine penicillin, cinoxacin, ciprofloxacin (*e*.*g*., HCl), clarithromycin, clofazimine, cloxacillin, demeclocycline, doxycycline, erythromycin, ethionamide, imipenem, nalidixic acid, nitrofurantoin, rifampicin, spiramycin, sulphabenzamide, sulphadoxine, sulphamerazine, sulphacetamide, sulphadiazine, sulphafurazole, sulphamethoxazole, sulphapyridine, tetracycline, trimethoprim), anticoagulants (*e*.*g*., dicoumarol, dipyridamole, nicoumalone, phenindione), antidepressants (*e*.*g*., amoxapine, maprotiline (*e*.*g*., HCl), mianserin (*e*.*g*., HCl), nortriptyline (*e*.*g*., HCl), trazodone (*e*.*g*., HCl), trimipramine (*e*.*g*., maleate)), antidiabetics (*e*.*g*., acetohexamide, chlorpropamide, glibenclamide, gliclazide, glipizide, tolazamide, tolbutamide), anti-epileptics (*e*.*g*., beclamide, carbamazepine, clonazepam, ethotoin, methoin, methsuximide, methylphenobarbitone, oxcarbazepine, paramethadione, phenacemide, phenobarbitone, phenytoin, phensuximide, primidone, sulthiame, valproic acid), antifungal agents (*e*.*g*., amphotericin, butoconazole (*e*.*g*., nitrate), clotrimazole, econazole (*e*.*g*., nitrate), fluconazole, flucytosine, griseofulvin, itraconazole, ketoconazole, miconazole, natamycin, nystatin, sulconazole (*e*.*g*., nitrate), terbinafine (*e.g*., HCl), terconazole, tioconazole, undecenoic acid), antigout agents (*e*.*g*., allopurinol, probenecid, sulphin-pyrazone), antihypertensive agents (*e*.*g*., amlodipine, benidipine, darodipine, dilitazem (*e*.*g*., HCl), diazoxide, felodipine, guanabenz (*e*.*g*., acetate), isradipine, minoxidil, nicardipine (*e*.*g*., HCl), nifedipine, nimodipine, phenoxybenzamine (*e*.*g*., HCl), prazosin (*e*.*g*., HCl), reserpine, terazosin (*e*.*g*., HCl)), antimalarials (*e*.*g*., amodiaquine, chloroquine, chlorproguanil (*e*.*g*., HCl), halofantrine (*e*.*g*., HCl), mefloquine (*e*.*g*., HCl), proguanil (*e*.*g*., HCl), pyrimethamine, quinine sulphate), anti-migraine agents (*e*.*g*., dihydroergotamine (*e*.*g*., mesylate), ergotamine (*e*.*g*., tartrate), methysergide (*e*.*g*., maleate), pizotifen (*e*.*g*., maleate), sumatriptan succinate), anti-muscarinic agents (*e*.*g*., atropine, benzhexol (*e*.*g*., HCl), biperiden, ethopropazine (*e*.*g*., HCl), hyoscyamine, mepenzolate (*e*.*g*., bromide), oxyphencylcimine (*e*.*g*., HCl), tropicamide), anticancer agents and immunosuppressants (*e*.*g*., aminoglutethimide, amsacrine, azathioprine, busulphan, chlorambucil, cyclosporin, dacarbazine, doxorubicin, estramustine, etoposide, lomustine, melphalan, mercaptopurine, methotrexate, mitomycin, mitotane, mitozantrone, paclitaxel, procarbazine (*e*.*g*., HCl), tamoxifen (*e*.*g*., citrate), testolactone), anti-protazoal agents (*e*.*g*., benznidazole, clioquinol, decoquinate, diiodohydroxyquinoline, diloxanide furoate, dinitolmide, furzolidone, metronidazole, nimorazole, nitrofurazone, ornidazole, tinidazole), anti-thyroid agents (*e*.*g*., carbimazole, propylthiouracil), anxiolytic, sedatives, hypnotics and neuroleptics (*e*.*g*., alprazolam, amylobarbitone, barbitone, bentazepam, bromazepam, bromperidol, brotizolam, butobarbitone, carbromal, chlordiazepoxide, chlormethiazole, chlorpromazine, clobazam, clotiazepam, clozapine, diazepam, droperidol, ethinamate, flunanisone, flunitrazepam, fluopromazine, flupenthixol decanoate, fluphenazine decanoate, flurazepam, haloperidol, lorazepam, lormetazepam, medazepam, meprobamate, methaqualone, midazolam, nitrazepam, oxazepam, pentobarbitone, perphenazine pimozide, prochlorperazine, sulpiride, temazepam, thioridazine, triazolam, zopiclone), β-Blockers (*e*.*g*., acebutolol, alprenolol, atenolol, labetalol, metoprolol, nadolol, oxprenolol, pindolol, propranolol), cardiac inotropic agents (*e*.*g*., amrinone, digitoxin, digoxin, enoximone, lanatoside C, medigoxin), corticosteroids (*e*.*g*., beclomethasone, betamethasone, budesonide, cortisone (*e*.*g*., acetate), desoxymethasone, dexamethasone, fludrocortisone (*e*.*g*., acetate), flunisolide, flucortolone, fluticasone (*e*.*g*., propionate), hydrocortisone, methylprednisolone, prednisolone, prednisone, triamcinolone), diuretics (*e*.*g*., acetazolamide, amiloride, bendrofluazide, bumetanide, chlorothiazide, chlorthalidone, ethacrynic acid, frusemide, metolazone, spironolactone, triamterene), anti-parkinsonian agents (*e*.*g*., bromocriptine (*e*.*g*., mesylate), lysuride (*e*.*g*., maleate)), gastrointestinal agents (*e*.*g*., bisacodyl, cimetidine, cisapride, diphenoxylate (*e*.*g*., HCl), domperidone, famotidine, loperamide, mesalazine, nizatidine, omeprazole, ondansetron (*e*.*g*., HCl), ranitidine (*e*.*g*., HCl), sulphasalazine), histamine H-receptor antagonists (*e*.*g*., acrivastine, astemizole, cinnarizine, cyclizine, cyproheptadine (*e*.*g*., HCl), dimenhydrinate, flunarizine (*e*.*g*., HCl), loratadine, meclozine (*e*.*g*., HCl), oxatomide, terfenadine), lipid regulating agents (*e*.*g*., bezafibrate, clofibrate, fenofibrate, gemfibrozil, probucol), nitrates and other anti-anginal agents (*e*.*g*., amyl nitrate, glyceryl trinitrate, isosorbide dinitrate, isosorbide mononitrate, pentaerythritol tetranitrate), opioid analgesics (*e*.*g*., codeine, dextropropyoxyphene, diamorphine, dihydrocodeine, meptazinol, methadone, morphine, nalbuphine, pentazocine), sex hormones (*e*.*g*., clomiphene (*e*.*g*., citrate), danazol, ethinyl estradiol, medroxyprogesterone (*e*.*g*., acetate), mestranol, methyltestosterone, norethisterone, norgestrel, estradiol, conjugated oestrogens, progesterone, stanozolol, stibestrol, testosterone, tibolone), and stimulants (*e*.*g*., amphetamine, dexamphetamine, dexfenfluramine, fenfluramine, mazindol). The invention may also include analogs of any of these agents (*e*.*g*., therapeutically effective analogs).

### Labels

A label can be linked to the targeting moiety to allow for diagnostic and/or therapeutic treatment. Examples of labels include detectable labels, such as an isotope, a radioimaging agent, a marker, a tracer, a fluorescent label (*e*.*g*., rhodamine), and a reporter molecule (*e*.*g*., biotin).

Examples of radioimaging agents emitting radiation (detectable radio-labels) that may be suitable are exemplified by indium-111, technitium-99, or low dose iodine-131. Detectable labels, or markers, for use in the present invention may be a radiolabel, a fluorescent label, a nuclear magnetic resonance active label, a luminescent label, a chromophore label, a positron emitting isotope for PET scanner, chemiluminescence label, or an enzymatic label. Fluorescent labels include but are not limited to, green fluorescent protein (GFP), fluorescein, and rhodamine. Chemiluminescence labels include but are not limited to, luciferase and β-galactosidase. Enzymatic labels include but are not limited to peroxidase and phosphatase. A histamine tag may also be a detectable label. For example, conjugates may comprise a carrier moiety and an antibody moiety (antibody or antibody fragment) and may further comprise a label. The label may be for example a medical isotope, such as for example and without limitation, technetium-99, iodine-123 and -131, thallium-201, gallium-67, fluorine-18, indium-111, etc.

### Therapeutic peptidic agents

Therapeutic peptidic agents include a broad class of agents based on proteins or peptides (*e*.*g*., any useful peptidic- or protein-based drug). Exemplary therapeutic peptidic agents include, without limitation, a peptidic- or protein-based drug (*e*.*g*., a positive pharmacological modulator (agonist) or a pharmacological inhibitor (antagonist)) *etc.*

The conjugate may be a therapeutic polypeptide (*e*.*g*., a fusion protein) consisting essentially of the targeting moiety and a protein. Exemplary therapeutic peptidic agents include cellular toxins (*e*.*g*., monomethyl auristatin E (MMAE), bacteria endotoxins and exotoxins, diphtheria toxins, botulinum toxin, tetanus toxins, perussis toxins, staphylococcus enterotoxins, toxic shock syndrome toxin TSST-1, adenylate cyclase toxin, shiga toxin, and cholera enterotoxin), anti-angiogenic compounds (*e*.*g*., endostatins, chemokines, inhibitors of matrix metalloproteinase (MMPIs), anastellin, vitronectin, antithrombin, tyrosine kinase inhibitors, and VEGF inhibitors), hormones (*e*.*g*., growth hormone), and cytokines (*e*.*g*., granulocyte-macrophage colony-stimulating factor, interleukins, lymphokines, and chemokines).

Other therapeutic peptidic agents that may be included in a conjugate of the invention are adrenocortiocotropic hormones (ACTH, corticotropin), growth hormone peptides (*e*.*g*., human placental lactogen (hPL), growth hormones, and prolactin (Prl)), melanocyte stimulating hormones (MSH), oxytocin, vasopressin (ADH), corticotropin releasing factor (CRF), gonadotropin releasing hormone associated peptides (GAP), growth hormone releasing factor (GRF), lutenizing hormone release hormones (LH-RH), orexins, prolactin releasing peptides, somatostatin, thyrotropin releasing hormone (THR), calcitonin (CT), caltitonin precursor peptide, calcitonins gene related peptide (CGRP), parathyroid hormones (PTH), parathyroid hormone related proteins (PTHrP), amylin, glucagon, insulin and insulin-like peptides, neuropeptide Y, pancreatic polypeptide (PP), peptide YY, somatostatin, cholecystokinin (CCK), gastrin releasing peptide (GRP), gastrin, gastrin inhibitory peptide, motilin, secretin, vasoactive intestinal peptide (VIP), natriuretic peptides (*e*.*g*., atrial natriuretic peptide (ANP), B-type natriuretic peptide (BNP), brain natriuretic peptide, and C-type natriuretic peptide (CNP)), tachykinins (*e*.*g*., neurokinin A, neurokinin B, and substance P), substance P, angiotensins (*e*.*g*., angiotensin I and angiotensin II), renin, endothelins (*e*.*g*., endothelin-1, endothelin-2, endothelin-3, sarafotoxin (a snake venom) and scorpion toxin), sarafotoxin peptides, opioid peptides (*e*.*g*., casomorphin peptides, demorphins, endorphins, enkephalins, deltorphins, dynorphins), thymic peptides (*e*.*g*., thymopoietin, thymulin, thymopentin, thymosin, thymic humoral factor (THF)), adrenomedullin peptides (AM), allatostatin peptides, amyloid beta-protein fragments (Aβ fragments), antimicrobial peptides (*e*.*g*., defensin, cecropin, buforin, and magainin), antioxidant peptides (*e*.*g*., natural killer-enhancing factor B (NKEF-B), bombesin, bone Gla protein peptides (*e*.*g*., osteocalcin (bone Gla-protein, or BGP), CART peptides, cell adhesion peptides, cortistatin peptides, fibronectin fragments and fibrin related peptides, FMRF peptides, galanin, guanylin and uroguanylin, and inhibin peptides.

In particular, the therapeutic peptidic agent is neurotensin or a neurotensin analog, a neurotensin receptor agonist, a neurotrophic factor or a neurotrophic factor analog (*e*.*g*., glial cell line-derived neurotrophic factor (GDNF) or a GDNF analog, or brain-derived neurotrophic factor (BDNF) or a BDNF analog), a GLP-1 agonist, or leptin or a leptin analog. More details regarding these agents are provided below.

### Neurotensin or a neurotensin analog

Neurotensin (NT) is a 13 amino acid peptide found in the central nervous system and in the gastrointestinal tract. In brain, NT is associated with dopaminergic receptors and other neurotransmitter system. Peripheral NT acts as a paracrine and endocrine peptide on both the digestive and cardiovascular systems. To exert its biological effects in the brain NT has to be injected or delivered directly to the brain because NT does not cross the BBB and is rapidly degraded by peptidases following systematic administration. Preclinical pharmacological studies, most of which involve direct injection of NT into the brain, strongly suggest that an agonist of NT receptors would be clinically useful for the treatment of neuropsychiatric conditions including psychosis, schizophrenia, Parkinson's disease, pain, and the abuse of psychostimulants. In particular, in various animal studies, intraventricular injection of NT led to hypothermia and analgesia in antinociception experiments.

The peptide therapeutic may be neurotensin or analog thereof. Human neurotensin is a thirteen amino acid peptide having the sequence QLYENKPRRPYIL. Exemplary neurotensin analogs include (VIP-neurotensin) hybrid antagonist, acetylneurotensin(8-13), JMV 1193, KK13 peptide, neuromedin N, neuromedin N precursor, neurotensin(1-10), neurotensin(1-11), neurotensin(1-13), neurotensin(1-6), neurotensin(1-8), neurotensin(4-13), neurotensin(6-13), neurotensin(8-13), Asp(12)-neurotensin(8-13), Asp(13)-neurotensin(8-13), Cit(8)-neurotensin(8-13), Lys(8)-newotensin(8-13), Cit(9)-neurotensin(8-13), Lys(9)-neurotensin(8-13), N-methyl-Arg(8)-Lys(9)-neo-Trp(11)-neo-Leu(12)-neurotensin(8-13), neurotensin(9-13), neurotensin 69L, Arg(9)-neurotensin, azidobenzoyl-Lys(6)-Trp(11)-neurotensin, Gln(4)-neurotensin, iodo-Tyr(11)-neurotensin, iodo-Tyr(3)-neurotensin, N-α-(fluoresceinylthiocarbamyl)glutamyl(1)-neurotensin, Lys(7)-D-Tyr(11)-neurotensin(7-13) (*e*.*g*., NT1 or KRRP(D-Y)IL), p-Glu(1)-neurotensin or p-Glu(1)-newotensin-OH (e.g, pELYENKPRRPYIL or pELYENKPRRPYIL-OH, where "pE" represents L-pyroglutamic acid), Phe(11)-neurotensin, Ser(7)-neurotensin, Trp(11)-(neurotensin, Tyr(11)-neurotensin, rat NT77, PD 149163, proneurotensin, stearyl-Nle(17)-neurotensin(6-11)VIP(7-28), ^{99m}Tc-NT-XI, TJN 950, and vasoactive intestinal peptide-neurotensin hybrid.

Other neurotensin analogs include shortened neurotensin peptides having one or more substitutions, including substitutions to corresponding D-isomers of the same L-amino acid residues. Exemplary neurotensin analogs include those for neurotensin(6-13) (KPRRPYIL) or neurotensin(7-13) (PRRPYIL), such as D-Lys(6)-neurotensin(6-13), D-Tyr(11)-neurotensin(6-13), D-Lys(6)-D-Tyr(11)-neurotensin(6-13), D-Arg(8)-neurotensin(6-13), D-Arg(9)-neurotensin(6-13), D-Arg(8)-D-Arg(9)-neurotensin(6-13), D-Pro(10)neurotensin(6-13), D-Tyr(11)-neurotensin(6-13), D-Trp(11)-neurotensin(6-13), D-Phe(11)-neurotensin(6-13), D-Arg(8)-D-Tyr(11)-neurotensin(6-13), D-Arg(8)-D-Trp(11)-neurotensin(6-13), and Lys(7)-D-Tyr(11)-neurotensin(7-13) (*e*.*g*., NT1 or KRRP(D-Y)IL); and for neurotensin(8-13), such as D-Arg(8)-neurotensin(8-13), D-Arg(9)-neurotensin(8-13), D-Arg(8)-D-Arg(9)-neurotensin(8-13), D-Pro(10)neurotensin(8-13), D-Tyr(11)-neurotensin(8-13), D-Trp(11)-neurotensin(8-13), D-Phe(11)-neurotensin(8-13), D-Arg(8)-D-Tyr(11)-neurotensin(8-13), and D-Arg(8)-D-Trp(11)-neurotensin(8-13), and acetylated analogs of any of the above.

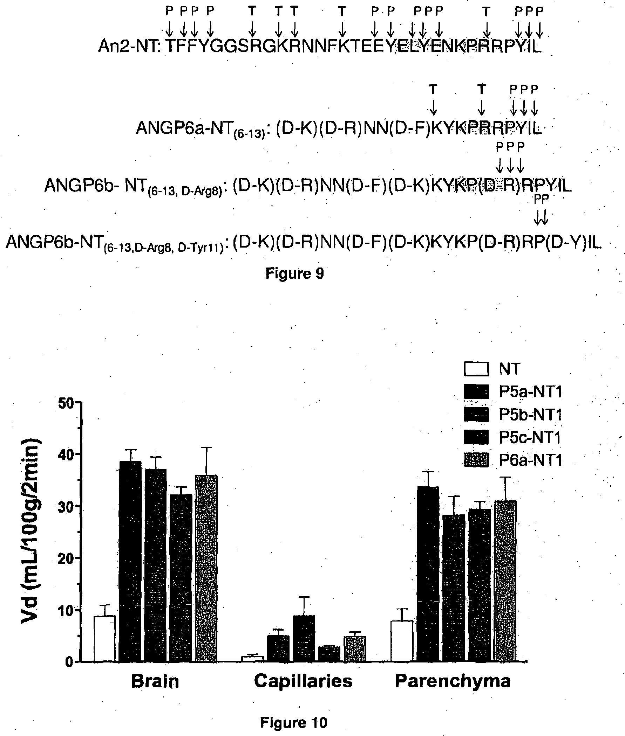

Additional neurotensin analogs include those having one or more D-amino acid substitutions for one or more cleavage sites for pepsin and trypsin. Exemplary cleavage sites for neurotensin are shown in Figure 9, such as C-terminal to positions 1, 2, 3, 11, 12, and 13 for cleavage by pepsin and C-terminal to position 8 for cleavage by trypsin. Accordingly, neurotensin analogs of the invention also include polypeptides shorter than neurotensin having one or more D-amino acid substitutions for one or more of positions 1, 2, 3, 8, 11, 12, and 13 in neurotensin.

Yet other neurotensin analogs include NT64L [L-neo-Trp11]NT(8-13), NT72D [D-Lys9,D-neo-Trp11,tert-Leu12]NT(9-13), NT64D [D-neo-Trp11]NT(8-13), NT73L [D-Lys9,L-neo-Trp11]NT(9-13),NT65L [L-neo-Trp11, tert-Leu12]NT(8-13), NT73D [D-Lys9,D-neo-Trp11]NT(9-13), NT65D [D-neo-Trp11, tert-Leu12]NT(8-13), NT74L [DAB9,L-neo-Trp11,tert-Leu12]NT(9-13), NT66L [D-Lys8, L-neo-Trp11, tert-Leu12]NT(8-13), NT74D [DAB9,Pro,D-neo-Trp11,tert-Leu12]NT(9-13), NT66D [D-Lys8, D-neo-Trp11, tert-Leu12]NT(8-13), NT75L [DAB8 L-neo-Trp11]NT(8-13), NT67L [D-Lys8, L-neo-Trp11]NT(8-13), NT75D [DAB8,D-neo-Trp11]NT(8-13), NT67D [D-Lys8, D-neo-Trp11]NT(8-13), NT76L [D-Orn9,L-neo-Trp11]NT(8-13), NT69L [N-methyl-Arg8,L-Lys9 L-neo-Trp11,tert-Leu12]NT(8-13), NT76D [D-Orn9,D-neo-Trp11]NT(8-13), NT69D [N-methyl-Arg8 L-Lys9,D-neo-Trp11,tert-Leu12]NT(8-13), NT77L [D-Orn9,L-neo-Trp11,tert-Leu12]NT(8-13), NT71L [N-methyl-Arg8,DAB9 L-neo-Trp11,tert-leu12]NT(8-13), NT77D [D-Orn9,D-neo-Trp11,tert-Leu12]NT(8-13),NT71D [N-methyl-Arg8,DAB9,D-neo-Trp11,tert-leu12]NT(8-13), NT78L [N-methyl-Arg8,D-Om9 L-neo-Trp11,tert-Leu12]NT(8-13), NT72L [D-Lys9,L-neo-Trp11,tert-Leu12]NT(9-13), and NT78D [N-methyl-Arg8,D-Orn9,D-neo-Trp11,tert-Leu12]NT(8-13), where neo-Trp is (2-amino-3-[1H-indolyl]propanoic acid). Other neurotensin analogs include Beta-lactotensin (NTR2 selective), JMV-449, and PD-149 or PD-163 (NTR1 selective; reduced amide bond 8-13 fragment of neurotensin).

Other neurotensin analogs include those with modified amino acids (*e*.*g*., any of those described herein). The neurotensin analog may also be a neurotensin receptor agonist. For example, the neurotensin analog can be selective for NTR1, NTR2, or NTR3 (*e*.*g*., may bind to or activate one of NTR1, NTR2, or NTR3 at least 2, 5, 10, 50, 100, 500, 1000, 5000, 10,000, 50,000, or 100,000 greater) as compared to at least one of the other NTR receptors or both.

### Glial cell line-derived neurotrophic factor (GDNF) or a GDNF analog

GDNF is secreted as a disulfide-linked homodimer, and is able to support survival of dopaminergic neurons, Purkinje cells, motoneurons, and sympathetic neurons. GDNF analogs or fragments having one or more of these activities may be used in the present invention, and activity of such analogs and fragments can be tested using any means known in the art.

Human GDNF is expressed as a 211 amino acid protein (isoform 1); a 185 amino acid protein (isoform 2), and a 133 amino acid protein. Mature GDNF is a 134 amino acid sequence that includes amino acids 118-211 of isoform 1, amino acids 92-185 of isoform 2. Isoform 3 includes a transforming growth factor like domain from amino acids 40-133. Other forms of GDNF include amino acids 78-211 of isoform 1.

In certain embodiments, the GDNF analog is a splice variant of GDNF. Such proteins are described in PCT Publication No. WO 2009/053536, and include the pre-(α)pro-GDNF, pre-(β)pro-GDNF, and pre-(γ)pro-GDNF splice variant, as well as the variants lacking the pre-pro region: (α)pro-GDNF, (β)pro-GDNF, and pre-(γ)pro-GDNF.

GDNF analogs also include fragments of a GDNF precursor protein or the biologically active variant. Exemplar GDNF analogs include Pro-Pro-Glu-Ala-Pro-Ala-Glu-Asp-Arg-Ser-Leu-Gly-Arg-Arg; Phe-Pro-Leu-Pro-Ala-Gly-Lys-Arg; FPLPA-amide, PPEAPAEDRSL-amide, LLEAPAEDHSL-amide, SPDKQMAVLP, SPDKQAAALP, SPDKQTPIFS, ERNRQAAAANPENSRGK-amide, ERNRQAAAASPENSRGK-amide, and ERNRQSAATNVENSSKK-amide. Other GDNF analogs are described in U.S. Patent Application Publication Nos. 2009/0069230 and 2006/0258576; and PCT Publication No. WO 2008/069876.

### Brain-derived neurotrophic factor (BDNF) or a BDNF analog

BDNF is glycoprotein of the nerve growth factor family of proteins. The protein is encoded as a 247 amino acid polypeptide (isoform A), a 255 amino acid polypeptide (isoform B), a 262 amino acid polypeptide (isoform C), a 276 amino acid polypeptide (isoform D), and a 329 amino acid polylpeptide (isoform E). The mature 119 amino acid glycoprotein is processed from the larger precursor to yield a neurotrophic factor that promotes the survival of neuronal cell populations. The mature protein includes amino acids 129-247 of the isoform A preprotein, amino acids 137-255 of the isoform B preprotein, amino acids 144-162 of isoform C preprotein, amino acids 158-276 of the isoform D preprotein, or amino acids 211 (or 212) - 329 of the isoform E preprotein. BDNF acts at the TrkB receptor and at low affinity nerve growth factor receptor (LNGFR or p75). BDNF is capable of supporting neuronal survival of existing neurons and can also promote growth and differentiation of new neurons. The BDNF fragments or analogs of the invention may have any of the aforementioned activities. Activity of such analogs and fragments can be tested using any means known in the art. Other BDNF analogs are described in U.S. Patent No. 6,800,607, U.S. Patent Application Publication No. 2004/0072291, and PCT Publication No. WO 96/15146.

### GLP-1 agonist

The targeting moieties described herein can be conjugated to a GLP-1 agonist. Particular GLP-1 agonists include GLP-1, exendin-4, and analogs or fragments thereof. Exemplary analogs are described below.

GLP-1 and GLP-1 analogs can be used in the conjugates and therapeutic polypeptides of the invention. In certain embodiments, the GLP-1 analog is a peptide, which can be truncated, may have one or more substitutions of the wild type sequence (*e*.*g*., the human wild type sequence), or may have other chemical modifications. GLP-1 agonists can also be non-peptide compounds, for example, as described in U.S. Patent No. 6,927,214. Particular analogs include LY548806, CJC-1131, and Liraglutide.

The GLP-1 analog can be truncated form of GLP-1. The GLP-1 peptide may be truncated by 1, 2, 3, 4, 5, 6, 7, 8, 9, 10, 11, 12, 13, 15, 20, or more residues from its N-terminus, its C-terminus, or a combination thereof. In certain embodiments, the truncated GLP-1 analog is the GLP-1(7-34), GLP-1(7-35), GLP-1(7-36), or GLP-1(7-37) human peptide or the C-terminal amidated forms thereof.

The GLP-1 analog can include substitutions, such as an amino acid other than alanine at position 8 or an amino acid other than glycine at position 22 (*e*.*g*., [Glu²²]GLP-1(7-37)OH, [Asp²²]GLP-1(7-37)OH, [Arg²²]GLP-1(7-37)OH, [Lys²²]GLP-1(7-37)OH, [Cya²²]GLP-1(7-37)OH, [Val⁸,Glu²²]GLP-1(7-37)OH, [Val⁸,Asp²²]GLP-1(7-37)OH, [Val⁸,Arg²²]GLP-1(7-37)OH, [Val⁸,Lys²²]GLP-1(7-37)OH, [Val⁸,Cya²²]GLP-1(7-37)OH, [Gly⁸,Glu²²]GLP-1(7-37)OH, [Gly⁸,Asp²²]GLP-1(7-37)OH, [Gly⁸,Arg²²]GLP-1(7-37)OH, [Gly⁸,Lys²²]GLP-1(7-37)OH, [Gly⁸,Cya²²]GLP-1(7-37)OH, [Glu²²]GLP-1(7-36)NH₂, [Asp²²]GLP-1(7-36)NH₂, [Arg²²]GLP-1(7-36)NH₂, [Lys²²]GLP-1(7-36)NH₂, [Cya²²]GLP-1(7-36)NH₂, [Val⁸,Glu²²]GLP-1(7-36)NH₂, [Val⁸,Asp²²]GLP-1(7-36)NH₂, [Val⁸,Arg²²]GLP-1(7-36)NH₂, [Val⁸,Lys²²]GLP-1(7-36)NH₂, [Val⁸,Cya²²]GLP-1(7-36)NH₂, [Gly⁸,Glu²²]GLP-1(7-36)NH₂, [Gly⁸,Asp²²]GLP-1(7-36)NH₂, [Gly⁸,Arg²²]GLP-1(7-36)NH₂, [Gly⁸,Lys²²]GLP-1(7-36)NH₂, [Gly⁸,Cya²²]GLP-1(7-36)NH₂, [Val⁸Lys²³]GLP-1(7-37)OH, [Val⁸,Ala²⁷]GLP-1(7-37)OH, [Val⁸,Glu³⁰]GLP-1(7-37)OH, [Gly⁸,Glu³⁰]GLP-1(7-37)OH, [Val⁸,His³⁵]GLP-1(7-37)OH, [Val⁸,His³⁷]GLP-1(7-37)OH, [Val⁸,Glu²²,Lys²³]GLP-1(7-37)OH, [Val⁸,Glu²²,Glu²]GLP-1(7-37)OH, [Val⁸,Glu²²,Ala²⁷]GLP-1(7-37)OH, [Val⁸,Gly³⁴,Lys³⁵]GLP-1(7-37)OH, [Val⁸,His³⁷]GLP-1(7-37)OH, [Gly⁸,His³⁷]GLP-1(7-37)OH), or a substitution at position 7 with the N-acylated or N-alkylated amino acids (e.g., [D-His⁷]GLP-1(7-37), [Tyr⁷]GLP-1(7-37), [N-acetyl-His⁷]GLP-1(7-37), [N-isopropyl-His⁷]GLP-1(7-37), [D-Ala⁸]GLP-1(7-37), [D-Glu⁹]GLP-1(7-37), [Asp⁹]GLP-1(7-37), [D-Asp⁹]GLP-1(7-37), [D-Phe¹⁰]GLP-1(7-37), [Ser²²,Arg²³,Arg²⁴,Gln²⁶]GLP-1(7-37), and [Ser⁸,Gln⁹,Tyr¹⁶,Lys¹⁸,Asp²¹]GLP-1(7-37)). Other GLP-1 analogs are described in U.S. Patent Nos. 5,545,618, 5,574,008, 5,981,488, 7,084,243, 7,101,843, and 7,238,670.

Exendin-4 and exendin-4 analogs can also be used in the conjugates and therapeutic polypeptides of the invention. The compounds of the invention can include fragments of the exendin-4 sequence. Exendin-4 has the sequence:

His-Gly-Glu-Gly-Thr-Phe-Thr-Ser-Asp-Leu-Ser-Lys-Gln-Met-Glu-Glu-Glu-Ala-Val-Arg-
Leu-Phe-Ile-Glu-Trp-Leu-Lys-Asn-Gly-Gly-Pro-Ser-Ser-Gly-Ala-Pro-Pro-Pro-Ser-NH2

Particular exendin-4 analogs include those having a cysteine substitution (*e*.*g*., [Cys³²]exendin-4); a lysine substitution (*e*.*g*., [Lys³⁹]exendin-4); a leucine substitution (*e*.*g*., [Leu¹⁴,Phe²⁵]exendin-4 amide, [Leu¹⁴,Phe²⁵]exendin-4(1-28) amide, and [Leu¹⁴,Ala²²,Phe²⁵]exendin-4(1-28) amide); or exendin fragments (*e*.*g*., exendin-4(1-30), exendin-4(1-30) amide, exendin-4(1-28) amide, and exendin-4(1-31)). Other exendin analogs are described in U.S. Patents Nos. 7,157,555, 7,220,721, and 7,223,725; and U.S. Patent Application Publication No. 2007/0037747.

### Leptin and leptin analogs

Leptin is an adipokine, and thus the therapeutic peptidic agent can include an adipokine or an analog thereof. Adipokines include adiponectin, leptin, and resistin. Adiponectins include human, mouse, and rat adiponectin. Leptins include leptin(116-130), leptin(22-56), leptin(57-92), leptin(93-105), LY396623, metreleptin, murine leptin analog, pegylated leptin, and methionyl human leptin. Resistins include human, mouse, and rat resistin. The leptin may be a cleaved sequence (*e*.*g*., amino acids 22-167 of the human sequence) or the full length protein. The polypeptide used in the invention may be any of these peptides or proteins or may be substantially identical to any of these peptides or proteins.

The leptin analog may be an OB receptor agonist. In certain embodiments, the OB receptor agonist is an agonist for the OB-Rb form, which is the predominant receptor found in the hypothalamus or the OB-R, which is found at the blood-brain barrier and is involved in leptin transport.

### Immunoglobulins and fragments thereof

The therapeutic peptidic agent can be an immnoglobulin (also referred to as an "antibody" and understood in the art to encompass proteins consisting of one or more polypeptides substantially encoded by immunoglobulin genes). The recognized human immunoglobulin genes include the kappa, lambda, alpha (IgA1 and IgA2), gamma (IgG1, IgG2, IgG3, IgG4), delta, epsilon, and mu constant region genes, as well as the myriad immunoglobulin variable region genes, and immunoglobulins encoded by such genes are useful within the present compositions. Full-length immunoglobulin "light chains" (about 25 kDa and 214 amino acids) are encoded by a variable region gene at the amino-terminus (about 110 amino acids) and a kappa or lambda constant region gene at the carboxy-terminus. Full-length immunoglobulin heavy chains (about 50 kDa and 446 amino acids), are similarly encoded by a variable region gene (about 116 amino acids) and one of the other aforementioned constant region genes, *e*.*g*., gamma (encoding about 330 amino acids). The antibodies or immunoglobulins usefully incorporated in the present compositions may include CDRs from a human or non-human source. The framework of the immunoglobulin can be human, humanized, or non-human, *e*.*g*., a murine framework modified to decrease antigenicity in humans, or a synthetic framework, *e*.*g*., a consensus sequence.

As noted, fragments of an immunoglobulin that specifically bind a biological molecule can also be included in the present compositions and we may refer to these fragments as "antigen-binding portions" of an antibody, as they specifically or selectively bind the same biological molecule bound by the complete immunoglobulin from which they were derived. Examples of binding portions encompassed within the term "fragment" include (i) an Fab fragment, a monovalent fragment consisting of the VLC, VHC, CL and CH1 domains; (ii) a F(ab').sub.2 fragment, a bivalent fragment comprising two Fab fragments linked by a disulfide bridge at the hinge region; (iii) a Fd fragment consisting of the VHC and CH1 domains; (iv) a Fv fragment consisting of the VLC and VHC domains of a single arm of an antibody, (v) a dAb fragment (Ward et al., Nature 341:544-546 (1989)), which consists of a VHC domain; and (vi) an isolated complementarity determining region (CDR) having sufficient framework to specifically bind, *e*.*g*., an antigen binding portion of a variable region. A fragment or antigen-binding portion of a light chain variable region and an antigen binding portion of a heavy chain variable region, *e*.*g*., the two domains of the Fv fragment, VLC and VHC, can be joined, using recombinant methods, by a synthetic linker that enables them to be made as a single protein chain in which the VLC and VHC regions pair to form monovalent molecules (known as single chain Fv (scFv); encompassed by the term "immunoglobulin" as used herein; *see e*.*g*., Bird et al. Science 242:423-426 (1988); and Huston et al. Proc. Natl. Acad. Sci. USA 85:5879-5883.(1988)). Immunoglobulins and fragments thereof can be obtained using conventional techniques known to one of ordinary skill in the art, and the fragments can be screened for utility in the same manner as are intact antibodies. An Fab fragment can result from cleavage of a tetrameric antibody with papain; Fab' and F(ab')2 fragments can be generated by cleavage with pepsin.

Also useful in the present compositions are human immunoglobulins or antibodies, which includes polypeptides in which the framework residues correspond to human germline sequences and the CDRs result from V(D)J recombination and somatic mutations. However, human antibodies may also comprise amino acid residues not encoded in human germline immunoglobulin nucleic acid sequences (*e*.*g*., mutations introduced by random or site-specific mutagenesis *in vitro*). It has been demonstrated that *in vivo* somatic mutation of human variable genes results in mutation of framework residues (see Nat. Immunol. 2:537, (2001)). Such an antibody would be termed "human" given its source, despite the framework mutations. Mouse antibody variable domains also contain somatic mutations in framework residues (see Sem. Immunol. 8:159 (1996)). The immunoglobulins can also be polyclonal or monoclonal, and may be mono-, bi-, or tri-specific. The immunoglobulins can be affinity matured, and any of the incorporated immunoglobulins may have been isolated (*e*.*g*., purified to some degree from an animal or cells in which they are produced). Single chain antibodies, and chimeric, humanized or CDR-grafted antibodies, as well as chimeric or CDR-grafted single chain antibodies, comprising portions derived from different species, are also encompassed by the present invention and the term "immunoglobulin."

Immunoglobulins incorporated in the present compositions can include a label (*e*.*g*., a polypeptide that serves as a marker or reporter sequence or that facilitates purification of the antibody sequence to which it is attached). Suitable labels include a FLAG tag, a histidine tag, or an enzymatically active or fluorescent protein. Alternatively, or in addition, the antibodies can include a toxin.

### Transport vectors

The conjugate can include any useful transport vector to bind or contain any therapeutic agent (*e*.*g*., as described herein). The transport vectors may include any lipid, carbohydrate, or polymer-based composition capable of transporting an agent (*e*.*g*., a therapeutic agent as described herein). Transport vectors include lipid vectors (*e*.*g*., liposomes, micelles, and polyplexes) and polymer-based vectors such as dendrimers. Other transport vectors include nanoparticles, which can include silica, lipid, carbohydrate, or other pharmaceutically-acceptable polymers. Transport vectors can protect against degradation of an agent (*e*.*g*., any described herein), thereby increasing the pharmacological half-life and bio-availability of these compounds.

Lipid vectors can be formed using any biocompatible lipid or combination of lipids capable for forming lipid vectors (*e*.*g*., liposomes, micelles, and lipoplexes). Encapsulation of an agent into a lipid vector can protect the agent from damage or degradation or facilitate its entry into a cell. Lipid vectors, as a result of charge interactions (*e*.*g*., a cationic lipid vector and anionic cell membrane), interact and fuse with the cell membrane, thus releasing the agent into the cytoplasm. A liposome is a bilayered vesicle comprising one or more of lipid molecules, polypeptide-lipid conjugates, and lipid components. A lipoplex is a liposome formed with cationic lipid molecules to impart an overall positive charge to the liposome. A micelle is vesicle with a single layer of surfactants or lipid molecules.

### Liposomes

In certain embodiments, the lipid vector is a liposome. Typically, the lipids used are capable of forming a bilayer and are cationic. Classes of suitable lipid molecules include phospholipids (*e*.*g*., phosphotidylcholine), fatty acids, glycolipids, ceramides, glycerides, and cholesterols, or any combination thereof. Alternatively or in addition, the lipid vector can include neutral lipids (*e*.*g*., dioleoylphosphatidyl ethanolamine (DOPE)). Other lipids that can form lipid vectors are known in the art and described herein.

As used herein, a "lipid molecule" is a molecule with a hydrophobic head moiety and a hydrophilic tail moiety and may be capable of forming liposomes. The lipid molecule can optionally be modified to include hydrophilic polymer groups. Examples of such lipid molecules include 1,2-distearoyl-*sn*-glycero-3-phosphoethanolamine-N-[methoxy(polyethylene glycol)-2000] and 1,2-distearoyl-*sn*-glycero-3-phosphoethanolamine-N-[carboxy(polyethylene glycol)-2000].

Examples of lipid molecules include natural lipids, such as cardiolipin (CL), phosphatidic acid (PA), phosphatidylcholine (PC), phosphatidylethanolamine (PE), phosphatidylglycerol (PG), phosphatidylinositol (PI), and phosphatidyl serine (PS); sphingolipids, such as sphingosine, ceramide, sphingomyelin, cerebrosides, sulfatides, gangliosides, and phytosphingosine; cationic lipids, such as 1,2-dioleoyl-3-trimethylammonium-propane (DOTAP), 1,2-dioleoyl-3-dimethylammonium-propane (DODAP), dimethyldioctadecyl ammonium bromide (DDAB), 3-β-[N-(N',N'-dimethylaminoethane)carbamoly]cholesterol (DC-Chol), N-[1-(2,3,-ditetradecyloxy)propyl]-N,N-dimethyl-N-hydroxyethylammonium bromide (DMRIE), N-[1-(2,3,-dioleyloxy)propyl]-N,N-dimethyl-N-hydroxy ethylammonium bromide (DORIE), and 1,2-di-O-octadecenyl-3-trimethylammonium propane (DOTMA); phosphatidylcholines, such as 1,2-dilauroyl-*sn-*glycero-3-ethylphosphocholine, 1,2-dilauroyl-sn-glycero-3-phosphocholine (DLPC), 1,2-dimyristoyl-sn-glycero-3-phosphocholine (DMPC), 1,2-dipalmitoyl-*sn*-glycero-3-phosphocholine (DPPC), 1,2-distearoyl-*sn*-glycero-3-phosphocholine (DSPC), 1,2-dioleoyl-*sn*-glycero-3-phosphocholine (DOPC), and 1-palmitoyl-2-oleoyl-*sn*-glycerol-3-phosphocholine (POPC); phosphoethanolamines, such as 1,2-dibutyryl-*sn*-glycero-3-phosphoethanolamine, 1,2-distearoyl-*sn*-glycero-3-phosphoethanolamine (DSPE), 1,2-dimyristoyl-*sn*-glycero-3-phosphoethanolamine (DMPE), 1,2-dipalmitoyl-*sn*-glycero-3-phosphoethanolamine (DPPE), 1,2-dioleoyl-*sn*-glycero-3-phosphoethanolamine (DOPE), and 1,2-diolcoyl-*sn*-glycero-3-phosphoethanolamine-N-(glutaryl); phosphatidic acids, such as 1,2-dimyristoyl-*sn*-glycero-3-phosphate, 1,2-dipalmitoyl-*sn*-glycero-3-phosphate, and 1,2-dioleoyl-*sn*-glycero-3-phosphate; phosphatidylglycerols, such as dipalmitoyl phosphatidylglycerol (DMPC), 1,2-dimyristoyl-*sn*-glycero-3-phospho-(1'-rac-glycerol), and 1,2-dioleoyl-*sn*-glycero-3-phospho-(1'-rac-glycerol); phosphatidylserines, such as 1,2-dimyristoyl-*sn*-glycero-3-phospho-L-serine, 1,2-dipalmitoyl-*sn*-glycero-3-phospho-L-serine, and 1,2-dioleoyl-*sn*-glycero-3-phospho-L-serine; cardiolipins, such as 1',3'-bis[1,2-dimyristoyl-*sn*-glycero-3-phospho]-*sn*-glycerol; and PEG-lipid conjugates, such as 1,2-dipalmitoyl-*sn*-glycero-3-phosphoethanolamine-N-[methoxy(polyethylene glycol)-750], 1,2-dipalmitoyl-*sn*-glycero-3-phosphoethanolamine-N-[methoxy(polyethylene glycol)-2000], 1,2-dipalmitoyl-*sn*-glycero-3-phosphoethanolamine-N-[methoxy(polyethylene glycol)-5000], 1,2-distearoyl-*sn*-glycero-3-phosphoethanolamine-N-[methoxy(polyethylene glycol)-2000], and 1,2-distearoyl-*sn*-glycero-3-phosphoethanolamine-N-[carboxy(polyethylene glycol)-2000].

Commercially available lipid compositions include Lipofectamine™ 2000 and Lipofectin® from Invitrogen Corp.; Transfectam® and Transfast™ from Promega Corp.; NeuroPORTER™ and Escort™ from Sigma-Aldrich Co.; FuGENE® 6 from Roche; and LipoTAXI® from Strategene. Known lipid compositions include the Trojan Horse Lipsome technology, as described in Boado, Pharm. Res. 24:1772-1787 (2007).

The liposomes can also include other components that aid in the formation or stability of liposomes. Examples of components include cholesterol, antioxidants (*e*.*g*., α-tocopherol, β-hydroxytoluidine), surfactants, and salts.

A lipid molecule can be bound to a targeting moiety by a covalent bond or a non-covalent bond (*e*.*g*., ionic interaction, entrapment or physical encapsulation, hydrogen bonding, absorption, adsorption, van der Waals forces, or any combinations thereof) with or without the use of a linker.

The liposome can be of any useful combination comprising lipid molecules, including polypeptide-lipid conjugates and other components that aid in the formation or stability of liposomes. A person of skill in that art will know how to optimize the combination that favor encapsulation of a particular agent, stability of the liposome, scaled-up reaction conditions, or any other pertinent factor. Exemplary combinations are described in Boado, Pharm. Res. 24:1772-1787 (2007). In one example, the liposome comprises 93% POPC, 3% DDAB, 3% distearoylphosphatidylethanolamine (DSPE)-PEG2000, and 1% DSPE-PEG2000 covalently linked to a targeting moiety.

Producing liposomes typically occur through a general two-step process. In the first step, the lipids and lipid components are mixed in a volatile organic solvent or mixtures of solvents to ensure a homogenous mixture of lipids. Examples of solvents include chloroform, methanol, cyclohexane, and t-butanol. The solvent is then removed to form a dry lipid mixture in a film, powder, or pellet. The solvent can also be removed by using any known analytical techniques, such as by using nitrogen, rotary evaporation, spray drying, lyophilization, and vacuum-drying.

In the second step, the dry lipid mixture is hydrated with an aqueous solution to form liposomes. The agent can be added to the aqueous solution, which results in the formation of liposomes with encapsulated agent. Alternatively, the liposomes are first formed with a first aqueous solution and then exposed to another aqueous solution containing the agent. Encapsulation of the agent can be promoted by any known technique, such as by repeat freeze-thaw cycles, sonication, or mixing. A further example of this approach is described in Boado, Pharm. Res. 24:1772-1787 (2007). Alternatively, the agent is coupled to a hydrophobic moiety (*e*.*g*., cholesterol) to produce a lipophilic derivative and the lipophilic derivative is used with other lipid molecules to from liposomes.

During the second step, the dry lipid mixture may or may not contain the polypeptide-lipid conjugate. The process can optionally include various additional steps, including heating the aqueous solution past the phase transition temperature of the lipid molecules before adding it to the dry lipid mixture, where particular ranges of temperatures include from about 40°C to about 70°C; incubating the combination of the dry lipid mixture and the aqueous solution, where particular time ranges include from about 30 minutes to about 2 hours; mixing of the dry lipid mixture and the aqueous solution during incubation, such as by vortex mixing, shaking, stirring, or agitation; addition of nonelectrolytes to the aqueous solution to ensure physiological osmolality, such as a solution of 0.9% saline, 5% dextrose, and 10% sucrose; disruption of large multilamellar vesicles, such as by extrusion or sonication; and additional incubation of the pre-formed liposomes with polypeptide-lipid conjugate, where the dry lipid mixture did not contain the lipid molecules. One of skill in the art will be able to identify the particular temperature and incubation times during this hydration step to ensure incorporation of the derivatized lipid molecule into the liposomes or to obtain stable liposomes.

The polypeptide-lipid conjugate can be added at any point in the process of forming liposomes. In one example, the polypeptide-lipid conjugate is added to the lipids and lipid components during the formation of the dry lipid mixture. In another example, the polypeptide-lipid conjugate is added to liposomes that are pre-formed with a dry lipid mixture containing the lipids and lipid components. In yet another example, micelles are formed with the polypeptide-lipid conjugate, liposomes are formed with a dry lipid mixture containing lipids and lipid components, and then the micelles and liposomes are incubated together. The aqueous solution can include additional components to stabilize the agent or the liposome, such as buffers, salts, chelating agents, saline, dextrose, sucrose, *etc.*

In one example of this procedure, a dry film composed of the lipid mixture is hydrated with an aqueous solution containing an agent. This mixture is first heated to 50°C for 30 minutes and then cooled to room temperature. Next, the mixture is transferred onto a dry film containing the polypeptide-lipid conjugate. The mixture is then incubated at 37°C for two hours to incorporate the polypeptide-lipid conjugate into the liposomes containing the agent. See, *e.g*., Zhang et al., J. Control. Release 112:229-239 (2006).

### Polyplexes

Complexes of polymers with agents are called polyplexes. Polyplexes typically consist of cationic polymers and their production is regulated by ionic interactions with an anionic agent (*e*.*g*., a polynucleotide). In some cases, polyplexes cannot release the bound agent into the cytoplasm. To this end, co-transfection with endosome-lytic agents (to lyse the endosome that is made during endocytosis) such as inactivated adenovirus must occur. In certain cases, polymers, such as polyethylenimine, have their own method of endosome disruption, as does chitosan and trimethylchitosan. Polyplexes are described, for example, in U.S. Patent Application Publication Nos. 2002/0009491; 2003/0134420; and 2004/0176282.

Polyplexes can be formed with any polymer and copolymer described herein, where non-charged or anionic polymers can be further derivatized to include cationic side chains. Examples of cationic side chains are amines, which are typically protonated under physiological conditions. Exemplary polymers that can be used to form polyplexes include polyamines, such as polylysine, polyarginine, polyamidoamine, and polyethylene imine.

### Dendrimers

A dendrimer is a highly branched macromolecule with a spherical shape. The surface of the particle may be functionalized in many ways and many of the properties of the resulting construct are determined by its surface. In particular, it is possible to construct a cationic dendrimer (*i.e.*, one with a positive surface charge). When in the presence of genetic material such as DNA or RNA, charge complimentarity leads to a temporary association of the polynucleotide with the cationic dendrimer. On reaching its destination the dendrimer-polynucleotide complex is then taken into the cell via endocytosis or across the BBB by transcytosis. Dendrimers are described, for example, in U.S. Patent Nos. 6,113,946 and 7,261,875.

Dendrimers can be produced by any process known in the art. Under the divergent method, the core of the dendrimer is built first and successive steps build outward from the core to form branched structures. Under the convergent method, wedges of the dendrimer (or dendrons) are built separately, where successive steps build inward from the molecules that will make up the outer surface of the dendrimer. The different dendrons can be formed with the same or different polymeric monomers. Then, the dendrons are covalent linked to a core molecule or structure to form the dendrimer. Further examples of these methods are described in Svenson et al., Adv. Drug. Deliv. Rev. 57:2106-2129 (2005).

For polyamidoamine (PAMAM) dendrimers, the core of the dendrimer typically comprises an amino group. Exemplary core molecules include ammonia; diamine molecules, such as ethylenediamine, 1,4-diaminobutane, 1,6-diaminohexane, 1,12-diaminododecane, and cystamine; and triamine molecules, such as triethanolamine. In the first step of the addition reaction, polymeric monomers are used to build upon the core by reacting the monomers with the amino groups of the core to form a tetra-branched molecule. Subsequent addition reactions with the diamine molecule and the polymeric monomer further build upon the dendrimer.

Examples of polymeric monomers that react with amino groups include methacrylate to form PAMAM dendrimers; and acrylonitrile to form poly(propylene imine) dendrimers. Examples of PAMAM dendrimers and synthetic reactions of dendrimers are set forth in U.S. Pat. Nos. 4,507,466, 5,527,524, and 5,714,166. Examples of PAMAM dendrimers formed with a triethanolamine core are set forth in Wu et al., Chem. Comm. 3:313-315 (2005); and Zhou et al., Chem. Comm. 22:2362-2364 (2006). Synthesis of the dendrimers can include additional steps, such as adding protecting groups to activated groups in order to prevent intramolecular reactions; and adding a deprotection step to remove protecting groups.

In addition to PAMAM dendrimers, other types of dendrimers can be used. For phosphorous dendrimers, the core of the dendrimer comprises a P=O group. Exemplary core molecules include a cyclotriphosphazene group and a thiophosphoryl group. Examples of polymeric monomers include phenoxymethyl(methylhydrazono) groups. Alternatively, the dendrimer is a hyperbranched polymer with a polyester core structure. Examples of such dendrimers include hyperbranched 2,2-bis(hydroxymethyl)propionic acid polyester-16-hydroxyl.

The outer surface groups of the dendrimer can have a variety of functional groups, including amidoethanol, amidoethylethanolamine, amino, hexylamide, carboxylate, succinamidyl, trimethoxysilyl, tris(hydroxymethyl)amidomethane, and 3-carbomethoxypyrrolidinone groups. In addition, these functional groups can be further treated with a coupling agent to form activated groups (as defined herein).

In one particular example, the polyamidoamine dendrimer is conjugated to a polyvalent linker containing a hydrophilic polymer group: α-malemidyl-ω-N-hydroxysuccinimidyl polyethyleneglycol (MW 3400). The amino group on the surface of the polyamidoamine dendrimer is reacted with the terminal N-hydroxysuccinimidyl activated group of the linker. The derivatized dendrimer is then purified, filtered, and dissolved in saline. Next, the terminal malemidyl group of the derivatized dendrimer is reacted with a sulfhydryl group of the targeting moiety. If the polypeptide does not contain a sulfhydryl group, then the amino group present in the polypeptide can be reacted with N-succinimidyl-S-acetylthioacetate or N-succinimidyl-S-acetylthiopropionate to introduce a protected sulfhydryl group. Alternatively, the polypeptide can be synthesized to include an additional cysteine group. The agent is associated with the derivatized dendrimer by incubating the agent and the derivatized dendrimer in a solvent and vortexing the mixture. Further examples of these approaches are described in Ke et al., J. Pharm. Sci. 97:2208-2216 (2008); Huang et al., J. Gene Med. 11:754-763 (2009); Huang et al., Biomaterials 29:238-246 (2008); and Liu et al. Biomaterials 30:4195-4202.

In another particular example, the polyamidoamine dendrimer is conjugated to a polyvalent linker containing an aliphatic group: 4-sulfosuccinimidyl-6-methyl-α-(2-pyridyldithio)toluamido]hexanoate. The amino group on the surface of the polyamidoamine dendrimer is reacted with the terminal sulfosuccinimidyl activated group of the linker. The derivatized dendrimer is then purified and dissolved in saline. Next, the terminal pyridyldithio group of the derivatized dendrimer is reacted with a sulfhydryl group of the polypeptide. The agent is associated with the derivatized dendrimer by incubating the agent and the derivatized dendrimer in a solvent and vortexing the mixture. Further examples of these approaches are described in Kang et al., Pharm. Res. 22:2099-2106 (2005).

Agents can be associated with the derivatized dendrimer by any number of methods, such as by covalent and non-covalent associations (*e*.*g*., ionic interaction, entrapment or physical encapsulation, hydrogen bonding, absorption, adsorption, van der Waals forces, or any combinations thereof).

### Nanoparticles

Nanoparticles may be used as a transport vector in the invention. As used herein, a "nanoparticle" is a colloidal, polymeric, or elemental particle ranging in size from about 1 nm to about 1000 nm. Nanoparticles can be made up of silica, carbohydrate, lipid, or polymer molecules. Molecules can be either embedded in the nanoparticle matrix or may be adsorbed onto its surface. In one example, the nanoparticle may be made up of a biodegradable polymer such as poly(butylcyanoacrylate) (PBCA). Examples of elemental nanoparticles include carbon nanoparticles and iron oxide nanoparticles, which can then be coated with oleic acid (OA)-Pluronic. In this approach, a drug (*e*.*g*., a hydrophobic or water insoluble drug) is loaded into the nanoparticle, as described in Jain et al., Mol. Pharm. 2:194-205 (2005). Other nanoparticles are made of silica, and include those described, for example, in Bums et al., Nano Lett, 9:442-448 (2009).

Nanoparticles can be formed from any useful polymer. Examples of polymers include biodegradable polymers, such as poly(butyl cyanoacrylate), poly(lactide), poly(glycolide), poly-ε-caprolactone, poly(butylene succinate), poly(ethylene succinate), and poly(p-dioxanone); poly(ethyleneglycol); poly-2-hydroxyethylmethacrylate (poly(HEMA)); copolymers, such as poly(lactide-co-glycolide), poly(lactide)-poly(ethyleneglycol), poly(poly(ethyleneglycol)cyanoacrylate-co-hexadecylcyanoacrylate, and poly[HEMA-co-methacrylic acid]; proteins, such as fibrinogen, collagen, gelatin, and elastin; and polysaccharides, such as amylopectin, α-amylose, and chitosan.

Polymeric nanoparticles can be produced by any useful process. Using the solvent evaporation method, the polymer and agent is dissolved in a solvent to form a nanoemulsion and the solvent is evaporated. Appropriate solvent systems and surfactants can be used to obtain either oil-in-water or water-in-oil nanoemulsions. This method can optionally include filtration, centrifugation, sonication, or lyophilization. Using the nanoprecipitation method, a solution of the polymer and an agent is formed in a first solvent. Then, the solution is added to a second solvent that is miscible with the first solvent but does not solubilize the polymer. During phase separation, nanoparticles are formed spontaneously. Using the emulsion polymerization method, the monomer is dispersed into an aqueous solution to form micelles. Initiator radicals (*e*.*g*., hydroxyl ions) in the aqueous solution initiate anionic polymerization of the monomers. In another variation of the emulsion polymerization method, the agent acts as the initiator radical that promotes anionic polymerization. For example, an agent that is a photosensitizer can initiate polymerization of cyanoacrylate monomers. Additional methods include dialysis, ionic gelation, interfacial polymerization, and solvent casting with porogens.

In an example of the solvent evaporation method, the polymer is a cyanoacrylate copolymer containing a hydrophilic polymer group: poly(aminopoly(ethyleneglycol) cyanoacrylate-co-hexadecyl cyanoacrylate), which was synthesized as described in Stella et al., J. Pharm. Sci. 89:1452-1464 (2000). The polymer and agent are added to an organic solvent, where the mixture is emulsified by adding an aqueous solution. Then, the organic solvent was evaporated under reduced pressure and the resultant nanoparticles were washed and lyophilized. In the particular example of the agent being transferrin, the terminal hydroxyl group on the carbohydrate moiety of transferrin is treated with sodium periodate to form an aldehyde group and oxidized transferrin is added to the nanoparticles. Further examples of this approach are described in Li et al., Int. J. Pharm. 259:93-101 (2003); and Yu et al., Int. J. Pharm. 288:361-368 (2005).

In an example of the emulsion polymerization method, the monomer is added drowise to an acidic aqueous solution. The mixture is stirred to promote polymerization and then neutralized. The nanoparticles are then filtered, centrifuged, sonicated, and washed. In one particular example of this method, the monomer of butyl cyanoacrylate monomer is provided and the aqueous solution also includes dextran in a dilute aqueous solution of hydrochloric acid. To introduce the agent, the poly(butyl cyanoacrylate) nanoparticles are lyophilized and then resuspended in saline. Agents are added to the saline solution with the nanoparticles under constant stirring. Alternatively, the agent is added to during the polymerization process. The nanoparticles are optionally coated with a surfactant, such as polysorbate 80. Further examples of this approach are described in Kreuter et al., Brain Res. 674:171-174 (1995); Kreuter et al., Pharm. Res. 20:409-416 (2003); and Steiniger et al., Int. J. Cancer 109:759-767 (2004).

Other nanoparticles include solid lipid nanoparticles (SLN). SLN approaches are described, for example, in Kreuter, Ch. 24, In V. P. Torchilin (ed), Nanoparticles as Drug Carriers pp. 527-548, Imperial College Press, 2006). Examples of lipid molecules for solid lipid nanoparticles include stearic acid and modified stearic acid, such as stearic acid-PEG 2000; soybean lechitin; and emulsifying wax. Solid lipid nanoparticles can optionally include other components, including surfactants, such as Epicuron® 200, poloxamer 188 (Pluronic® F68), Brij 72, Brij 78, polysorbate 80 (Tween 80); and salts, such as taurocholate sodium. Agents can be introduced into solid lipid nanoparticles by a number of methods discussed for liposomes, where such methods can further include high-pressure homogenization, and dispersion of microemulsions.

In one example, SLNs include stearic acid, Epicuron 2000 (surfactant), and taurocholate sodium loaded with an agent (*e*.*g*., an anticancer agent such as doxorubicin, tobramycin, idarubicin, or paclitaxel, or a paclitaxel derivative). In another example, SLNs include stearic acid, soybean lecithin, and poloxamer 188. SLNs can also be made from polyoxyl 2-stearyl ether (Brij 72), or a mixture of emulsifying wax and polyoxyl 20-stearyl ether (Brij 78) (see, *e*.*g*., Koziara et al., Pharm. Res. 20:1772-1778,2003). In one example of making solid lipid nanoparticles, a microemulsion was formed by adding a surfactant (*e*.*g*. Brij 78 or Tween 80) to a mixture of emulsifying wax in water at 50°C to 55°C. Emulsifying wax is a waxy solid that is prepared from cetostearyl alcohol and contains a polyoxyethylene derivative of a fatty acid ester of sorbitan. Nanoparticles are formed by cooling the mixture while stirring. The agent can be introduced by adding the agent to the heated mixture containing the emulsifying wax in water. Further examples of this approach are described in Koziara et al., Pharm. Res. 20: 1772-1778 (2003).

Nanoparticles can also include nanometer-sized micelles. Micelles can be formed from any polymers described herein. Exemplary polymers for forming micelles include block copolymers, such as poly(ethylene glycol) and poly(ε-caprolactone). In one particular example, PEO-b-PCL block copolymer is synthesized via controlled ring-opening polymerization of ε-caprolactone by using an α-methoxy-ω-hydroxy-poly(ethylene glycol) as a macroinitiator. To form micelles, the PEO-b-PCL block copolymers were dissolved in an organic solvent (*e*.*g*., tetrahydrofuran) and then deionized water was added to form a micellar solution. The organic solvent was evaporated to obtain nanometer-sized micelles.

In certain embodiments, the properties of the nanoparticle are altered by coating with a surfactant. Any biocompatible surfactant may be used, for example, polysorbate surfactants, such as polysorbate 20, 40, 60, and 80 (Tween 80); Epicuron® 200; poloxamer surfactants, such as 188 (Pluronic® F68) poloxamer 908 and 1508; and Brij surfactants, such as Brij 72 and Brij 78. In other embodiments, the surfactant is covalently attached to the nanoparticle, as is described in PCT Publication No. WO 2008/085556. Such an approach may reduce toxicity by preventing the surfactant from leeching out of the nanoparticle. Nanoparticles can be optionally coated with a surfactant.

Nanoparticles can optionally be modified to include hydrophilic polymer groups (*e.g.*, poly(ethyleneglycol) or poly(propyleneglycol)). The surface of the nanoparticle can be modified by covalently attaching hydrophilic polymer groups. Alternatively, nanoparticles can be formed by using polymers that contain hydrophilic polymer groups, such as poly[methoxy poly (ethyleneglycol) cyanoacrylate-co-hexadecyl cyanoacrylate]. Nanoparticles can be optionally cross-linked, which can be particularly use for protein-based nanoparticles.

Therapeutic agents can be introduced to nanoparticles by any useful method. Agents can be incorporated into the nanoparticle at, during, or after the formation of the nanoparticle. In one example, the agent is added to the solvent with the polymer or monomer before the formation of the nanoparticles. In another example, the agent is incorporated into pre-formed nanoparticles by adsorption. In yet another example, the agent is covalently bound to the nanoparticle. The agent can be physically adsorbed to the surface of the nanoparticle with the optional step of further coating the nanoparticle with a surfactant. Examples of surfactants include polysorbate 80 (Tween 80). Further examples of this approach are described in Kreuter, Nanoparticular Carriers for Drug Delivery to the Brain, Chapter 24, in Torchilin (ed.), Nanoparticulates as Drug Carriers (2006), Imperial College Press.

### Carbohydrate-based delivery methods

Carbohydrate-based polymers such as chitosan can be used as a transport vector *e*.*g*., in the formation of micelles or nanoparticles. As chitosan polymers can be amphiphilic, these polymers are especially useful in the delivery of hydrophobic agents (*e.g*., those described herein). Exemplary chitosan polymers include quaternary ammonium palmitoyl glycol chitosan, which can be synthesized as described in Qu et al., Biomacromolecules 7:3452-3459, 2006.

### Hybrid methods

Some hybrid methods combine two or more techniques and can be useful for administering the conjugates of the invention to a cell, tissue, or organ of a subject. Virosomes, for example, combine liposomes with an inactivated virus. This combination has more efficient gene transfer in respiratory epithelial cells than either viral or liposomal methods alone. Other methods involve mixing other viral vectors with cationic lipids or hybridizing viruses.

### Multimeric targeting moieties, conjugates, and therapeutic polypeptides

Disclosed are multimeric (*e.g*., dimeric or trimeric) forms of the targeting moieties, conjugates, and therapeutic polypeptides described herein. The targeting moieties are joined by a chemical bond either directly (*e.g.*, a covalent bond such as a disulfide or a peptide bond) or indirectly (*e*.*g*., through a linker such as those described herein). Exemplary multimeric targeting moieties, conjugates, and therapeutic polypeptides are described below. Any linker described herein can be used for multimeric targeting moieties, conjugates, and therapeutic polypeptides (*e*.*g*., polyvalent linkers).

### Multimeric targeting moieties

Disclosed is that the multimeric targeting moiety is a dimer having the formula:

A¹-X-A²,

where A¹ and A² are each, independently, a targeting moiety (*e*.*g*., any targeting moiety described herein) and X is a linker, The linker may be any linker described herein. In particular embodiments, the linker contains a maleimido moiety and binds to a cysteine present in the peptide vector (*e*.*g*., a peptide vector to which an N-terminal or C-terminal cysteine residue has been added).

It is disclosed that the multimeric targeting moiety has or includes a formula selected from the group consisting of: 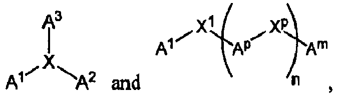 where A¹, A², A³, A^{m}, and each A^{p} are, independently, a targeting moiety (*e*.*g*., any targeting moiety described herein); X, X¹, and each X^{p} are, independently, a linker (e.g., any linker described herein) that joins together two targeting moieties; n is 0, 1, 2, 3, 4, 5. 6, 7, 8, 9, or 10; m is n + 2; and p is an integer from 2 to n + 1. In particular embodiments, n is 1, and the compound has the formula:

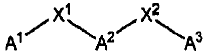

Higher order multimeric targeting moieties can also be described by the formula: 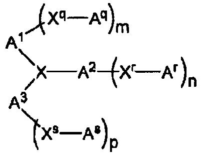 where A¹, A², each A^{q}, each A^{r}, and each A^{s} are, independently, targeting moieties (e.g., any of those described herein); A³ is a targeting moiety or is absent; X, each X^{q}, each X^{r}, and each X^{s} are, independently, linkers that join targeting moieties; m, n, and p are each, independently, 0, 1, 2, 3, 4, 5, 6, 7, 8, 9, or 10; q is an integer from 4 to m + 3; r is an integer from m + 4 to m + n + 3; and s is an integer from m + n + 4 to m + n + p + 3.

It is disclosed that the multimeric targeting moieties include any of modifications or further conjugations described herein for polypeptides (*e*.*g*., posttranslational processing or by chemical modification, including ubiquitination, pegylation, acetylation, acylation, cyclization, amidation, oxidation, sulfation, formation of cysteine, or covalent attachment of one or more therapeutic agents).

### Multimeric conjugates

The therapeutic agent or transport vector can be joined to one or more multimeric targeting moieties joined (*e*.*g*., by a covalent bond) to form a multimeric conjugate or a therapeutic polypeptide.

Compounds including a therapeutic agent and dimeric targeting moiety can be conjugated either through the targeting moiety portion of the molecule or through the linker portion of the molecule. Compounds disclosed in which the agent is joined (*e*.*g*., through a linker where the linker is a chemical linker, peptide, or a covalent bond, such as a peptide bond) to the targeting moiety can be represented by the formula: 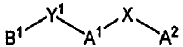 where A¹ and A² are each, independently, targeting moieties (*e.g.*, any described herein); X is a linker (*e.g*., chemical linker, peptide, or covalent bond) that joins A¹ and A²; B¹ is a therapeutic agent or transport vector; and Y¹ is a linker that joins B¹ and A¹.

Disclosed is that two or more (*e*.*g*., 3, 4, 5, 6, 7, 8, 9, or 10) therapeutic agents or transport vectors are joined to one or both of the targeting moieties. Such compounds can be represented by the formula: 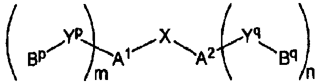 where A¹, A², and X are as defined above; m is 1, 2, 3, 4, 5, 6, 7, 8, 9, or 10; n is 0, 1, 2, 3, 4, 5, 6, 7, 8, 9, or 10; p is an integer from 1 to m; q is an integer from m + 1 to m + n; each B^{p} and each B^{q} are, independently, a therapeutic agent or transport vector (*e*.*g*., any described herein); and each Y^{p} and each Y^{q} are, independently, a linker that joins each B^{p} or each B^{q} to A¹ or A², respectively.

It is disclosed that the therapeutic agent or transport vector is joined (*e*.*g*., through a covalent bond or a chemical linker such as those described herein) to the dimer through the linker that joins the targeting moieties forming the dimer. Such compounds can have the formula: 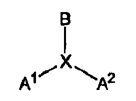 where A¹ and A² are targeting moieties (*e*.*g*., any described herein); B is a therapeutic agent or transport vector; and X is a linker that joins A¹, A², and B.

It is disclosed that the therapeutic agent or transport vector can be joined to both the linker and a targeting moiety. Such compounds can be represented by the formula: 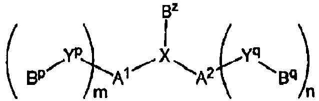 where A¹ and A² are, independently, targeting moieties; B^{z} is an agent or is absent; m is 0, 1, 2, 3, 4, 5, 6, 7, 8, 9, or 10; n is 0, 1, 2, 3, 4, 5, 6, 7, 8, 9, or 10; p is an integer from 1 to m; q is an integer from m + 1 to m + n; Each B^{p} and B^{q} is, independently, a therapeutic agent or transport vector (*e*.*g*., any described herein); and each Y^{p} and Y^{q} is, independently, a linker that joins each **B^{p}** or each B^{q} to A¹ or A², respectively, where at least one (*e*.*g*., at least two) of the following is true (i) B1 is present; (ii) m is at least 1; and (iii) n is at least 1. Disclosed compounds of the invention can also include a trimeric targeting moiety. Where the trimeric targeting moiety is joined to a single agent through one of the targeting moieties, the compound can have one of the following formulas: 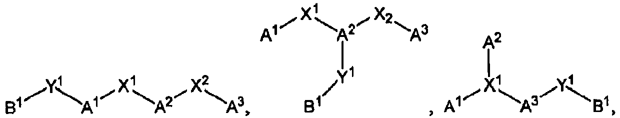 and 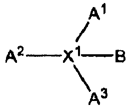 where A¹, A², and A³ are each, independently, a targeting moiety (*e.g*., any described herein); X¹ and X² are linkers; B¹ is a therapeutic agent or transport vector; and Y¹ is a linker that joins B¹ to a targeting moiety (*e.g*., A¹, A², and A³) or to the linker X¹.

Disclosed is that the trimeric targeting moiety is conjugated to one or more than one therapeutic agent or transport vector. Such conjugation can be through either the targeting moiety, or through the linker(s). Such compounds can include one of the following formulas: 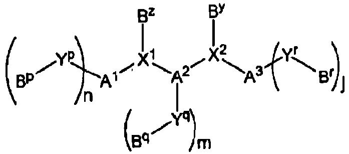 where A¹, A², and A³ are targeting moieties; n, m, and j are 0, 1, 2, 3, 4, 5, 6, 7, 8, 9, or 10; Each B^{p}, each B^{q}, and each B^{r} are, independently, therapeutic agents or transport vectors (*e.g*., any described herein); B^{z} and B^{y} are, independently, therapeutic agents or transport vectors or are absent; X¹ is a linker joining A¹, A², and B^{z}, if present; X² is a linker joining A², A³, and B^{y}, if present. In certain embodiments, at least one of n, m, or j is at least one, B^{z} is present, or B^{y} is present. In other embodiments, at least two (*e*.*g*., at least 3, 4, 5, 6, 7, 8, 9, 10, 15, 20, 25, or 30) of B^{p}, B^{q}, B^{r}, B^{y}, and B^{z} are present.

Disclosed compounds can also include targeting moiety multimers of a higher order (*e*.*g*., quatromers, pentomers, etc.). Such multimers can be described by the formula: 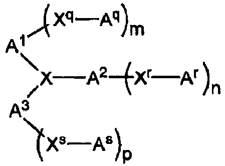 where A¹, A², each A^{q}, each A^{r}, and each A^{s} are, independently, targeting moieties; A³ is a targeting moiety or is absent; X, each X^{q}, X^{r}, and X^{s} are, independently, linkers that join targeting moieties; m, n, and p are each, independently, 0, 1, 2, 3, 4, 5, 6, 7, 8, 9, or 10; q is an integer from 4 to m + 3; r is an integer from m + 4 to m + n + 3; and s is an integer from m + n + 4 to m + n + p + 3. One or more agents can be joined to either the linkers (X, any X^{q}, X^{r}, or X^{s}) or the targeting moieties (A¹, A², A³, each A^{q}, each A^{r}, and each A^{s}) of this formula in order to form higher order multimer conjugates.

### Multimeric therapeutic polypeptides

Multimeric therapeutic polypeptides are also disclosed. It is disclosed that the multimeric therapeutic polypeptide is in the form of a fusion protein. The fusion protein may contain 2, 3, 4, 5, or more targeting moieties, either joined directly by a peptide bond, or through peptide linkers. In one example, fusion protein dimers are described by the formula:

A¹-X-A²

where A¹ and A² are, independently, a targeting moiety (*e.g*., any described herein) and X is either (a) a peptide bond that joins A¹ and A² or (b) one or more amino acids joined to A¹ and A² by peptide bonds. In certain embodiments, the peptide linker is a single amino acid (e.g., a naturally occurring amino acid), a flexible linker, a rigid linker, or an alpha-helical linker. Exemplary peptide linkers that can be used are described in the section entitled "Amino acid and peptide linkers" below. In certain embodiments A¹ and A² are the same targeting moiety.

Fusion protein multimers can be described by the formula:

A¹-(Xⁿ-A^{m})ₙ

where n is or is at least 1,2, 3, 4, 5, 6, 7, 8, 9, or 10; m is an integer from 2 to n + 1; A¹ and each A^{m} are, independently, a targeting moiety (*e*.*g*., any described herein); and each Xⁿ is, independently, either (a) a targeting moiety that joins A¹ and A² or (b) one or more amino acids joined to the adjacent targeting moiety (A¹ or Aⁿ) by peptide bonds.

The targeting moieties forming the multimer may each be fewer than 19, 18, 17, 16, 15, 14, 13, 12, 11, 10, 9, 8, 7, or 6 amino acids in length. The fusion protein may be fewer than 1,000, 500, 250, 150, 100, 90, 80, 75, 70, 65, 60, 55, 50, 45, 40, or 35 amino acids in length.

### Modified polypeptides

The targeting moieties, therapeutic peptidic agents, and therapeutic polypeptides used in the invention may have a modified amino acid sequence. In certain embodiments, the modification does not destroy significantly a desired biological activity (*e*.*g*., ability to cross the BBB or agonist activity). The modification may reduce (*e*.*g*., by at least 5%, 10%, 20%, 25%, 35%, 50%, 60%, 70%, 75%, 80%, 90%, or 95%), may have no effect, or may increase (*e*.*g*., by at least 5%, 10%, 25%, 50%, 100%, 200%, 500%, or 1000%) the biological activity of the original polypeptide. The modified peptide or polypeptide may have or may optimize a characteristic of a polypeptide, such as *in vivo* stability, bioavailability, toxicity, immunological activity, immunological identity, and conjugation properties.

Modifications include those by natural processes, such as posttranslational processing, or by chemical modification techniques known in the art. Modifications may occur anywhere in a polypeptide including the polypeptide backbone, the amino acid side chains and the amino- or carboxy-terminus. The same type of modification may be present in the same or varying degrees at several sites in a given polypeptide, and a polypeptide may contain more than one type of modification. Polypeptides may be branched as a result of ubiquitination, and they may be cyclic, with or without branching. Cyclic, branched, and branched cyclic polypeptides may result from posttranslational natural processes or may be made synthetically. Other modifications include pegylation, acetylation, acylation, addition of acetomidomethyl (Acm) group, ADP-ribosylation, alkylation, amidation, biotinylation, carbamoylation, carboxyethylation, esterification, covalent attachment to fiavin, covalent attachment to a heme moiety, covalent attachment of a nucleotide or nucleotide derivative, covalent attachment of drug, covalent attachment of a marker (*e*.*g*., fluorescent or radioactive), covalent attachment of a lipid or lipid derivative, covalent attachment of phosphatidylinositol, cross-linking, cyclization, disulfide bond formation, demethylation, formation of covalent crosslinks, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation and ubiquitination.

A modified polypeptide can also include an amino acid insertion, deletion, or substitution, either conservative or non-conservative (*e*.*g*., D-amino acids, desamino acids) in the polypeptide sequence (*e*.*g*., where such changes do not substantially alter the biological activity of the polypeptide). In particular, the addition of one or more cysteine residues to the amino or carboxy terminus of any of the polypeptides of the invention can facilitate conjugation of these polypeptides by, *e*.*g*., disulfide bonding. For example, P1 has a cysteine residue at the N-terminus. Amino acid substitutions can be conservative (*i.e*., wherein a residue is replaced by another of the same general type or group) or non-conservative (*i*.*e.*, wherein a residue is replaced by an amino acid of another type). In addition, a non-naturally occurring amino acid can be substituted for a naturally occurring amino acid (*i*.*e*., non-naturally occurring conservative amino acid substitution or a non-naturally occurring non-conservative amino acid substitution).

Polypeptides made synthetically can include substitutions of amino acids not naturally encoded by DNA (*e*.*g*., non-naturally occurring or unnatural amino acid). Examples of non-naturally occurring amino acids include D-amino acids, an amino acid having an acetylaminomethyl group attached to a sulfur atom of a cysteine, a pegylated amino acid, the omega amino acids of the formula NH₂(CH₂)ₙCOOH wherein n is 2-6, neutral nonpolar amino acids, such as sarcosine, t-butyl alanine, t-butyl glycine, N-methyl isoleucine, and norleucine. Phenylglycine may substitute for Trp, Tyr, or Phe; citrulline and methionine sulfoxide are neutral nonpolar, cysteic acid is acidic, and ornithine is basic. Proline may be substituted with hydroxyproline and retain the conformation conferring properties.

Analogs may be generated by substitutional mutagenesis and retain the biological activity of the original polypeptide. Examples of substitutions identified as "conservative substitutions" are shown in Table 1. If such substitutions result in a change not desired, then other type of substitutions, denominated "exemplary substitutions" in Table 1, or as further described herein in reference to amino acid classes, are introduced and the products screened.

### Polypeptide derivatives and peptidomimetics

In addition to polypeptides consisting of naturally occurring amino acids, peptidomimetics or polypeptide analogs are also encompassed by the present invention and can form the targeting moiety or peptide/polypeptide agents used in the compounds of the invention. Polypeptide analogs are commonly used in the pharmaceutical industry as non-peptide drugs with properties analogous to those of the template polypeptide. The non-peptide compounds are termed "peptide mimetics" or peptidomimetics (Fauchere et al., Infect. Immun. 54:283-287 (1986) and Evans et al., J. Med. Chem. 30:1229-1239 (1987)). Peptide mimetics that are structurally related to therapeutically useful peptides or polypeptides may be used to produce an equivalent or enhanced therapeutic or prophylactic effect. Generally, peptidomimetics are structurally similar to the paradigm polypeptide (*i*.*e*., a polypeptide that has a biological or pharmacological activity) such as naturally-occurring receptor-binding polypeptides, but have one or more peptide linkages optionally replaced by linkages such as -CH₂NH-, -CH₂S-, -CH₂-CH₂-, -CH=CH- (cis and trans), -CH₂SO-, -CH(OH)CH₂-, -COCH₂- etc., by methods well known in the art (Spatola, Peptide Backbone Modifications, Vega Data, 1:267, 1983; Spatola et al., Life Sci. 38:1243-1249, 1986; Hudson et al., Int. J. Pept. Res. 14:177-185, 1979; and Weinstein, 1983, Chemistry and Biochemistry, of Amino Acids, Peptides and Proteins, Weinstein eds, Marcel Dekker, New York). Such polypeptide mimetics may have significant advantages over naturally occurring polypeptides including more economical production, greater chemical stability, enhanced pharmacological properties (*e*.*g*., half-life, absorption, potency, and efficiency), reduced antigenicity, and others.

While the targeting moieties described herein may efficiently cross the BBB or target particular cell types (*e.g*., those described herein), their effectiveness may be reduced by the presence of proteases. Likewise, the effectiveness of the peptide/polypeptide agents used in the invention may be similarly reduced. Serum proteases have specific substrate requirements, including L-amino acids and peptide bonds for cleavage. Furthermore, exopeptidases, which represent the most prominent component of the protease activity in serum, usually act on the first peptide bond of the polypeptide and require a free N-terminus (Powell et al., Pharm. Res. 10:1268-1273, (1993)). In light of this, it is often advantageous to use modified versions of polypeptides. The modified polypeptides retain the structural characteristics of the original L-amino acid polypeptides, but advantageously are not readily susceptible to cleavage by protease and/or exopeptidases.

Systematic substitution of one or more amino acids of a consensus sequence with D-amino acid of the same type (*e*.*g*., an enantiomer; D-lysine in place of L-lysine) may be used to generate more stable polypeptides. Thus, a polypeptide derivative or peptidomimetic as described herein may be all L-, all D-, or mixed D, L polypeptides. The presence of an N-terminal or C-terminal D-amino acid increases the *in vivo* stability of a polypeptide because peptidases cannot utilize a D-amino acid as a substrate (Powell et al., Pharm. Res. 10:1268-1273 (1993)). Reverse-D polypeptides are polypeptides containing D-amino acids, arranged in a reverse sequence relative to a polypeptide containing L-amino acids. Thus, the C-terminal residue of an L-amino acid polypeptide becomes N-terminal for the D-amino acid polypeptide, and so forth. Reverse D-polypeptides retain the same tertiary conformation and therefore the same activity, as the L-amino acid polypeptides, but are more stable to enzymatic degradation *in vitro* and *in vivo,* and thus have greater therapeutic efficacy than the original polypeptide (Brady and Dodson, Nature 368:692-693,1994 and Jameson et al., Nature 368:744-746 (1994)). In addition to reverse-D-polypeptides, constrained polypeptides including a consensus sequence may be generated by methods well known in the art (Rizo et al., Ann. Rev. Biochem. 61:387-418, (1992)). For example, constrained polypeptides may be generated by adding cysteine residues capable of forming disulfide bridges and, thereby, resulting in a cyclic polypeptide. Cyclic polypeptides have no free N- or C-termini. Accordingly, they are not susceptible to proteolysis by exopeptidases, although they are, of course, susceptible to endopeptidases, which do not cleave at polypeptide termini. The amino acid sequences of the polypeptides with N-terminal or C-terminal D-amino acids and of the cyclic polypeptides are usually identical to the sequences of the polypeptides to which they correspond, except for the presence of N-terminal or C-terminal D-amino acid residue, or their circular structure, respectively.

A cyclic derivative containing an intramolecular disulfide bond may be prepared by conventional solid phase synthesis while incorporating suitable S-protected cysteine or homocysteine residues at the positions selected for cyclization such as the amino and carboxy termini (Sah et al., J. Pharm. Pharmacol. 48:197 (1996)). Following completion of the chain assembly, cyclization can be performed either (1) by selective removal of the S-protecting group with a consequent on-support oxidation of the corresponding two free SH-functions, to form a S-S bonds, followed by conventional removal of the product from the support and appropriate purification procedure or (2) by removal of the polypeptide from the support along with complete side chain de-protection, followed by oxidation of the free SH-functions in highly dilute aqueous solution.

The cyclic derivative containing an intramolecular amide bond may be prepared by conventional solid phase synthesis while incorporating suitable amino and carboxyl side chain protected amino acid derivatives, at the position selected for cyclization. The cyclic derivatives containing intramolecular -S-alkyl bonds can be prepared by conventional solid phase chemistry while incorporating an amino acid residue with a suitable amino-protected side chain, and a suitable S-protected cysteine or homocysteine residue at the position selected for cyclization.

Another effective approach to confer resistance to peptidases acting on the N-terminal or C-terminal residues of a polypeptide is to add chemical groups at the polypeptide termini, such that the modified polypeptide is no longer a substrate for the peptidase. One such chemical modification is glycosylation of the polypeptides at either or both termini. Certain chemical modifications, in particular N-terminal glycosylation, have been shown to increase the stability of polypeptides in human serum (Powell et al., Pharm. Res. 10:1268-1273 (1993)). Other chemical modifications which enhance serum stability include, but are not limited to, the addition of an N-terminal alkyl group, consisting of a lower alkyl of from one to twenty carbons, such as an acetyl group, and/or the addition of a C-terminal amide or substituted amide group. In particular, the present invention includes modified polypeptides consisting of polypeptides bearing an N-terminal acetyl group and/or a C-terminal amide group.

Also included by the present invention are other types of polypeptide derivatives containing additional chemical moieties not normally part of the polypeptide, provided that the derivative retains the desired functional activity of the polypeptide. Examples of such derivatives include (1) N-acyl derivatives of the amino terminal or of another free amino group, wherein the acyl group may be an alkanoyl group (*e*.*g*., acetyl, hexanoyl, octanoyl) an aroyl group (*e*.*g*., benzoyl) or a blocking group such as F-moc (fluorenylmethyl-O-CO-); (2) esters of the carboxy terminal or of another free carboxy or hydroxyl group; (3) amide of the carboxy-terminal or of another free carboxyl group produced by reaction with ammonia or with a suitable amine; (4) phosphorylated derivatives; (5) derivatives conjugated to an antibody or other biological ligand and other types of derivatives.

Other derivatives included in the present invention are dual polypeptides consisting of two of the same, or two different polypeptides, as described herein, covalently linked to one another either directly or through a spacer, such as by a short stretch of alanine residues or by a putative site for proteolysis (*e*.*g*., by cathepsin, see *e.g.*, U.S. Patent No. 5,126,249 and European Patent No. 495 049).

The present invention also encompasses polypeptide derivatives that are chimeric or fusion proteins containing a polypeptide described herein, or fragment thereof, linked at its amino- or carboxy-terminal end, or both, to an amino acid sequence of a different protein. Such a chimeric or fusion protein may be produced by recombinant expression of a nucleic acid encoding the protein. For example, a chimeric or fusion protein may contain at least 7 amino acids shared with one of the described polypeptides which desirably results in a chimeric or fusion protein that has an equivalent or greater functional activity.

### Assays to identify peptidomimetics

As described above, non-peptidyl compounds generated to replicate the backbone geometry and pharmacophore display (peptidomimetics) of the polypeptides described herein often possess attributes of greater metabolic stability, higher potency, longer duration of action, and better bioavailability.

Peptidomimetics compounds can be obtained using any of the numerous approaches in combinatorial library methods known in the art, including biological libraries, spatially addressable parallel solid phase or solution phase libraries, synthetic library methods requiring deconvolution, the 'one-bead one-compound' library method, and synthetic library methods using affinity chromatography selection. The biological library approach is limited to peptide libraries, while the other four approaches are applicable to peptide, non-peptide oligomer, or small molecule libraries of compounds (Lam, Anticancer Drug Des. 12:145 (1997)). Examples of methods for the synthesis of molecular libraries can be found in the art, for example, in: DeWitt et al. (Proc. Natl. Acad. Sci. USA 90:6909 (1993)); Erb et al. (Proc. Natl. Acad. Sci. USA 91:11422 (1994)); Zuckermann et al. (J. Med. Chem. 37:2678 (1994)); Cho et al. (Science 261:1303 (1993)); Carell et al. (Angew. Chem. Int. Ed. Engl. 33:2059 (1994) and *ibid* 2061); and in Gallop et al. (Med. Chem. 37:1233 (1994)). Libraries of compounds may be presented in solution (*e*.*g*., Houghten, Biotechniques 13:412-421 (1992)) or on beads (Lam, Nature 354:82-84 (1991)), chips (Fodor, Nature 364:555-556 (1993)), bacteria or spores (U.S. Patent No. 5,223,409), plasmids (Cull et al., Proc. Natl. Acad. Sci. USA 89:1865-1869 (1992)) or on phage (Scott and Smith, Science 249:386-390 (1990)), or luciferase, and the enzymatic label detected by determination of conversion of an appropriate substrate to product.

Once a polypeptide as described herein is identified, it can be isolated and purified by any number of standard methods including, but not limited to, differential solubility (*e.g*., precipitation), centrifugation, chromatography (*e.g*., affinity, ion exchange, and size exclusion), or by any other standard techniques used for the purification of peptides, peptidomimetics, or proteins. The functional properties of an identified polypeptide of interest may be evaluated using any functional assay known in the art. Desirably, assays for evaluating downstream receptor function in intracellular signaling are used (*e.g.*, cell proliferation).

For example, the peptidomimetics compounds of the present invention may be obtained using the following three-phase process: (1) scanning the polypeptides described herein to identify regions of secondary structure necessary for targeting the particular cell types described herein; (2) using conformationally constrained dipeptide surrogates to refine the backbone geometry and provide organic platforms corresponding to these surrogates; and (3) using the best organic platforms to display organic pharmocophores in libraries of candidates designed to mimic the desired activity of the native polypeptide. In more detail the three phases are as follows. In phase 1, the lead candidate polypeptides are scanned and their structure abridged to identify the requirements for their activity. A series of polypeptide analogs of the original are synthesized. In phase 2, the best polypeptide analogs are investigated using the conformationally constrained dipeptide surrogates. Indolizidin-2-one, indolizidin-9-one and quinolizidinone amino acids (I²aa, I⁹aa and Qaa respectively) are used as platforms for studying backbone geometry of the best peptide candidates. These and related platforms (reviewed in Halab et al., Biopolymers 55:101-122 (2000) and Hanessian et al., Tetrahedron 53:12789-12854 (1997)) may be introduced at specific regions of the polypeptide to orient the pharmacophores in different directions. Biological evaluation of these analogs identifies improved lead polypeptides that mimic the geometric requirements for activity. In phase 3, the platforms from the most active lead polypeptides are used to display organic surrogates of the pharmacophores responsible for activity of the native peptide. The pharmacophores and scaffolds are combined in a parallel synthesis format. Derivation of polypeptides and the above phases can be accomplished by other means using methods known in the art.

Structure function relationships determined from the polypeptides, polypeptide derivatives, peptidomimetics or other small molecules described herein may be used to refme and prepare analogous molecular structures having similar or better properties.

In summary, based on the disclosure herein, those skilled in the art can develop peptides and peptidomimetics screening assays which are useful for identifying compounds for targeting an agent to particular cell types (*e*.*g*., those described herein). The assays may be developed for low-throughput, high-throughput, or ultra-high throughput screening formats. Assays include assays amenable to automation.

### Diseases and conditions

The compounds of the invention can be used to treat a variety of diseases and conditions. Because the compounds of the invention are able to cross the BBB or enter particular cell types (*e.g*., liver, eye, lung, kidney, spleen, muscle, or ovary), treatments of neurological disorders, including neurodegenerative diseases and cancer, and treatments of disorders related to particular cell types can be enhanced using the conjugates or therapeutic polypeptides of the invention.

### Delivery to particular target cell types and target tissues

The compounds of the invention can be used to delivery therapeutic agents or transport vectors to various organs and tissues (*e*.*g*., liver, eye, lung, kidney, spleen, muscle, or ovary). In accordance with the present invention, the targeting moiety may promote accumulation of a therapeutic agent in a tissue such as, for example, a liver (liver tissue), an eye (eye tissue), the lungs (lung tissue), a kidney (kidney tissue), a spleen (spleen tissue), muscle (muscle tissue), and ovary (ovary tissue) of a subject. Accordingly, the compounds may be used to treat a disease associated with these tissues (*e*.*g*., a cancer, such as any described herein; an infection, such as a bacterial infection or a viral infection; or an inflammatory condition).

Exemplary liver diseases include amebic liver abscess, cirrhosis, disseminated coccidioidomycosis, drug-induced cholestasis, hemochromatosis, hepatitis A, hepatitis B, hepatitis C, hepatitis D, hepatocellular carcinoma, liver cancer, liver disease due to alcohol, primary biliary cirrhosis, pyogenic liver abscess, Reye syndrome, sclerosing cholangitis, and Wilson's disease. Amebic liver abscess may be treated by administration of a therapeutic moiety conjugated to metronidazole. Hepatitis B may be treated, for example, by administration of a therapeutic moiety conjugated to inteiferon-alpha, lamivudine, adefovir dipivoxil, entecavir, or other antiviral agent. Hepatitis C may be treated, for example, by administration of a therapeutic moiety conjugated to pegylated interferon or ribavirin, or a combination thereof. Exemplary lung diseases include lung cancers such as small cell carcinoma (*e.g*., oat cell cancer), mixed small cell/large cell carcinoma, combined small cell carcinoma, and metastatic tumors. Metastatic tumors can originate from cancer of any tissue, including breast cancer, colon cancer, prostate cancer, sarcoma, bladder cancer, neuroblastoma, and Wilm's tumor. Exemplary spleen diseases include cancers, such as lymphoma, non-Hodgkin's lymphoma, and certain T-cell lymphomas.

The targeting moieties described herein may be capable of targeting therapeutic agents or transport vectors to a particular cell type (*e*.*g*., those described herein). Because the conjugates of the invention transport therapeutic agents or transport vectors to specific tissues, conjugates may result in lower toxicity (*e*.*g*., fewer side effects), higher efficacy (*e.g.*, because the agent is concentrated into a target tissue due to increased uptake or decreased efflux from the tissue or cells or because the agent has greater stability when conjugated), or a combination thereof. Such activities are described below and in International Publication No. WO 2007/009229.

Accordingly, a method of treating a subject having a disease or condition (*e*.*g*., any disease or condition associated with a target tissue, such as cancer) is disclosed by administering to the subject a conjugate or a composition including a conjugate of the invention, wherein the conjugate includes the therapeutic agent, in a dose lower (*e*.*g*., 5%, 10%, 15%, 20%, 30%, 50%, 70%, 80%, 90%, 95%, 98%, 99%, 99.9% lower) than the dose of the therapeutic agent alone.

### Cancer therapy

Compounds of the invention including anticancer agents may be used to treat any brain or central nervous system disease (*e.g*., a brain cancer such as glioblastoma, astrocytoma, glioma, meduloblastoma, and oligodendroma, neuroglioma, ependymoma, and meningioma). The compounds of the invention (*e.g.*, P1 to P6) can be used for transport to the liver, eye, lung, kidney, spleen, muscle, or ovary and may also be used, in conjunction with an appropriate therapeutic agent, to treat a disease associated with these tissues (*e*.*g*., a cancer such as hepatocellular carcinoma, liver cancer, small cell carcinoma (*e*.*g*., oat cell cancer), mixed small cell/large cell carcinoma, combined small cell carcinoma, and metastatic tumors). Metastatic tumors can originate from cancer of any tissue, including breast cancer, colon cancer, prostate cancer, sarcoma, bladder cancer, neuroblastoma, Wilm's tumor, lymphoma, non-Hodgkin's lymphoma, and certain T-cell lymphomas). Additional exemplary cancers that may be treated using a composition of the invention include hepatocellular carcinoma, breast cancer, cancers of the head and neck including various lymphomas such as mantle cell lymphoma, non-Hodgkin's lymphoma, adenoma, squamous cell carcinoma, laryngeal carcinoma, cancers of the retina, cancers of the esophagus, multiple myeloma, ovarian cancer, uterine cancer, melanoma, colorectal cancer, bladder cancer, prostate cancer, lung cancer (including non-small cell lung carcinoma), pancreatic cancer, cervical cancer, head and neck cancer, skin cancers, nasopharyngeal carcinoma, liposarcoma, epithelial carcinoma, renal cell carcinoma, gallbladder adenocarcinoma, parotid adenocarcinoma, endometrial sarcoma, multidrug resistant cancers; and proliferative diseases and conditions, such as neovascularization associated with tumor angiogenesis, macular degeneration (*e*.*g*., wet/dry AMD), corneal neovascularization, diabetic retinopathy, neovascular glaucoma, myopic degeneration and other proliferative diseases and conditions such as restenosis and polycystic kidney disease. Brain cancers that may be treated with vector that is transported efficiently across the BBB include glioma, mixed glioma, glioblastoma multiforme, astrocytoma, pilocytic astrocytoma, dysembryoplastic neuroepithelial tumor, oligodendroglioma, ependymoma, oligoastrocytoma, medulloblastoma, retinoblastoma, neuroblastoma, germinoma, and teratoma.

### Neurotensin-based therapy

The compounds of the invention can be used in any appropriate therapeutic application where the activity of neurotensin activity is beneficial. In brain, NT is associated with dopaminergic receptors and other neurotransmitter systems. Peripheral NT acts as a paracrine and endocrine peptide on both the digestive and cardiovascular systems. Various therapeutic applications have been suggested for neurotensin, including psychiatric disorders, metabolic disorder, and pain. Because neurotensin has been shown to modulate neurotransmission in areas of the brain associated with schizophrenia, neurotensin and neurotensin receptor agonists have been proposed as antipsychotic agents.

Because polypeptides described herein are capable of transporting an agent across the BBB, the compounds of the invention are also useful for the treatment of neurological diseases such as neurodegenerative diseases or other conditions of the central nervous system (CNS), the peripheral nervous system, or the autonomous nervous system (*e*.*g*., where neurons are lost or deteriorate). Neurotensin has been suggested an antipsychotic therapy, and thus may be useful in the treatment of diseases such as schizophrenia and bipolar disorder. Many neurodegenerative diseases are characterized by ataxia (*i.e*., uncoordinated muscle movements) and/or memory loss. Neurodegenerative diseases include Alexander disease, Alper disease, Alzheimer's disease, amyotrophic lateral sclerosis (ALS; *i.e*., Lou Gehrig's disease), ataxia telangiectasia, Batten disease (Spielmeyer-Vogt-Sjogren-Batten disease), bovine spongiform encephalopathy (BSE), Canavan disease, Cockayne syndrome, corticobasal degeneration, Creutzfeldt-Jakob disease, Huntington's disease, HIV-associated dementia, Kennedy's disease, Krabbé disease, Lewy body dementia, Machado-Joseph disease (Spinocerebellar ataxia type 3), multiple sclerosis, multiple system atrophy, narcolepsy, neuroborreliosis, Parkinson's disease, Pelizaeus-Merzbacher disease, Pick's disease, primary lateral sclerosis, prion diseases, Refsum's disease, Schilder's disease (*i.e*., adrenoleukodystrophy), schizophrenia, spinocerebellar ataxia, spinal muscular atrophy, Steele-Richardson, Olszewski disease, and tabes dorsalis.

The compounds of the invention may be used to reduce the body temperature of a subject. Because reduction in body temperature has been shown to be beneficial in subjects who may be suffering from, or may have recently suffered from, a stroke, cerebral ischemia, cardiac ischemia, or a nerve injury such as a spinal cord injury, such a treatment would therefore be useful in reducing complications of these conditions.

Neurotensin is also known to have analgesic effects. Thus the compounds of the invention may be used to reduce pain in a subject. The subject may be suffering from an acute pain (*e*.*g*., selected from the group consisting of mechanical pain, heat pain, cold pain, ischemic pain, and chemical-induced pain). Other types of pain include peripheral or central neuropathic pain, inflammatory pain, migraine-related pain, headache-related pain, irritable bowel syndrome-related pain, fibromyalgia-related pain, arthritic pain, skeletal pain, joint pain, gastrointestinal pain, muscle pain, angina pain, facial pain, pelvic pain, claudication, postoperative pain, post traumatic pain, tension-type headache, obstetric pain, gynecological pain, or chemotherapy-induced pain.

There is evidence that neurotensin-can be used to treat metabolic disorders; see, *e.g*., U.S. Patent Application No. 2001/0046956. Thus, the compounds of the invention may be used to treat such disorders. The metabolic disorder may be diabetes (*e*.*g*., Type I or Type II), obesity, diabetes as a consequence of obesity, hyperglycemia, dyslipidemia, hypertriglyceridemia, syndrome X, insulin resistance, impaired glucose tolerance (IGT), diabetic dyslipidemia, hyperlipidemia, a cardiovascular disease, or hypertension. The subject may be overweight, obese, or bulimic.

Neurotensin has also been suggested to be able to treat drug addiction or reduce drug abuse in subjects, particularly with psychostimulant. Thus the compounds of the invention may be useful in treating addiction to or abuse of drugs such as amphetamine, methamphetamine, 3,4-methylenedioxymethamphetamine, nicotine, cocaine, methylphenidate, and arecoline.

### GDNF/BNDF-based therapy

GDNF and BDNF-based compounds may be used to treat any disease or condition where enhancing neuronal survival (*e*.*g*., decreasing neuronal death rate) or increasing the rate of neuronal formation is beneficial. Such conditions include neurodegenerative disorders, *e*.*g*., a disorder selected from the group consisting of a polyglutamine expansion disorder (*e*.*g*., Huntington's disease (HD), dentatorubropallidoluysian atrophy, Kennedy's disease (also referred to as spinobulbar muscular atrophy), and spinocerebellar ataxia (*e*.*g*., type 1, type 2, type 3 (also referred to as Machado-Joseph disease), type 6, type 7, and type 17)), another trinucleotide repeat expansion disorder (*e.g.*, fragile X syndrome, fragile XE mental retardation, Friedreich's ataxia, myotonic dystrophy, spinocerebellar ataxia type 8, and spinocerebellar ataxia type 12), Alexander disease, Alper's disease, Alzheimer's disease, amyotrophic lateral sclerosis (ALS), ataxia telangiectasia, Batten disease (also referred to as Spielmeyer-Vogt-Sjogren-Batten disease), Canavan disease, Cockayne syndrome, corticobasal degeneration, Creutzfeldt-Jakob disease, ischemia stroke, Krabbe disease, Lewy body dementia, multiple sclerosis, multiple system atrophy, Parkinson's disease, Pelizaeus-Merzbacher disease, Pick's disease, primary lateral sclerosis, Refsum's disease, Sandhoff disease, Schilder's disease, spinal cord injury, spinal muscular atrophy, Steele-Richardson-Olszewski disease, and Tabes dorsalis. Other conditions include injury (*e*.*g*., spinal cord injury), concussion, ischemic stroke, and hemorrhagic stroke.

### GLP-1-based therapy

The compounds of the invention including a GLP-1 agonist can be used in any therapeutic application where a GLP-1 agonist activity in the brain, or in a particular tissue, is desired. GLP-1 agonist activity is associated with stimulation of insulin secretion (*i*.*e*., to act as an incretin hormone) and inhihition glucagon secretion, thereby contributing to limit postprandial glucose excursions. GLP-1 agonists can also inhibit gastrointestinal motility and secretion, thus acting as an enterogastrone and part of the "ileal brake" mechanism. GLP-1 also appears to be a physiological regulator of appetite and food intake. Because of these actions, GLP-1 and GLP-1 receptor agonists can be used for therapy of metabolic disorders, as reviewed in, *e.g*., Kinzig et al., J. Neurosci. 23:6163-6170 (2003). Such disorders include obesity, hyperglycemia, dyslipidemia, hypertriglyceridemia, syndrome X, insulin resistance, IGT, diabetic dyslipidemia, hyperlipidemia, a cardiovascular disease, and hypertension.

GLP-1 is also has neurological effects including sedative or anti-anxiolytic effects, as described in U.S. Patent No. 5,846,937. Thus, GLP-1 agonists can be used in the treatment of anxiety, aggression, psychosis, seizures, panic attacks, hysteria, or sleep disorders. GLP-1 agonists can also be used to treat Alzheimer's disease, as GLP-1 agonists have been shown to protect neurons against amyloid-β peptide and glutamate-induced apoptosis (Perry et al., Curr. Alzheimer Res. 2:377-85 (2005)).

Other therapeutic uses for GLP-1 agonists include improving learning, enhancing neuroprotection, and alleviating a symptom of a disease or disorder of the central nervous system, *e.g*., through modulation of neurogenesis, and *e.g*., Parkinson's Disease, Alzheimer's Disease, Huntington's Disease, ALS, stroke, ADD, and neuropsychiatric syndromes (U.S. Patent No. 6,969,702 and U.S. Patent Application No. 2002/0115605). Stimulation of neurogenesis using GLP-1 agonists has been described, for example, in Bertilsson et al., J. Neurosci. Res. 86:326-338 (2008).

Still other therapeutic uses include converting liver stem/progenitor cells into functional pancreatic cells (U.S. Patent Application Publication No. 2005/0053588); preventing beta-cell deterioration (U.S. Patent Nos. 7,259,233 and 6,569,832) and stimulation of beta-cell proliferation (U.S. Patent Application Publication No. 2003/0224983); treating obesity (U.S. Patent No. 7,211,557); suppressing appetite and inducing satiety (U.S. Patent Application Publication No. 2003/0232754); treating irritable bowel syndrome (U.S. Patent No. 6,348,447); reducing the morbidity and/or mortality associated with myocardial infarction (U.S. Patent No. 6,747,006) and stroke (PCT Publication No. WO 00/16797); treating acute coronary syndrome characterized by an absence of Q-wave myocardial infarction (U.S. Patent No. 7,056,887); attenuating post-surgical catabolic changes (U.S. Patent No. 6,006,753); treating hibernating myocardium or diabetic cardiomyopathy (U.S. Patent No. 6,894,024); suppressing plasma blood levels of norepinepherine (U.S. Patent No. 6,894,024); increasing urinary sodium excretion, decreasing urinary potassium concentration (U.S. Patent No. 6,703,339); treating conditions or disorders associated with toxic hypervolemia, *e.g*., renal failure, congestive heart failure, nephrotic syndrome, cirrhosis, pulmonary edema, and hypertension (U.S. Patent No. 6,703,339); inducing an inotropic response and increasing cardiac contractility (U.S. Patent No. 6,703,359); treating polycystic ovary syndrome (U.S. Patent No. 7,105,489); treating respiratory distress (U.S. Patent Application Publication No. 2004/0235726); improving nutrition via a non-alimentary route, *i.e.*, via intravenous, subcutaneous, intramuscular, peritoneal, or other injection or infusion (U.S. Patent No. 6,852,690); treating nephropathy (U.S. Patent Application Publication No. 2004/0209803); treating left ventricular systolic dysfunction, *e.g.*, with abnormal left ventricular ejection fraction (U.S. Patent No. 7,192,922); inhibiting antro-duodenal motility, *e.g.*, for the treatment or prevention of gastrointestinal disorders such as diarrhea, postoperative dumping syndrome and irritable bowel syndrome, and as premedication in endoscopic procedures (U.S. Patent No. 6,579,851); treating critical illness polyneuropathy (CIPN) and systemic inflammatory response syndrome (SIRS) (U.S. Patent Application Publication No. 2003/0199445); modulating triglyceride levels and treating dyslipidemia (U.S. Patent Application Publication Nos. 2003/0036504 and 2003/0143183); treating organ tissue injury caused by reperfusion of blood flow following ischemia (U.S. Patent No. 6,284,725); treating coronary heart disease risk factor (CHDRF) syndrome (U.S. Patent No. 6,528,520); and others.

### Leptin-based therapy

Compounds of the invention that include leptin or a related molecule can be used to treat metabolic disorders, neurological diseases, as well as other indications.

In certain embodiments, the compound of the invention is used to treat a metabolic disorder. Such disorders include diabetes (type I or type II), obesity, hyperglycemia, dyslipidemia, hypertriglyceridemia, syndrome X, insulin resistance, IGT, diabetic dyslipidemia, hyperlipidemia, a cardiovascular disease, and hypertension. Leptin decreases food intake and thus can be used to reduce weight and to treat diseases where reduced food intake or weight loss is beneficial.

### Administration and dosage

The present invention also features pharmaceutical compositions that contain a therapeutically effective amount of a therapeutic polypeptide or a conjugate of the invention. The composition can be formulated for use in a variety of drug delivery systems. One or more physiologically acceptable excipients or carriers can also be included in the composition for proper formulation. Suitable formulations for use in the present invention are found in Remington's Pharmaceutical Sciences, Mack Publishing Company, Philadelphia, PA, 17th ed., 1985. For a brief review of methods for drug delivery, see, *e*.*g*., Langer (Science 249:1527-1533, 1990).

The pharmaceutical compositions are intended for parenteral, intranasal, topical, oral, or local administration, such as by a transdermal means, for prophylactic and/or therapeutic treatment. The pharmaceutical compositions can be administered parenterally (*e*.*g*., by intravenous, intramuscular, or subcutaneous injection), or by oral ingestion, or by topical application or intraarticular injection at areas affected by the vascular or cancer condition. Additional routes of administration include intravascular, intra-arterial, intratumor, intraperitoneal, intraventricular, intraepidural, as well as nasal, ophthalmic, intrascleral, intraorbital, rectal, topical, or aerosol inhalation administration. Sustained release administration is also specifically included in the invention, by such means as depot injections or erodible implants or components. Thus, the invention provides compositions for parenteral administration that include the above mention agents dissolved or suspended in an acceptable carrier, preferably an aqueous carrier, *e*.*g*., water, buffered water, saline, PBS, and the like. The compositions may contain pharmaceutically acceptable auxiliary substances as required to approximate physiological conditions, such as pH adjusting and buffering agents, tonicity adjusting agents, wetting agents, detergents and the like. The invention also provides compositions for oral delivery, which may contain inert ingredients such as binders or fillers for the formulation of a tablet, a capsule, and the like. Furthermore, this invention provides compositions for local administration, which may contain inert ingredients such as solvents or emulsifiers for the formulation of a cream, an ointment, and the like.

These compositions may be sterilized by conventional sterilization techniques, or may be sterile filtered. The resulting aqueous solutions may be packaged for use as is, or lyophilized, the lyophilized preparation being combined with a sterile aqueous carrier prior to administration. The pH of the preparations typically will be between 3 and 11, more preferably between 5 and 9 or between 6 and 8, and most preferably between 7 and 8, such as 7 to 7.5. The resulting compositions in solid form may be packaged in multiple single dose units, each containing a fixed amount of the above-mentioned agent or agents, such as in a sealed package of tablets or capsules. The composition in solid form can also be packaged in a container for a flexible quantity, such as in a squeezable tube designed for a topically applicable cream or ointment.

The compositions containing an effective amount can be administered for prophylactic or therapeutic treatments. In prophylactic applications, compositions can be administered to a subject with a clinically determined predisposition or increased susceptibility to a neurological or neurodegenerative disease. Compositions of the invention can be administered to the subject (*e*.*g*., a human) in an amount sufficient to delay, reduce, or preferably prevent the onset of clinical disease. In therapeutic applications, compositions are administered to a subject (*e.g.*, a human) already suffering from disease (*e*.*g*., a neurological condition or neurodegenerative disease) in an amount sufficient to cure or at least partially arrest the symptoms of the condition and its complications. An amount adequate to accomplish this purpose is defined as a "therapeutically effective amount," an amount of a compound sufficient to substantially improve some symptom associated with a disease or a medical condition. For example, in the treatment of a neurodegenerative disease (*e*.*g*., those described herein), an agent or compound that decreases, prevents, delays, suppresses, or arrests any symptom of the disease or condition would be therapeutically effective. A therapeutically effective amount of an agent or compound is not required to cure a disease or condition but will provide a treatment for a disease or condition such that the onset of the disease or condition is delayed, hindered, or prevented, or the disease or condition symptoms are ameliorated, or the term of the disease or condition is changed or, for example, is less severe or recovery is accelerated in an individual.

Amounts effective for this use may depend on the severity of the disease or condition and the weight and general state of the subject, but generally range from about 0.05 µg to about 1000 µg (*e.g.*, 0:5-100 µg) of an equivalent amount of the agent per dose per subject. Suitable regimes for initial administration and booster administrations are typified by an initial administration followed by repeated doses at one or more hourly, daily, weekly, or monthly intervals by a subsequent administration. The total effective amount of an agent present in the compositions of the invention can be administered to a mammal as a single dose, either as a bolus or by infusion over a relatively short period of time, or can be administered using a fractionated treatment protocol, in which multiple doses are administered over a more prolonged period of time (*e*.*g*., a dose every 4-6, 8-12,14-16, or 18-24 hours, or every 2-4 days, 1-2 weeks, once a month). Alternatively, continuous intravenous infusions sufficient to maintain therapeutically effective concentrations in the blood are contemplated.

The therapeutically effective amount of one or more agents present within the compositions of the invention and used in this invention applied to mammals (*e*.*g*., humans) can be determined by the ordinarily-skilled artisan with consideration of individual differences in age, weight, and the condition of the mammal. Because certain compounds of the invention exhibit an enhanced ability to cross the BBB, the dosage of the compounds of the invention can be lower than (e.g., less than or equal to about 90%, 75%, 50%, 40%, 30%, 20%, 15%, 12%, 10%, 8%, 7%, 6%, 5%, 4%, 3%, 2%, 1%, 0.5%, or 0.1 % of) the equivalent dose of required for a therapeutic effect of the unconjugated agent. The agents of the invention are administered to a subject (*e.g.* a mammal, such as a human) in an effective amount, which is an amount that produces a desirable result in a treated subject (*e*.*g*., preservation of neurons, new neuronal growth). Therapeutically effective amounts can also be determined empirically by those of skill in the art.

The subject may also receive an agent in the range of about 0.05 to 1,000 µg equivalent dose as compared to unconjugated agent per dose one or more times per week (*e*.*g*., 2, 3, 4, 5, 6, or 7 or more times per week), 0.1 to 2,500 (*e*.*g*., 2,000, 1,500, 1,000, 500, 100, 10, 1, 0.5, or 0.1) µg dose per week. A subject may also receive an agent of the composition in the range of 0.1 to 3,000 µg per dose once every two or three weeks.

Single or multiple administrations of the compositions of the invention including an effective amount can be carried out with dose levels and pattern being selected by the treating physician. The dose and administration schedule can be determined and adjusted based on the severity of the disease or condition in the subject, which may be monitored throughout the course of treatment according to the methods commonly practiced by clinicians or those described herein.

The compounds of the present invention may be used in combination with either conventional methods of treatment or therapy or may be used separately from conventional methods of treatment or therapy.

When the compounds of this invention are administered in combination therapies with other agents, they may be administered sequentially or concurrently to an individual. Alternatively, pharmaceutical compositions according to the present invention may be comprised of a combination of a compound of the present invention in association with a pharmaceutically acceptable excipient, as described herein, and another therapeutic or prophylactic agent known in the art.

### Further conjugation

It is disclosed that the conjugate or therapeutic polypeptide may be further linked to another agent, such as a therapeutic agent, a detectable label, or any other agent described herein. The conjugate may be labeled with a detectable label such as a radioimaging agent, such as those emitting radiation, for detection of a disease or condition. It is disclosed that the carrier or functional derivative thereof or mixtures thereof may be linked to a therapeutic agent, to treat a disease or condition, or may be linked to or labeled with mixtures thereof. Treatment may be effected by administering a conjugate of the present invention that has been further conjugated to a therapeutic compound to an individual under conditions which allow transport of the agent across the BBB or to other cells or tissues where such treatment is beneficial.

A therapeutic agent as used herein may be a drug, a medicine, an agent emitting radiation, a cellular toxin (for example, a chemotherapeutic agent) and/or biologically active fragment thereof, and/or mixtures thereof to allow cell killing or it may be an agent to treat, cure, alleviate, improve, diminish or inhibit a disease or condition in an individual treated. A therapeutic agent may be a synthetic product or a product of fungal, bacterial or other microorganism, such as mycoplasma, viral etc., animal, such as reptile, or plant origin. A therapeutic agent and/or biologically active fragment thereof may be an enzymatically active agent and/or fragment thereof, or may act by inhibiting or blocking an important and/or essential cellular pathway or by competing with an important and/or essential naturally occurring cellular component.

Covalent modifications of the compounds, conjugates, and compositions of the invention are disclosed. A chemical derivative may be conveniently prepared by direct chemical synthesis, using methods well known in the art. Such modifications may be, for example, introduced into a polypeptide, agent, or conjugate by reacting targeted amino acid residues with an organic derivatizing agent that is capable of reacting with selected side chains or terminal residues. A transport vector derivative may be able, *e.g*., to cross the BBB and be attached to or conjugated with another agent, thereby transporting the agent across the BBB. The conjugate of the invention may be joined (*i.e.*, conjugated), through sulfhydryl groups, amino groups (amines) and/or carbohydrates to suitable detectable labels or therapeutic agents. Homobifunctional and heterobifunctional cross-linkers (conjugation agents) are available from many commercial sources. Regions available for cross-linking may be found on the carriers of the present invention. The cross-linker may comprise a flexible arm, such as for example, a short arm (< 2 carbon chain), a medium-size arm (from 2-5 carbon chain), or a long arm (3-6 carbon chain). Exemplary cross-linkers include BS3 ([Bis(sulfosuccinimidyl)suberate]; BS3 is a homobifunctional N-hydroxysuccinimide ester that targets accessible primary amines), NHS/EDC (N-hydroxysuccinimide and N-ethyl-'(dimethylaminopropyl)carbodimide; NHS/EDC allows for the conjugation of primary amine groups with carboxyl groups), sulfo-EMCS ([N-e-Maleimidocaproic acid]hydrazide; sulfo-EMCS are heterobifunctional reactive groups (maleimide and NHS-ester) that are reactive toward sulfhydryl and amino groups), hydrazide (most proteins contain exposed carbohydrates and hydrazide is a useful reagent for linking carboxyl groups to primary amines), and SATA (N-succinimidyl-S-acetylthioacetate; SATA is reactive towards amines and adds protected sulfhydryls groups).

### Example 1

### Synthesis of shorter analogs of Angiopep-2-Cys (P1 to P6)

SPPS (Solid phase peptide synthesis) was carried out on a Protein Technologies, Inc. Symphony® peptide synthesizer using Fmoc (9-fluorenylmethyloxycarbonyl) amino-terminus protection. Shorter Angiopeps were synthesized on a 100 µmol scale using a 5-fold excess of Fmoc-amino acids (200 mM) relative to the resin. The crude peptide was precipitated using ice-cold ether, and purified by RP-HPLC chromatography (Waters Delta Prep 4000). Acetonitrile was evaporated from the collected fractions and lyophilized to give a pure white solid (purity >95 %). The mass was confirmed by ESI-TOF MS (Bruker Daltonics). Table 4 provides the sequences of the Angiopep-2-Cys (AN2-Cys) and various shorter analogs (P1 to P6).

**Table 4. Shorter analogs of Angiopep-2-Cys (P1 to P6)**

| **Peptide** | N (AA) | C | Av. Hydrophilicity | Mw | Sequence |
|---|---|---|---|---|---|
| **AN2Cys-NH2** | 20 | + 3 | 0.2 | 2403.6 | TFFYGGSRG KRNNFK TEEYC-NH2 |
| **P1** | 18 | + 3 | 0.4 | 2155.4 | FYGGSRG KRNNFK TEEYC-NH2 |
| **P2** | 16 | + 3 | 0.8 | 1845.0 | GGSRG KRNNFK TEEYC-NH2 |
| **P3** | 14 | + 3 | 0.9 | 1730.9 | SRG KRNNFK TEEYC-NH2 |
| **P4** | 12 | + 2 | 0.8 | 1487.7 | G KRNNFK TEEYC-NH2 |
| **P5** | 11 | + 2 | 0.8 | 1430.6 | KRNNFK TEEYC-NH2 |
| **P6** | 8 | + 4 | 0.5 | 1071.3 | -------- KRNNFK YC-NH2 |

### Example 2

### Transport of shorter analogs P1 and P5

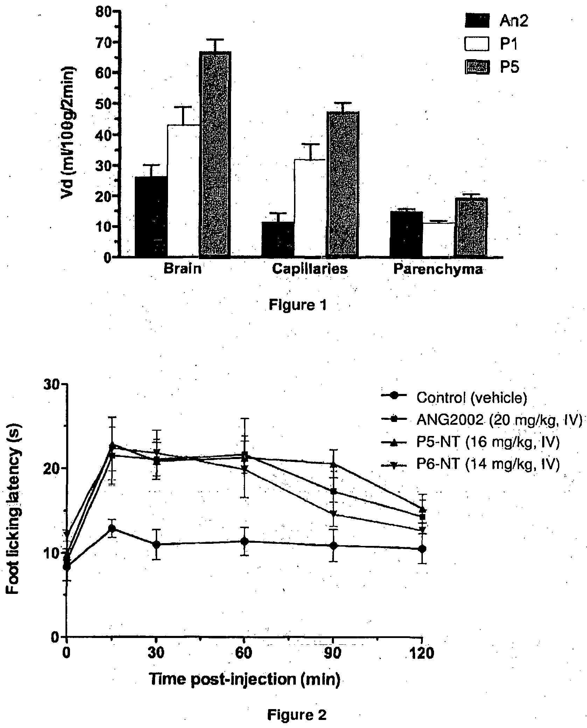

To confirm that the shorter analogs P1 and P5 cross the BBB, *in situ* brain perfusion was performed using methods standard in the art. The initial transport was measured as a function of time. Results indicate that the brain uptake for P1 and P5 is similar to or higher than for the Angiopep-2 (An2) (Figure 1). Capillary depletion was also done to quantify the amount of the analog found in the brain parenchyma (Figure 1). Similar or higher levels of P5 were found in the brain parenchyma when compared to An2 and P1. In addition, these results indicate that the analogs are not trapped in the brain capillaries. Overall, our results demonstrate that the new shorter analogs P1 and P5 effectively cross the BBB.

### Example 3

### Characterization of neurotensin derivatives of P5 and P6

We performed experiments to test whether the shorter analogs were able to induce analgesia or sustained hypothermia in mice, as compared to ANG2002 (modified neurotensin (NT) conjugated to Angiopep-2 via an EMCS linker) having the structure: 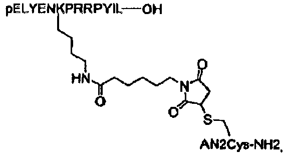 The tested conjugates included P5-NT having the sequence KRNNFKTEEYC-pELYENKPRRPYIL and P6-NT having the sequence KRNNFKYC-pEL YENKPRRPYIL, where P5 and P6 are both conjugated on the lysine of NT via an EMCS linker and pE denotes pyro-L-glutamic acid.

To determine induction of analgesia, we tested the latency between hot plate foot exposure and foot licking behavior in control mice and in mice receiving ANG2002, P5-NT and P6-NT at an equivalent dose of neurotensin. Thus, mice received either an intravenous 20 mg/kg bolus injection of ANG2002, an intravenous 16 mg/kg bolus injection of P5-NT, or an intravenous 14 mg/kg bolus injection of P6-NT. All of the tested conjugates increased the latency of foot licking behavior 15 minutes following injection, thus indicating that ANG2002, P5-NT, and P6-NT can act as an analgesic (Figure 2).

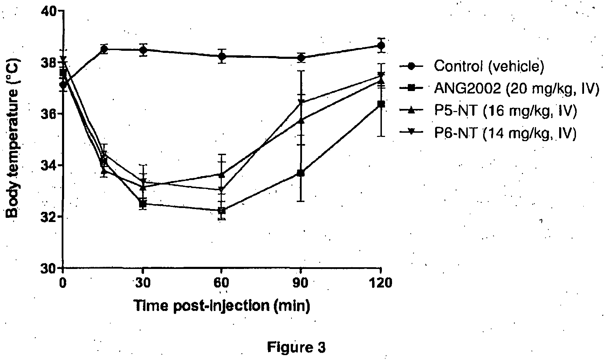

We performed additional experiments to test whether the shorter analogs were able to induce sustained hypothermia in mice, as compared to ANG2002. Mice received an intravenous 20 mg/kg bolus injection of ANG2002, an intravenous 16 mg/kg bolus injection of P5-NT, or an intravenous 14 mg/kg bolus injection of P6-NT. The body temperature continued to decrease after the injection; thus, ANG2002, P5-NT, and P6-NT have comparable activity in inducing hypothermia (Figure 3).

### Example 4

### Exemplary oral formulation of shorter analogs having D-isomers

Oral formulations can be made having Angoipep-2 and shorter analogs thereof. Shorter analogs were prepared by determining the cleavage sites of pepsin and trypsin and by substituting particular amino acids with its corresponding D-isomers. Figure 4 shows predicted cleavage sites for Angiopep-2 (*i*.*e.*, C-terminal of positions 1,2,3,4, 14, 18, and 19 for pepsin and positions 8, 10, 11, and 15 for trypsin) and P6a (*i.e.*, C-terminal of position 6 for trypsin).

The stability of oral formulations (1 mg/mL of Angiopep peptide) was determined in the presence of 1 mg/25 mL of pepsin (diluted 41 times, pH = 1.4), an enzyme present in gastric fluid, at 37°C. Table 5 provides the half-life (t_{1/2}) for degradation of various Angiopep peptides.

**Table 5. Stability of oral formulations of Angiopep peptides**

| **Angiopep Peptides** | **Amino acid sequence** | | | | | | | | | | | | | | | | | | | | **t_{½}** |
|---|---|---|---|---|---|---|---|---|---|---|---|---|---|---|---|---|---|---|---|---|---|
| An2 | T | F | F | Y | G | G | S | R | G | K | R | N | N | F | K | T | E | E | Y | | 40 min. |
| 3D-An2 | T | F | F | Y | G | G | S | D-R | G | D-K | D-R | N | N | F | K | T | E | E | Y | | 60 min. |
| P5 | | | | | | | | | | K | R | N | N | F | K | T | E | E | Y | C | 24 min. |
| P5a | | | | | | | | | | D-K | D-R | N | N | D-F | K | T | E | E | Y | C | 21 min. |
| P6 | | | | | | | | | | K | R | N | N | F | K | - | - | - | Y | C | > 4 hrs |
| P6a | | | | | | | | | | D-K | D-R | N | N | D-F | K | - | - | - | Y | C | > 18 hrs |

Generally, half-life increased for analogs having D-isomers compared to analogs having all L-isomers of the same amino acid residues. In particular, a shortened analog having D-amino acid substitutions (corresponding to positions 10, 11, and 14 of An2) provided a polypeptide having a half-life of > 18 hours and only one cleavage site for trypsin (see P6a in Table 5 and Figure 4). Overall, our results demonstrate that the new shorter analogs having D-isomers generally are more stable against degradation.

### Example 5

### In vitro characterization of neurotensin derivatives of analogs having D-isomers

We performed experiments to test whether shorter conjugates having D-isomers were able to competitively bind the neurotensin receptor in a human colon adenocarcinoma (HT29) cell assay with [³H]-neurotensin. The tested conjugates included ANG2002, P5a-NT, P6-NT, and P6a-NT, where sequences for P5a, P6, and P6a are provided in Table 5. For these conjugates, "NT" is pELYENKPRRPYIL, where pE denotes pyro-L-glutamic acid, and P5a, P6, and P6a are conjugated on the lysine of NT via an EMCS linker.

P5a-NT and P6a-NT included three D-amino acid substitutions, as shown in Table 6. Results indicate that P5a, P6, and P6a are more potent than Angiopep-2 (*i.e.*, as ANG2002) (Figure 5 and Table 6). In particular, P6a-NT having three D-amino acid substitutions had a comparable IC₅₀ value to P6-NT having the same sequence but only L-amino acid residues.

**Table 6. Binding of neurotensin derivatives of shorter analogs**

| Peptide | IC₅₀ [nM] |
|---|---|
| Neurotensin | 0.4 |
| ANG2002* | 1.7 |
| P5-NT | ND |
| P5a-NT | 1.4 |
| P6-NT | 0.4 |
| P6a-NT | 0.6 |

| | |
|---|---|
| ND: not determined *: see structure for ANG2002 provided herein in Example 3 | |

### Example 6

### Transport of neurotensin derivatives of P6a having D-isomers

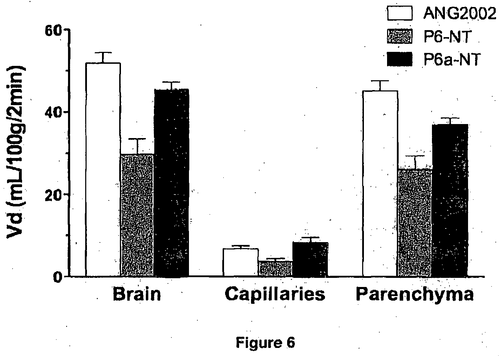

To confirm that shorter conjugates having D-isomers cross the BBB, *in situ* brain perfusion was performed using methods standard in the art. The initial transport was measured as a function of time. The tested conjugates included P6-NT, P6a-NT, and ANG2002, as described above in Examples 3 and 5. Results indicate that the brain uptake for P6-NT and P6a-NT is similar to that for ANG2002 and that the brain uptake for P6a-NT is higher than that for P6-NT (Figure 6). Capillary depletion was also done to quantify the amount of the analog found in the brain parenchyma, and P6a-NT showed increased presence in the parenchyma compared to P6-NT (Figure 6). In addition, our results demonstrate that the new shorter conjugate P6a-NT having D-amino acid residues crosses the BBB more effectively than conjugate P6-NT lacking D-amino acid residues.

### Example 7

### Hypothermia induction by neurotensin derivatives having D-isomers

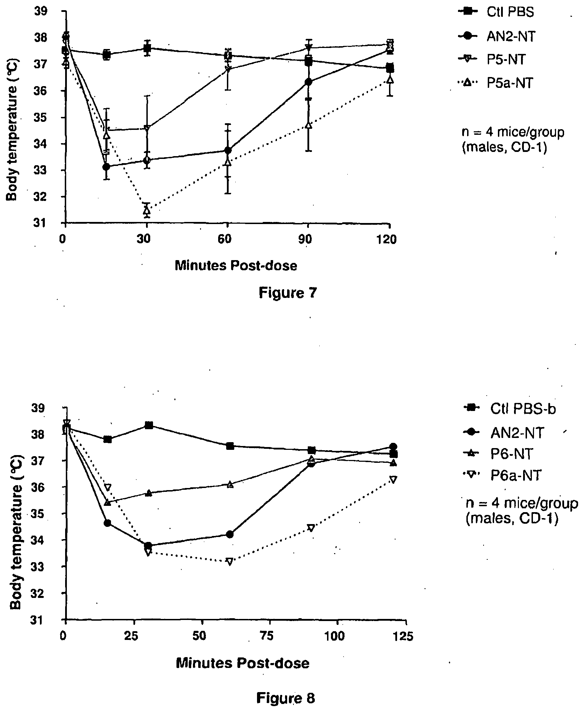

We performed experiments to test whether the shorter conjugates having D-isomers were able to induce sustained hypothermia in mice, as compared to AN2-NT (a fusion protein including Angiopep-2 and neurotensin, as shown in Figure 9). The tested conjugates included P5-NT, P5a-NT, P6-NT, and P6a-NT, as provided in Table 6 above. Mice received an intravenous 4.682 µmol/kg bolus injection (equivalent to 20 mg/kg of AN2-NT) of AN2-NT, P5-NT, P5a-NT, P6-NT, and P6a-NT. The body temperature continued to decrease after the injection of tested conjugates (Figures 7 and 8). In particular, shorter conjugates P5a-NT and P6a-NT having D-isomers had an increased effect on the body temperature in mice compared to AN2-NT and shorter peptides lacking D-isomers,

### Example 8

### Synthesis of conjugates having shorter NT1 neurotensin derivatives

To reduce the length of the conjugate, further shortened derivatives of neurotensin (NT1) were also prepared. SPPS (Solid phase peptide synthesis) was carried out on a Protein Technologies, Inc. Symphony® peptide synthesizer using Fmoc (9-fluorenylmethyl oxycarbonyl) amino-terminus protection. Shorter Angiopeps with and without neurotensin analogs were synthesized on a 100 µmol scale using a 5-fold excess of Fmoc-amino acids (200 mM) relative to the resin. The crude peptide was precipitated using ice-cold ether, and purified by RP-HPLC chromatography (Waters Delta Prep 4000). Acetonitrile was evaporated from the collected fractions and lyophilized to give a pure white solid (purity >95 %). The mass was confirmed by ESI-TOF MS (Bruker Daltonics). Table 7 provides the sequences of various shorter analogs (P5a to P6c) and corresponding conjugates having neurotensin derivative NT1: Ac-KRRP(D-Y)IL, where shorter analogs (P5a to P6c) are conjugated on the lysine of NT1 via an EMCS linker.

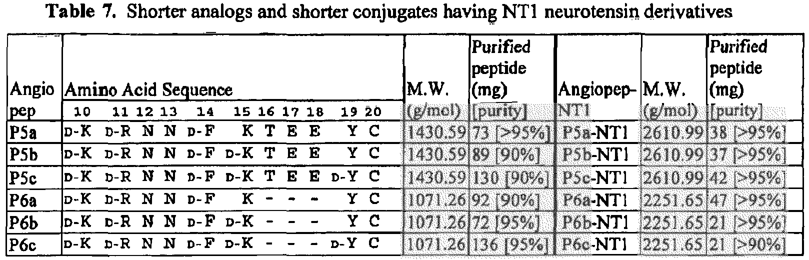

### Example 9

### Cleavage sites for conjugates of neurotensin(6-13) analogs having D-isomers

Shorter conjugates were also designed by determining the possible cleavage sites for pepsin and trypsin and by replacing amino acid residues at those cleavage sites with one or more D-isomers of the same amino acid residue. Figure 9 shows exemplary cleavage sites for An2-NT and shorter conjugates determined by PeptideCutter, a predictive model for determining enzyme cleavage sites, and Table 8 provides an alignment of the sequences of Figure 9. Overall, reduced cleavage was predicted by including D-amino acid substitutions in the targeting moiety (*i.e.*, in the P6a or P6b sequence) and in the neurotensin derivative portion (Figure 9).

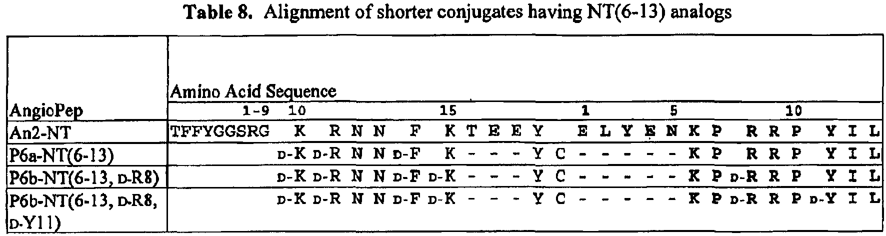

### Example 10

### Transport of conjugates of shorter NT1 neurotensin derivatives

To confirm that conjugates having both shorter Angiopep analogs and shorter neurotensin derivatives cross the BBB, *in situ* brain perfusion was performed using methods standard in the art. The initial transport of P5a-NT1, P5b-NT1, P5c-NT1, and P6a-NT1 was measured as a function of time. The sequences for these peptides are described in Example 8. Results indicate that the brain uptake for these peptides is higher than for neurotensin (NT) (Figure 10). Capillary depletion was also done to quantify the amount of the analog found in the brain parenchyma (Figure 10). Overall, our results demonstrate that the new shorter conjugates having D-amino acid residues cross the BBB.

### Example 11

### Comparison of activity for various neurotensin derivatives

We performed experiments to test whether the shorter conjugates were able to induce sustained analgesia, hypothermia, and/or hypotension in mice, as compared to ANG2002 (as shown above in Example 3), AN2-Ahx-NT (Angiopep-2 conjugated to neurotensin having sequence ELYENKPRRPYIL via an aminohexanoic acid (Ahx) linker), and AN2-NT (Angiopep-2 directly conjugated (*i.e.*, without a linker) to neurotensin having sequence ELYENKPRRPYIL, as shown in Figure 9). The tested conjugates included P5-NT, P5a-NT, P6-NT, P6a-NT, P5a-NT1, and P6a-NT1 (as described above in Examples 5 and 8).

Table 9 provides a summary of these results. An2-Ahx-NT induced analgesia, hypothermia, and hypotension similarly to ANG2002, where the use of a linker maintained activity. Shorter peptides P5a-NT and P6a-NT including D-isomers provided an increased effect on body temperature in mice and retained their analgesic properties compared to ANG2002. Short Angiopeps having neurotensin fragment NT1 (Ac-KRRP(D-Y)IL) resulted in decreased activity, but activity may be retained by using a linker between the targeting moiety and NT1 or by replacing the NT1 sequence with a longer NT sequence.

**Table 9. Comparison of analgesia, hypothermia, and hypotension**

| **ANG-NT analogs** | **Analgesia (hot plate assay)** | **Hypothermia** | **Hypotension** |
|---|---|---|---|
| ANG2002 | √√√ | √√√ | √√√ |

| **Direct synthesis** | | | |
|---|---|---|---|
| An2-NT (no linker) | - | √√√ | TBD |
| An2-Ahx-NT | √√√ | √√√ | √√√ |

| **Short ANG + native NT** | | | |
|---|---|---|---|
| P5-NT | √ | √ | TBD |
| P5a-NT | √√√ | √√√√ | TBD |
| P6-NT | √ | √ | TBD |
| P6a-NT | √√√ | √√√√ | TBD |

| **Short ANG + NT fragment** | | | |
|---|---|---|---|
| P5a-NT1 | - | - | - |
| P6a-NT1 | - | √ | - |

## Claims

1. A purified polypeptide, or a pharmaceutically acceptable salt thereof, comprising the amino acid sequence Lys-Arg-X3-X4-X5-Lys (formula Ia) wherein:
X3 is Asn or Gln;
X4 is Asn or Gln; and
X5 is Phe,
wherein said polypeptide is fewer than 19 amino acids in length, and
wherein said polypeptide optionally comprises one or more D-isomers of an amino acid recited in formula la, wherein the following is not claimed:
- a polypeptide which is Ala-Lys-Arg-Asn-Asn-Phe-Lys-Ser,
- a polypeptide which is Cys-Arg-Ala-Lys-Arg-Asn-Asn-Phe-Lys-Ser-Ala.

2. The polypeptide of claim 1, wherein said amino acid sequence is Z1-Lys-Arg-X3-X4-X5-Lys-Z2 (formula 1b),
wherein:
X3 is Asn or Gln;
X4 is Asn or Gln;
X5 is Phe;
Z1 is absent, Cys, Gly, Cys-Gly, Arg-Gly, Cys-Arg-Gly, Ser-Arg-Gly, Cys-Ser-Arg-Gly, Gly-Ser-Arg-Gly, Cys-Gly-Ser-Arg-Gly, Gly-Gly-Ser-Arg-Gly, Cys-Gly-Gly-Ser-Arg-Gly, Tyr-Gly-Gly-Ser-Arg-Gly, Cys-Tyr-Gly-Gly-Ser-Arg-Gly, Phe-Tyr-Gly-Gly-Ser-Arg-Gly, Cys-Phe-Tyr-Gly-Gly-Ser-Arg-Gly, Phe-Phe-Tyr-Gly-Gly-Ser-Arg-Gly, Cys-Phe-Phe-Tyr-Gly-Gly-Ser-Arg-Gly,-Thr-Phe-Phe-Tyr-Gly-Gly-Ser-Arg-Gly, or Cys-Thr-Phe-Phe-Tyr-Gly-Gly-Ser-Arg-Gly; and
Z2 is absent, Cys, Tyr, Tyr-Cys, Cys-Tyr, Thr-Glu-Glu-Tyr, or Thr-Glu-Glu-Tyr-Cys; and
wherein said polypeptide optionally comprises one or more D-isomers of an amino acid recited in formula 1b, Z1, or Z2.

3. The polypeptide of claim 1, having the formula X1-X2-Asn-Asn-X5-X6 (formula IIa), wherein:
X1 is Lys or D-Lys;
X2 is Arg or D-Arg;
X5 is Phe or D-Phe; and
X6 is Lys or D-Lys,
wherein at least one of X1, X2, X5, or X6 is a D-amino acid.

4. The polypeptide of claim 1, wherein said amino acid sequence X1-X2-Asn-Asn-X5-X6-X7 (formula IIb),
wherein:
X1 is Lys or D-Lys;
X2 is Arg or D-Arg;
X5 is Phe or D-Phe;
X6 is Lys or D-Lys; and
X7 is Tyr or D-Tyr; and
wherein at least one of X1, X2, X5, X6, or X7 is a D-amino acid.

5. The polypeptide of claim 1, wherein said amino acid sequence is Z1-X1-X2-Asn-Asn-X5-X6-X7-Z2 (formula IIc),
wherein:
X1 is Lys or D-Lys;
X2 is Arg or D-Arg;
X5 is Phe or D-Phe;
X6 is Lys or D-Lys;
X7 is Tyr or D-Tyr;
Z1 is absent, Cys, Gly, Cys-Gly, Arg-Gly, Cys-Arg-Gly, Ser-Arg-Gly, Cys-Ser-Arg-Gly, Gly-Ser-Arg-Gly, Cys-Gly-Ser-Arg-Gly, Gly-Gly-Ser-Arg-Gly, Cys-Gly-Gly- Ser-Arg-Gly, Tyr-Gly-Gly-Ser-Arg-Gly, Cys-Tyr-Gly-Gly-Ser-Arg-Gly, Phe-Tyr-Gly-Gly-Ser-Arg-Gly, Cys-Phe-Tyr-Gly-Gly-Ser-Arg-Gly, Phe-Phe-Tyr-Gly-Gly-Ser-Arg-Gly, Cys-Phe-Phe-Tyr-Gly-Gly-Ser-Arg-Gly, Thr-Phe-Phe-Tyr-Gly-Gly-Ser-Arg-Gly, or Cys-Thr-Phe-Phe-Tyr-Gly-Gly-Ser-Arg-Gly; and
Z2 is absent, Cys, Tyr, Tyr-Cys, Cys-Tyr, Thr-Glu-Glu-Tyr, or Thr-Glu-Glu-Tyr-Cys;
wherein at least one of X1, X2, X5, X6, or X7 is a D-amino acid; and
wherein said polypeptide optionally comprises one or more D-isomers of an amino acid recited in Z1 or Z2.

6. The polypeptide of any of claims 1-5, wherein said polypeptide is fewer than 15 amino acids in length.

7. The polypeptide of any of claims 1-5, wherein said polypeptide is fewer than 10 amino acids in length.

8. The polypeptide of claim 1, wherein said polypeptide is selected from the group consisting of: 
Phe-Tyr-Gly-Gly-Ser-Arg-Gly-Lys-Arg-Asn-Asn-Phe-Lys-Thr-Glu-Glu-Tyr-Cys
(P1);
 
Phe-Tyr-Gly-Gly-Ser-Arg-Gly-D-Lys-D-Arg-Asn-Asn-D-Phe-Lys-Thr-Glu-Glu-Tyr-
Cys (P1a);
 
Phe-Tyr-Gly-Gly-Ser-Arg-Gly-D-Lys-D-Arg-Asn-Asn-D-Phe-D-Lys-Thr-Glu-Glu-
Tyr-Cys (P1b);
 
Phe-Tyr-Gly-Gly-Ser-Arg-Gly-D-Lys-D-Arg-Asn-Asn-D-Phe-D-Lys-Thr-Glu-Glu-D-
Tyr-Cys (P1c);
 
D-Phe-D-Tyr-Gly-Gly-Ser-D-Arg-Gly-D-Lys-D-Arg-Asn-Asn-D-Phe-D-Lys-Thr-
Glu-D-Glu-D-Tyr-Cys (P1d);
Gly-Gly-Ser-Arg-Gly-Lys-Arg-Asn-Asn-Phe-Lys-Thr-Glu-Glu-Tyr-Cys (P2);
Ser-Arg-Gly-Lys-Arg-Asn-Asn-Phe-Lys-Thr-Glu-Glu-Tyr-Cys (P3);
Gly-Lys-Arg-Asn-Asn-Phe-Lys-Thr-Glu-Glu-Tyr-Cys (P4);
Lys-Arg-Asn-Asn-Phe-Lys-Thr-Glu-Glu-Tyr-Cys (P5);
D-Lys-D-Arg-Asn-Asn-D-Phe-Lys-Thr-Glu-Glu-Tyr-Cys (P5a);
D-Lys-D-Arg-Asn-Asn-D-Phe-D-Lys-Thr-Glu-Glu-Tyr-Cys (P5b);
D-Lys-D-Arg-Asn-Asn-D-Phe-D-Lys-Thr-Glu-Glu-D-Tyr-Cys (P5c);
Lys-Arg-Asn-Asn-Phe-Lys-Tyr-Cys (P6);
D-Lys-D-Arg-Asn-Asn-D-Phe-Lys-Tyr-Cys (P6a);
D-Lys-D-Arg-Asn-Asn-D-Phe-D-Lys-Tyr-Cys (P6b); and
D-Lys-D-Arg-Asn-Asn-D-Phe-D-Lys-D-Tyr-Cys (P6c).

9. The polypeptide of any of claims 1-8, wherein said polypeptide is efficiently transported across the blood-brain barrier.

10. A conjugate having the formula A-X-B, wherein:
A is a targeting moiety of any of claims 1-9;
X is a linker; and
B is a therapeutic agent or transport vector.

11. The conjugate of claim 10, wherein said therapeutic agent is selected from the group consisting of an anticancer agent, a therapeutic nucleic acid agent, a small molecule drug, a label, or a therapeutic peptidic agent.

12. The conjugate of claim 11, wherein said anticancer agent is paclitaxel, docetaxel, etoposide, doxorubicin, or an analog thereof.

13. The conjugate of claim 11 or 12 for use in a method of treating or prophylactically treating cancer.

14. The conjugate for use as described in claim 13, wherein said cancer is brain cancer.

15. The conjugate of claim 11, wherein said therapeutic peptidic agent is neurotensin, a neurotensin analog, or a neurotensin receptor agonist.

16. The conjugate of claim 15, wherein said neurotensin analog is selected from the group consisting of neurotensin(6-13), neurotensin(8-13), Lys(7)-D-Tyr(11)-neurotensin(7-13), p-Glu(1)-neurotensin, p-Glu(1)-neurotensin-OH, D-Lys(6)-neurotensin(6-13), D-Tyr(11)-neurotensin(6-13), D-Lys(6)-D-Tyr(11)-neurotensin(6-13), D-Arg(8)-neurotensin(6-13), D-Arg(9)-neurotensin(6-13), D-Arg(8)-D-Arg(9)-neurotensin(6-13), D-Pro(10)-neurotensin(6-13), D-Tyr(11)-neurotensin(6-13), D-Trp(11)-neurotensin(6-13), D-Phe(11)-neurotensin(6-13), D-Arg(8)-D-Tyr(11)-neurotensin(6-13), D-Arg(8)-D-Trp(11)-neurotensin(6-13), D-Arg(8)-neurotensin(8-13), D-Arg(9)-neurotensin(8-13), D-Arg(8)-D-Arg(9)-neurotensin(8-13), D-Pro(10)-neurotensin(8-13), D-Tyr(11)-neurotensin(8-13), D-Trp(11)-neurotensin(8-13), D-Phe(11)-neurotensin(8-13), D-Arg(8)-D-Tyr(11)-neurotensin(8-13), and D-Arg(8)-D-Trp(11)-neurotensin(8-13), or an acetylated form thereof.

17. The conjugate of claim 15 or 16 for use in a method of treating or prophylactically treating pain or decreasing sensitivity to pain in a subject.

18. The conjugate for use as described in claim 17, wherein said pain is acute pain, peripheral or central neuropathic pain, inflammatory pain, migraine-related pain, headache-related pain, irritable bowel syndrome-related pain, fibromyalgia-related pain, arthritic pain, skeletal pain, joint pain, gastrointestinal pain, muscle pain, angina pain, facial pain, pelvic pain, claudication, postoperative pain, post traumatic pain, tension-type headache, obstetric pain, gynecological pain, or chemotherapy-induced pain.

## Patentansprüche

1. Gereinigtes Polypeptid oder ein pharmazeutisch annehmbares Salz davon, umfassend die Aminosäuresequenz Lys-Arg-X3-X4-X5-Lys (Formel la), wobei:
X3 Asn oder Gln ist;
X4 Asn oder Gln ist; und
X5 Phe ist,
wobei das Polypeptid eine Länge von weniger als 19 Aminosäuren hat und
wobei das Polypeptid gegebenenfalls ein oder mehrere D-Isomere einer in Formel la genannte Aminosäure umfasst, wobei das nachfolgende nicht beansprucht wird:
- ein Polypeptid, das Ala-Lys-Arg-Asn-Asn-Phe-Lys-Ser ist,
- ein Polypeptid, das Cys-Arg-Ala-Lys-Arg-Asn-Asn-Phe-Lys-Ser-Ala ist.

2. Polypeptid nach Anspruch 1, wobei die Aminosäuresequenz Z1-Lys-Arg-X3-X4-X5-Lys-Z2 (Formel 1b) ist,
wobei:
X3 Asn oder Gln ist;
X4 Asn oder Gln ist;
X5 Phe ist;
Z1 abwesend ist, Cys, Gly, Cys-Gly, Arg-Gly, Cys-Arg-Gly, Ser-Arg-Gly, Cys-Ser-Arg-Gly, Gly-Ser-Arg-Gly, Cys-Gly-Ser-Arg-Gly, Gly-Gly-Ser-Arg-Gly, Cys-Gly-Gly-Ser-Arg-Gly, Tyr-Gly-Gly-Ser-Arg-Gly, Cys-Tyr-Gly-Gly-Ser-Arg-Gly, Phe-Tyr-Gly-Gly-Ser-Arg-Gly, Cys-Phe-Tyr-Gly-Gly-Ser-Arg-Gly, Phe-Phe-Tyr-Gly-Gly-Ser-Arg-Gly, Cys-Phe-Phe-Tyr-Gly-Gly-Ser-Arg-Gly, Thr-Phe-Phe-Tyr-Gly-Gly-Ser-Arg-Gly oder Cys-Thr-Phe-Phe-Tyr-Gly-Gly-Ser-Arg-Gly; und
Z2 abwesend ist, Cys, Tyr, Tyr-Cys, Cys-Tyr, Thr-Glu-Glu-Tyr oder Thr-Glu-Glu-Tyr-Cys ist; und
wobei das Polypeptid gegebenenfalls ein oder mehrere D-Isomere einer in Formel 1 b, Z1 oder Z2 genannten Aminosäure umfasst.

3. Polypeptid nach Anspruch 1, das die Formel X1-X2-Asn-Asn-X5-X6 (Formel IIa) aufweist, wobei:
X1 Lys oder D-Lys ist;
X2 Arg oder D-Arg ist;
X5 Phe oder D-Phe ist; und
X6 Lys oder D-Lys ist,
wobei mindestens eines von X1, X2, X5 oder X6 eine D-Aminosäure ist.

4. Polypeptid nach Anspruch 1, wobei die Aminosäuresequenz X1-X2-Asn-Asn-X5-X6-X7 (Formel IIb) ist,
wobei:
X1 Lys oder D-Lys ist;
X2 Arg oder D-Arg ist;
X5 Phe oder D-Phe ist;
X6 Lys oder D-Lys ist; und
X7 Tyr oder D-Tyr ist; und
wobei mindestens eines von X1, X2, X5, X6 oder X7 eine D-Aminosäure ist.

5. Polypeptid nach Anspruch 1, wobei die Aminosäuresequenz Z1-X1-X2-Asn-Asn-X5-X6-X7-Z2 (Formel IIc) ist,
wobei:
X1 Lys oder D-Lys ist;
X2 Arg oder D-Arg ist;
X5 Phe oder D-Phe ist;
X6 Lys oder D-Lys ist;
X7 Tyr oder D-Tyr ist;
Z1 abwesend, Cys, Gly, Cys-Gly, Arg-Gly, Cys-Arg-Gly, Ser-Arg-Gly, Cys-Ser-Arg-Gly, Gly-Ser-Arg-Gly, Cys-Gly-Ser-Arg-Gly, Gly-Gly-Ser-Arg-Gly, Cys-Gly-Gly-Ser-Arg-Gly, Tyr-Gly-Gly-Ser-Arg-Gly, Cys-Tyr-Gly-Gly-Ser-Arg-Gly, Phe-Tyr-Gly-Gly-Ser-Arg-Gly, Cys-Phe-Tyr-Gly-Gly-Ser-Arg-Gly, Phe-Phe-Tyr-Gly-Gly-Ser-Arg-Gly, Cys-Phe-Phe-Tyr-Gly-Gly-Ser-Arg-Gly, Thr-Phe-Phe-Tyr-Gly-Gly-Ser-Arg-Gly oder Cys-Thr-Phe-Phe-Tyr-Gly-Gly-Ser-Arg-Gly ist; und
Z2 abwesend ist, Cys, Tyr, Tyr-Cys, Cys-Tyr, Thr-Glu-Glu-Tyr oder Thr-Glu-Glu-Tyr-Cys ist;
wobei mindestens eines von X1, X2, X5, X6 oder X7 eine D-Aminosäure ist; und wobei das Polypeptid gegebenenfalls ein oder mehrere D-Isomere einer in Z1 oder Z2 genannten Aminosäuren umfasst.

6. Polypeptid nach einem beliebigen der Ansprüche 1 bis 5, wobei das Polypeptid eine Länge von weniger als 15 Aminosäuren hat.

7. Polypeptid nach einem beliebigen der Ansprüche 1 bis 5, wobei das Polypeptid eine Länge von weniger als 10 Aminosäuren hat.

8. Polypeptid nach Anspruch 1, wobei das Polypeptid ausgewählt ist aus der Gruppe bestehend aus: 
Phe-Tyr-Gly-Gly-Ser-Arg-Gly-Lys-Arg-Asn-Asn-Phe-Lys-Thr-Glu-Glu-Tyr-
Cys (P1);
 
Phe-Tyr-Gly-Gly-Ser-Arg-Gly-D-Lys-D-Arg-Asn-Asn-D-Phe-Lys-Thr-Glu-
Glu-Tyr-Cys (P1a);
 
Phe-Tyr-Gly-Gly-Ser-Arg-Gly-D-Lys-D-Arg-Asn-Asn-D-Phe-D-Lys-Thr-Glu-
Glu-Tyr-Cys (P1b);
 
Phe-Tyr-Gly-Gly-Ser-Arg-Gly-D-Lys-D-Arg-Asn-Asn-D-Phe-D-Lys-Thr-Glu-
Glu-D-Tyr-Cys (P1c);
 
D-Phe-D-Tyr-Gly-Gly-Ser-D-Arg-Gly-D-Lys-D-Arg-Asn-Asn-D-Phe-D-Lys-
Thr-Glu-D-Glu-D-Tyr-Cys (P1d);
Gly-Gly-Ser-Arg-Gly-Lys-Arg-Asn-Asn-Phe-Lys-Thr-Glu-Glu-Tyr-Cys (P2);
Ser-Arg-Gly-Lys-Arg-Asn-Asn-Phe-Lys-Thr-Glu-Glu-Tyr-Cys (P3);
Gly-Lys-Arg-Asn-Asn-Phe-Lys-Thr-Glu-Glu-Tyr-Cys (P4);
Lys-Arg-Asn-Asn-Phe-Lys-Thr-Glu-Glu-Tyr-Cys (P5);
D-Lys-D-Arg-Asn-Asn-D-Phe-Lys-Thr-Glu-Glu-Tyr-Cys (P5a);
D-Lys-D-Arg-Asn-Asn-D-Phe-D-Lys-Thr-Glu-Glu-Tyr-Cys (P5b);
D-Lys-D-Arg-Asn-Asn-D-Phe-D-Lys-Thr-Glu-Glu-D-Tyr-Cys (P5c);
Lys-Arg-Asn-Asn-Phe-Lys-Tyr-Cys (P6);
D-Lys-D-Arg-Asn-Asn-D-Phe-Lys-Tyr-Cys (P6a);
D-Lys-D-Arg-Asn-Asn-D-Phe-D-Lys-Tyr-Cys (P6b); und
D-Lys-D-Arg-Asn-Asn-D-Phe-D-Lys-D-Tyr-Cys (P6c).

9. Polypeptid nach einem beliebigen der Ansprüche 1 bis 8, wobei das Polypeptid effizient durch die Blut-Hirn-Schranke transportiert wird.

10. Konjugat, das die Formel A-X-B aufweist, wobei:
A eine Gruppe ist, die auf ein Ziel gerichtet ist nach einem beliebigen der Ansprüche 1 bis 9;
X ein Linker ist; und
B ein therapeutisches Mittel oder ein Transportvektor ist.

11. Konjugat nach Anspruch 10, wobei das therapeutische Mittel ausgewählt ist aus der Gruppe bestehend aus einem Anti-Krebs-Mittel, einem therapeutischen Nukleinsäure-Mittel, einem kleinen-Molekül Arzneimittel, einer Markierung (label) oder einem therapeutischen peptidischen Mittel.

12. Konjugat nach Anspruch 11, wobei das Anti-Krebsmittel Paclitaxel, Docetaxel, Etoposide, Doxorubicin oder ein Analogon davon ist.

13. Konjugat nach Anspruch 11 oder 12, zur Verwendung in einem Verfahren zum Behandeln oder prophylaktischen Behandeln von Krebs.

14. Konjugat zur Verwendung wie in Anspruch 13 beschrieben, wobei der Krebs ein Hirnkrebs ist.

15. Konjugat nach Anspruch 11, wobei das therapeutische peptidische Mittel Neurotensin, ein Neurotensin-Analogon oder ein Neurotensin-Rezeptoragonist ist.

16. Konjugat nach Anspruch 15, wobei das Neurotensin-Analogon ausgewählt ist aus der Gruppe bestehend aus Neurotensin(6-13), Neurotensin(8-13), Lys(7)-D-Tyr(11)-Neurotensin(7-13), p-Glu(1)-Neurotensin, p-Glu(1)-Neurotensin-OH, D-Lys(6)-Neurotensin(6-13), D-Tyr(11)-Neurotensin(6-13), D-Lys(6)-D-Tyr(11)-Neurotensin(6-13), D-Arg(8)-Neurotensin(6-13), D-Arg(9)-Neurotensin(6-13), D-Arg(8)-D-Arg(9)-Neurotensin(6-13), D-Pro(10)-Neurotensin(6-13), D-Tyr(11)-Neurotensin(6-13), D-Trp(11)-Neurotensin(6-13), D-Phe(11)-Neurotensin(6-13), D-Arg(8)-D-Tyr(11)-Neurotensin(6-13), D-Arg(8)-D-Trp(11)-Neurotensin(6-13), D-Arg(8)-Neurotensin(8-13), D-Arg(9)-Neurotensin(8-13), D-Arg(8)-D-Arg(9)-Neurotensin(8-13), D-Pro(10)-Neurotensin(8-13), D-Tyr(11)-Neurotensin(8-13), D-Trp(11)-Neurotensin(8-13), D-Phe(11)-Neurotensin(8-13), D-Arg(8)-D-Tyr(11)-Neurotensin(8-13), und D-Arg(8)-D-Trp(11)-Neurotensin(8-13) oder eine acetylierte Form davon.

17. Konjugat nach Anspruch 15 oder 16, zur Verwendung in einem Verfahren zum Behandeln oder prophylaktischen Behandeln von Schmerz oder zum Verringern der Schmerzempfindlichkeit in einem Individuum.

18. Konjugat zur Verwendung wie in Anspruch 17 beschrieben, wobei der Schmerz akuter Schmerz, peripherer oder zentraler neuropathischer Schmerz, entzündlicher Schmerz, Schmerz, der mit Migräne einhergeht, Schmerz, der mit Kopfschmerzen einhergeht, Schmerz, der mit Reizdarmsyndrom einhergeht, Schmerz, der mit Fibromyalgie einhergeht, arthritischer Schmerz, Skelettschmerz, Gelenkschmerz, gastrointestinaler Schmerz, Muskelschmerz, Angina-Schmerz, Gesichtsschmerz, Beckenschmerz, Claudicatio, post-operativer Schmerz, post-traumatischer Schmerz, Spannungstyp-Kopfschmerz, Geburtsschmerz, gynäkologischer Schmerz oder Chemotherapie-induzierter Schmerz ist.

## Revendications

1. Polypeptide purifié, ou l'un de ses sels pharmaceutiquement acceptables, comprenant la séquence d'acides aminés Lys-Arg-X3-X4-X5-Lys (formule Ia) dans laquelle :
X3 représente Asn ou Gln ;
X4 représente Asn ou Gln ; et
X5 représente Phe,
dans lequel ledit polypeptide a une longueur inférieure à 19 acides aminés, et
dans lequel ledit polypeptide comprend éventuellement un ou plusieurs isomères D d'un acide aminé cité dans la formule Ia, où le suivant n'est pas revendiqué :
- un polypeptide qui est Ala-Lys-Arg-Asn-Asn-Phe-Lys-Ser,
- un polypeptide qui est Cys-Arg-Ala-Lys-Arg-Asn-Asn-Phe-Lys-Ser-Ala.

2. Polypeptide selon la revendication 1, dans lequel ladite séquence d'acides aminés est Z1-Lys-Arg-X3-X4-X5-Lys-Z2 (formule Ib),
dans laquelle :
X3 représente Asn ou Gln ;
X4 représente Asn ou Gln ;
X5 représente Phe ;
Z1 est absent ou représente Cys, Gly, Cys-Gly, Arg-Gly, Cys-Arg-Gly, Ser-Arg-Gly, Cys-Ser-Arg-Gly, Gly-Ser-Arg-Gly, Cys-Gly-Ser-Arg-Gly, Gly-Gly-Ser-Arg-Gly, Cys-Gly-Gly-Ser-Arg-Gly, Tyr-Gly-Gly-Ser-Arg-Gly, Cys-Tyr-Gly-Gly-Ser-Arg-Gly, Phe-Tyr-Gly-Gly-Ser-Arg-Gly, Cys-Phe-Tyr-Gly-Gly-Ser-Arg-Gly, Phe-Phe-Tyr-Gly-Gly-Ser-Arg-Gly, Cys-Phe-Phe-Tyr-Gly-Gly-Ser-Arg-Gly, Thr-Phe-Phe-Tyr-Gly-Gly-Ser-Arg-Gly, ou Cys-Thr-Phe-Phe-Tyr-Gly-Gly-Ser-Arg-Gly ; et
Z2 est absent ou représente Cys, Tyr, Tyr-Cys, Cys-Tyr, Thr-Glu-Glu-Tyr, ou Thr-Glu-Glu-Tyr-Cys ; et
dans lequel ledit polypeptide comprend éventuellement un ou plusieurs isomères D d'un acide aminé cité dans la formule Ib, Z1, ou Z2.

3. Polypeptide selon la revendication 1, ayant la formule X1-X2-Asn-Asn-X5-X6 (formule IIa), dans laquelle :
X1 représente Lys ou D-Lys ;
X2 représente Arg ou D-Arg ;
X5 représente Phe ou D-Phe ; et
X6 représente Lys ou D-Lys ;
dans lequel au moins l'un de X1, X2, X5, ou X6 représente un acide D-aminé.

4. Polypeptide selon la revendication 1, dans lequel ladite séquence d'acides aminés est X1-X2-Asn-Asn-X5-X6-X7 (formule IIb),
dans laquelle :
X1 représente Lys ou D-Lys ;
X2 représente Arg ou D-Arg ;
X5 représente Phe ou D-Phe ;
X6 représente Lys ou D-Lys ; et
X7 représente Tyr ou D-Tyr ; et
dans lequel au moins l'un de X1, X2, X5, X6, ou X7 représente un acide D-aminé.

5. Polypeptide selon la revendication 1, dans lequel ladite séquence d'acides aminés est Z1-X1-X2-Asn-Asn-X5-X6-X7-Z2 (formule IIc),
dans laquelle :
X1 représente Lys ou D-Lys ;
X2 représente Arg ou D-Arg ;
X5 représente Phe ou D-Phe ;
X6 représente Lys ou D-Lys ;
X7 représente Tyr ou D-Tyr ;
Z1 est absent ou représente Cys, Gly, Cys-Gly, Arg-Gly, Cys-Arg-Gly, Ser-Arg-Gly, Cys-Ser-Arg-Gly, Gly-Ser-Arg-Gly, Cys-Gly-Ser-Arg-Gly, Gly-Gly-Ser-Arg-Gly, Cys-Gly-Gly-Ser-Arg-Gly, Tyr-Gly-Gly-Ser-Arg-Gly, Cys-Tyr-Gly-Gly-Ser-Arg-Gly, Phe-Tyr-Gly-Gly-Ser-Arg-Gly, Cys-Phe-Tyr-Gly-Gly-Ser-Arg-Gly, Phe-Phe-Tyr-Gly-Gly-Ser-Arg-Gly, Cys-Phe-Phe-Tyr-Gly-Gly-Ser-Arg-Gly, Thr-Phe-Phe-Tyr-Gly-Gly-Ser-Arg-Gly, ou Cys-Thr-Phe-Phe-Tyr-Gly-Gly-Ser-Arg-Gly ; et
Z2 est absent ou représente Cys, Tyr, Tyr-Cys, Cys-Tyr, Thr-Glu-Glu-Tyr, ou Thr-Glu-Glu-Tyr-Cys ;
dans lequel au moins l'un de X1, X2, X5, X6, ou X7 représente un acide D-aminé ; et
dans lequel ledit polypeptide comprend éventuellement un ou plusieurs isomères D d'un acide aminé cité dans Z1 ou Z2.

6. Polypeptide selon l'une quelconque des revendications 1 à 5, dans lequel ledit polypeptide a une longueur inférieure à 15 acides aminés.

7. Polypeptide selon l'une quelconque des revendications 1 à 5, dans lequel ledit polypeptide a une longueur inférieure à 10 acides aminés.

8. Polypeptide selon la revendication 1, dans lequel ledit polypeptide est choisi dans le groupe constitué de : 
Phe-Tyr-Gly-Gly-Ser-Arg-Gly-Lys-Arg-Asn-Asn-Phe-Lys-
Thr-Glu-Glu-Tyr-Cys (P1) ;
 
Phe-Tyr-Gly-Gly-Ser-Arg-Gly-D-Lys-D-Arg-Asn-Asn-D-Phe-
Lys-Thr-Glu-Glu-Tyr-Cys (P1a) ;
 
Phe-Tyr-Gly-Gly-Ser-Arg-Gly-D-Lys-D-Arg-Asn-Asn-D-Phe-
D-Lys-Thr-Glu-Glu-Tyr-Cys (P1b) ;
 
Phe-Tyr-Gly-Gly-Ser-Arg-Gly-D-Lys-D-Arg-Asn-Asn-D-Phe-
D-Lys-Thr-Glu-Glu-D-Tyr-Cys (P1c) ;
 
D-Phe-D-Tyr-Gly-Gly-Ser-D-Arg-Gly-D-Lys-D-Arg-Asn-Asn-
D-Phe-D-Lys-Thr-Glu-D-Glu-D-Tyr-Cys (P1d) ;
 
Gly-Gly-Ser-Arg-Gly-Lys-Arg-Asn-Asn-Phe-Lys-Thr-Glu-
Glu-Tyr-Cys (P2) ;
 
Ser-Arg-Gly-Lys-Arg-Asn-Asn-Phe-Lys-Thr-Glu-Glu-Tyr-
Cys (P3) ;
 Gly-Lys-Arg-Asn-Asn-Phe-Lys-Thr-Glu-Glu-Tyr-Cys (P4) ;
Lys-Arg-Asn-Asn-Phe-Lys-Thr-Glu-Glu-Tyr-Cys (P5) ; 
D-Lys-D-Arg-Asn-Asn-D-Phe-Lys-Thr-Glu-Glu-Tyr-Cys
(P5a) ;
 
D-Lys-D-Arg-Asn-Asn-D-Phe-D-Lys-Thr-Glu-Glu-Tyr-Cys
(P5b) ;
 
D-Lys-D-Arg-Asn-Asn-D-Phe-D-Lys-Thr-Glu-Glu-D-Tyr-Cys
(P5c) ;
 Lys-Arg-Asn-Asn-Phe-Lys-Tyr-Cys (P6) ;
D-Lys-D-Arg-Asn-Asn-D-Phe-Lys-Tyr-Cys (P6a) ;
D-Lys-D-Arg-Asn-Asn-D-Phe-D-Lys-Tyr-Cys (P6b) ; et
D-Lys-D-Arg-Asn-Asn-D-Phe-D-Lys-D-Tyr-Cys (P6c).

9. Polypeptide selon l'une quelconque des revendications 1 à 8, dans lequel ledit polypeptide est transporté de façon efficace à travers la barrière hémato-encéphalique.

10. Conjugué ayant la formule A-X-B, dans laquelle :
A représente un radical de ciblage selon l'une quelconque des revendications 1 à 9 ;
X représente un lieur ; et
B représente un agent thérapeutique ou un vecteur de transport.

11. Conjugué selon la revendication 10, dans lequel ledit agent thérapeutique est choisi dans le groupe constitué d'un agent anticancéreux, un agent d'acide nucléique thérapeutique, un médicament à petite molécule, un marqueur, ou un agent peptidique thérapeutique.

12. Conjugué selon la revendication 11, dans lequel ledit agent anticancéreux est le paclitaxel, le docétaxel, l'étoposide, la doxorubicine, ou l'un de leurs analogues.

13. Conjugué selon la revendication 11 ou 12 pour une utilisation dans un procédé de traitement ou de traitement prophylactique du cancer.

14. Conjugué pour une utilisation telle que décrite dans la revendication 13, dans lequel ledit cancer est un cancer du cerveau.

15. Conjugué selon la revendication 11, dans lequel ledit agent peptidique thérapeutique est la neurotensine, un analogue de la neurotensine, ou un agoniste du récepteur de la neurotensine.

16. Conjugué selon la revendication 15, dans lequel ledit analogue de la neurotensine est choisi dans le groupe constitué de neurotensine(6-13), neurotensine(8-13), Lys(7)-D-Tyr(11)-neurotensine(7-13), p-Glu(1)-neurotensine, p-Glu(1)-neurotensine-OH, D-Lys(6)-neurotensine(6-13), D-Tyr(11)-neurotensine(6-13), D-Lys(6)-D-Tyr(11)-neurotensine(6-13), D-Arg(8)-neurotensine(6-13), D-Arg(9)-neurotensine(6-13), D-Arg(8)-D-Arg(9)-neurotensine(6-13), D-Pro(10)neurotensine(6-13), D-Tyr(11)-neurotensine(6-13), D-Trp(11)-neurotensine(6-13), D-Phe(11)-neurotensine(6-13), D-Arg(8)-D-Tyr(11)-neurotensine(6-13), D-Arg(8)-D-Trp(11)-neurotensine(6-13), D-Arg(8)-neurotensine(8-13), D-Arg(9)-neurotensine(8-13), D-Arg(8)-D-Arg(9)-neurotensine-(8-13), D-Pro(10)neurotensine(8-13), D-Tyr(11)-neuro-tensine(8-13), D-Trp(11)-neurotensine(8-13), D-Phe(11)-neurotensine(8-13), D-Arg(8)-D-Tyr(11)-neurotensine(8-13), et D-Arg(8)-D-Trp(11)-neurotensine(8-13), ou l'une de leurs formes acétylées.

17. Conjugué selon la revendication 15 ou 16 pour une utilisation dans un procédé de traitement ou de traitement prophylactique de la douleur ou de diminution de la sensibilité à la douleur chez un sujet.

18. Conjugué pour une utilisation telle que décrite dans la revendication 17, dans lequel ladite douleur est une douleur aiguë, une douleur neuropathique périphérique ou centrale, une douleur inflammatoire, une douleur associée à la migraine, une douleur associée à une céphalée, une douleur associée au syndrome du côlon irritable, une douleur associée à une fibromyalgie, une douleur arthritique, une douleur squelettique, une douleur articulaire, une douleur gastro-intestinale, une douleur musculaire, une douleur associée à une angine de poitrine, une douleur faciale, une douleur pelvienne, une claudication, une douleur post-opératoire, une douleur post-traumatique, une céphalée de type tension, une douleur obstétrique, une douleur gynécologique, ou une douleur induite par une chimiothérapie.
